# EUROPEAN PATENT SPECIFICATION

(11) **EP 1 660 134 B1**
(45) Date of publication and mention of the grant of the patent: **29.12.2010**
(21) Application number: 04763853.1
(22) Date of filing: 06.08.2004
(51) Int. Cl.: A61K 47/48, A61P 35/00, A61P 35/02, A61P 31/00

(54) **CONJUGATES OF HYDROXYALKYL STARCH AND G-CSF**
KONJUGATE VON HYDROXYALKYL-STÄRKE UND G-CSF
CONJUGES D'AMIDON HYDROXYALKYLIQUE ET DU G-CSF

(30) Priority: 08.08.2003 WO PCT/EP03/08859; 08.08.2003 WO PCT/EP03/08829; 08.08.2003 WO PCT/EP03/08858; 11.03.2004 EP 04005874; 11.03.2004 US 552281 P
(43) Date of publication of application: 31.05.2006
(73) Proprietor: Fresenius Kabi Deutschland GmbH, 61352 Bad Homburg v.d.H. (DE)
(72) Inventor: EICHNER, Wolfram, 35510 Butzbach (DE); KNOLLER, Helmut, 61169 Friedberg (DE); LUTTERBECK, Katharina, 61169 Friedberg (DE); ZANDER, Norbert, 38527 Meine (DE); FRANK, Ronald, 38527 Meine-Grassel (DE); SOMMERMEYER, Klaus, 61191 Rosbach (DE); CONRADT, Harald, S., 38114 Braunschweig (DE); GRABENHORST, Eckart, 38104 Braunschweig (DE)
(74) Representative: Altmann, Andreas
(86) International application number: PCT/EP2004/008818
(87) International publication number: WO 2005/014050

(56) References cited:
- EP-A- 1 398 327
- EP-A- 1 398 328
- WO-A-94/29370
- WO-A-99/49897
- WO-A-02/080979
- WO-A-03/074087
- WO-A-2004/024761
- SADAMOTO, REIKO ET AL: "Control of bacteria adhesion by cell-wall engineering" JOURNAL OF THE AMERICAN CHEMICAL SOCIETY , 126(12), 3755-3761 CODEN: JACSAT; ISSN: 0002-7863, 2004, XP008035406

## Description

The present invention relates to conjugates of hydroxyalkyl starch and a granulocyte colony stimulating factor protein (G-CSF) wherein these conjugates are formed by a covalent linkage between the hydroxyalkyl starch or a derivative of the hydroxyalkyl starch and the protein. The present invention also relates to the method of producing these conjugates and the use of these conjugates.

It is generally accepted that the stability of proteins can be improved and the immune response against these proteins is reduced when these proteins are coupled to polymeric molecules. WO 94/28024 discloses that physiologically active proteins modified with polyethylene glycol (PEG) exhibit reduced immunogenicity and antigenicity and circulate in the bloodstream considerably longer than unconjugated proteins, i.e. have a reduced clearance rate.

G-CSF is a 21 kDa glycoprotein stabilized by two intrachain disulfide bonds and containing a single O-linked carbohydrate moiety. Mature G-CSF has 174 amino acids. In the animal body, G-CSF is synthesized by bone marrow stromal cells, macrophages and fibroblasts. It main function is to be a growth and differentiation factor for neutrophils and their precursor cells. However, it is also known in the art that G-CSF activates mature neutrophils. In addition, it stimulates growth/differentiation of various other haemopoietic progenitor cells (in synergy with additional haemopoietic growth factors) and promotes proliferation and migration of endothelial cells. Clinically, G-CSF is administered for the treatment of deficiencies in neutrophil levels (caused, e.g. by aplastic anaemia, myelodysplasia, AIDS, or chemotherapy).

WO 02/09766 discloses, among others, biocompatible protein-polymer compounds which are produced by conjugation of biologically active protein with a biocompatible polymer derivative. The biocompatible polymers used are highly reactive branched polymers, and the resulting conjugates contain a long linker between polymer derivative and protein. As biocompatible polymers, polymers of formula (P-OCH₂CO-NH-CHR-CO-)ₙ-L-Qₖ-A are described, wherein P and Q are polymeric residues and k may be 1 or 0. For P and Q, polyethylene glycol, polypropylene glycol, polyoxyethylene, polytrimethylene glycol, polylactic acid and its derivatives, polyacrylic acid and its derivatives, polyamino acid, polyvinyl alcohol, polyurethane, polyphosphazene, poly(L-lysine), polyalkylene oxide, polyacryl amide and water soluble polymers such as dextran or polysaccharide are mentioned. As proteins, among others, alpha, beta and gamma interferons, blood factors, cytokines such as interleukins, G-CSF, GM-CSF are mentioned. In the examples of WO 02/09766, only mono-, di- and tri-polyethyleneglycol derivatives are disclosed which are coupled exclusively to interferon and epidermal growth factor, and human growth hormone.

WO 94/01483 discloses biocompatible polymer conjugates which are formed by covalently binding a biologically inactive polymer or polymer derivative to a pharmaceutically pure, synthetic hydrophilic polymer via specific types of chemical bonds. As naturally occuring polymers and derivatives thereof, polysaccharides such as hyaluronic acid, proteoglycans such as chondroitin sulfates A, B and C, chitin, heparin, heparin sulfate, dextrans such as cyclodextran, hydroxyethyl cellulose, cellulose ether and starch, lipids such as triglycerides and phospholipids are disclosed. As synthetic polymers, among others, polyethylene and derivatives thereof are described having an average molecular weight of from about 100 to about 100,000. As proteins linked to the polymer or the polymer derivative, cytokines and growth factors are described, including interferons, tumor necrosis factors, interleukins, colony stimulating factors, growth factors such as osteogenic factor extract, epidermal growth factor, transforming growth factor, platelet derived growth factor, acidic fibroblast growth factor and others are disclosed. In all working examples of WO 94/01483, polyethylene glycols derivatives are used as polymer.

WO 96/11953 discloses N-terminally chemically modified protein compounds and methods of their production. Specifically, G-CSF compositions are described which result from coupling a water soluble polymer to the N terminus of G-CSF. In the context of WO 96/11953, also consensus interferone N-terminally coupled to water soluble polymers are disclosed. While a wide variety of water polymers are listed in WO 96/11953 (e.g. copolymers of ethylene glycol and propylene glycol, carboxymethyl cellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), poly(n-vinyl pyrrolidone)polyethylene glycol, polypropylene glycol homopolymers, polypropylene oxide/ethylene oxide copolymers or polyoxyethylated polyols), only PEGylated G-CSF or consensus IFN compositions are described in the working examples of WO 96/11953.

US 6,555,660 B2 discloses polypeptide conjugates comprising a polypeptide exhibiting G-CSF activity and having an amino acid sequence that differs from the amino acid sequence of human G-CSF in at least one specified introduced and/or removed amino acid residue, wherein the conjugate comprises an attachment group for a non-polypeptide moiety, and further comprises at least one non-polypeptide moiety attached to the attachment group of the polypeptide. The non-polypeptide moiety may be a polymer such as polyethylene glycol or an oligosaccharide. In US 6,555,660 B2, it is explicitly and unambiguously stated that PEG is by far the most preferred polymer molecule since it has only few reactive groups capable of cross-linking compared to polysaccharides such as dextran.

WO 97/30148 relates to polypeptide conjugates with reduced allergenicity comprising a polymeric carrier molecule having two or more polypetide molecules coupled thereto. These conjugates are preferably part of compositions used in the personal care market. Said conjugates are produced by activating a polymeric carrier molecule, reacting two or more polypeptide molecules with the activated polymeric carrier molecule and blocking of residual active groups on the conjugate. As polymeric carrier moelcule, a vast variety is listed in WO 97/30148, including such different groups of compound like natural or synthetic homopolymers such as polyols, polyamines, polycarboxylic acids and heteropolymers comprising at least two different attachment groups. Examples are given, which comprise star PEGs, branched PEGs, polyvinyl alcohols, polycarboxylates, polyvinylpyrrolidones and poly-D,L-amino acids. Among others, also dextrans such as carboxymethyl dextran, celluloses such as hydroxyethyl cellulose or hydroxypropyl cellulose, hydrolysates of chitosan, starches such as hydroxyethyl starches or hydroxypropyl starches, glycogen, agarose, guar gum, inulin, pullulan, xanthan gum, carrageenin, pectin, alginic acid etc. are disclosed. As polypeptides, only some enzymes are explicitly disclosed.

Baldwin, J.E. et al., Tetrahedron, vol. 27 (1981), pp. 1723 - 1726 describe the chemical modification of dextran and hydroxyethyl starch to give aldehyde substituted polymers which are allowed to react with hemoglobin to give soluble polymer-bound hemoglobins. These were shown to be capable of binding oxygen, but heart perfusion experiments clearly indicated that the polymer-bound hemoglobins were not suitable for use as blood substitutes.

WO 99/49897 describes conjugates of hemoglobin formed by reacting polysaccharides such as dextrane or hydroxyethyl starch with amino groups of the hemoglobin. As functional groups of the polysaccharide, aldehyde groups produced by oxidative saccharide ring-opening are used. As preferred reducing agent used, borane dimethylamine is disclosed. Moreover, WO 99/49897 is exclusively limited to hemoglobin.

WO 03/074087 relates to a method of coupling proteins to a starch-derived modified polysaccharide. The binding action between the protein and the polysaccharide, hydroxyalkyl starch, is a covalent linkage which is formed between the terminal aldehyde group or a functional group resulting from chemical modification of said terminal aldehyde group of the hydroxy alkyl starch molecule, and a functional group of the protein. As reactive group of the protein, amino groups, thio groups and carboxyl groups are disclosed, and aldehyde groups of the protein are not mentioned. Moreover, while a vast variety of possibilities of different linkages is given in the form of many lists, including different functional groups, theoretically suitable different linker molecules, and different chemical procedures, the working examples describe only two alternatives: first, an oxidized hydroxyethyl starch is used and coupled directly to proteins using ethyldimethylaminopropyl carbodiimide (EDC)-activation, or a non-oxidized hydroxyethyl starch is used and coupled directly to a protein forming a Schiff's base which is subsequently reduced to the respective amine. Thus, the working examples of WO 03/074087 neither disclose a single conjugate coupled via a thio group or a carboxy group of the protein, nor describe a conjugate comprising hydroxyethyl starch, the protein, and one or more linker molecules. Additionally, no G-CSF molecule is used in the working examples.

Therefore, it was an object of the present invention to provide conjugates of hydroxyalkyl starch, preferably hydroxy ethyl starch, and G-CSF which are not yet described in the prior art.

It is a further object of the present invention to provide methods of producing these conjugates.

Therefore, the present invention relates to a method for preparing a conjugate comprising a protein and a polymer derivative wherein the polymer is a hydroxyalkyl search (HAS) and the protein is a granulocyte colony stimulating factor (G-CSF), the method comprising reacting at least one functional group A of the polymer derivative with at least one functional group Z of the protein and thereby forming a covalent linkage, wherein Z is an amino group, and wherein
A is selected from the group consisting of an aldehyde group, a keto group or a hemiacetal group,
- wherein the method further comprises introducing A in the polymer to give a polymer derivative
- by reacting the polymer with an at least bifunctional compound, one functional group of which reacts with the polymer and at least one other - functional group of which is an aldehyde group, a keto group or a hemiacetal group, or is a functional group which is further chemically modified to give an aldehyde group, a keto group or a hemiacetal group, and wherein the reaction of the polymer derivative with the protein is a reductive amination.

Accordingly, the present invention also relates to a conjugate as obtainable by a method as described above.

The G-CSF can be produced by chemical synthetic procedures or can be of any human (see e.g. Burgess, A.W. et al. 1977, Stimulation by human placental conditioned medium of hemopoietic colony formation by human marrow cells, Blood 49 (1977), 573-583; Shah, RG. et al. 1977, Characterization of colony-stimulating activity produced by human monocytes and phytohemagglutinin-stimulated lymphocytes, Blood 50 (1977), 811) or another mammalian source and can be obtained by purification from naturally occurring sources like human placenta, human blood or human urine. In addition, a lot of Epithelial carcinomas, acute myeloid leukemia cells and various tumor cell lines (bladder carcinomas, medulloblastomas), are capable to express this factor.

Furthermore, the expression G-CSF encompasses also a G-CSF variant wherein one or more amino acids (e.g. 1 to 25, preferably 1 to 10, more preferably 1 to 5, most preferred 1 or 2) have been exchanged by another amino acid and which exhibits G-CSF activity (see e.g. Riedhaar-Olson, J.F. et al. 1996, Identification of residues critical to the activity of human granulocyte colony stimulating factor, Biochemistry 35:9034-9041 1996; U.S. Pat. Nos. 5,581476; 5,214,132; 5,362,853; 4,904,584). The measurement of G-CSF activity is described in the art (for measurement of G-CSF activity in vitro see e.g. Shirafuji, N. et al.1989, A new bioassay for human granulocyte colony-stimulating factor (hG-CSF) using murine myeloblastic NFS-60 cells as targets and estimation of its levels in sera from normal healthy persons and patients with infectious and hematological disorders, Exp. Hematol. 1989, 17, 116-119; for measurement of G-CSF activity in vivo see e.g. Tanaka, H. et al. 1991, Pharmacokinetics of recombinant human granulocyte colony-stimulating factor conjugated to polyethylene glycol in rats, Cancer Research 51, 3710-3714,1991). Further publications where tests for the measurement of the activity of G-CSF are U.S. Pat. No. 6,555,660; Nohynek, G.J. et al.1997, Comparison of the potency of glycosylated and nonglycosylated recombinant human granulocyte colony-stimulating factors in neutropenic and nonneutropenic CD rats, Cancer Chemother Pharmacol (1997) 39;259-266.

Preferably, the G-CSF is recombinantly produced. This includes prokaryotic or eukaryotic host expression of exogenous DNA sequences obtained by genomic or cDNA cloning or by DNA synthesis. Suitable prokaryotic hosts include various bacteria such as E. coli. Suitable eukaryotic hosts include yeast such as S. cerevisiae and mammalian cells such as Chinese hamster ovary cells and monkey cells.

The recombinant production of a protein is known in the art. In general, this includes the transfection of host cells with an appropriate expression vector, the cultivation of the host cells under conditions which enable the production of the protein and the purification of the protein from the host cells. For detailed information see e.g. Souza, L.M.et al. 1986, Recombinant human granulocyte colony-stimulating factor: effects on normal and leukemic myeloid cells, Science 1986 232:61-65,1986; Nagata, S. et.al. 1986, Molecular cloning and expression of cDNA for human granulocyte colony-stimulating factor, Nature 319:415-418, 1986; Komatsu, Y. et al. 1987, Cloning of granulocyte colony-stimulating factor cDNA from human macrophages and its expression in Escherichia coli, Jpn J Cancer Res. 1987 78(11):1179-1181.

In a preferred embodiment, the G-CSF has the amino acid sequence of human mature G-CSF (see e.g.; Nagata, S. et. al. 1986, Molecular cloning and expression of cDNA for human granulocyte colony-stimulating factor, Nature 319:415-418, 1986 ), and may further contain a methionin at its amino terminus, which then results in a protein of 175 amino acids. Furthermore, instead of the methionine, G-CSF may contain a serine or a threonine residue.

The G-CSF used in the methods of the present invention and the conjugates according to the present invention may comprise one carbohydrate side chain attached to the G-CSF via O-linked glycosylation at the position Thr 133, i.e. the G-CSF is glycosylated (V. Gervais et al.,Eur. J. Biochem. 1997, 247, 386-395). The structure of the carbohydrate side chain may be NeuNAc(alpha2-3)Gal(beta1-3)[NeuNAc(alpha2-6)]GalNAc und (alpha2-3)Gal(beta1-3)GalNAc (NeuNAc = N-acetylneuramic acid, GalNAc = N-acetylgalactosamine).

Modification of G-CSF and other polypeptides so as to introduce at least one additional carbohydrate chain as compared to the native polypeptide has been suggested (U.S. Pat. No. 5,218,092). Depending on the host employed, the G-CSF expression product may be glycosylated with mammalian or other eukaryotic carbohydrates. Usually, when G-CSF is produced in eukaryotic cells, the protein is posttranslationally glycosylated. Consequently, the carbohydrate side chain may have been attached to the G-CSF during biosynthesis in mammalian, especially human, insect or yeast cells.

Recombinant human G-CSF (rhG-CSF) is generally used for treating various forms of leukopenia. Thus, commercial preparations of rhG-CSF are available under the names filgrastim (Gran® and Neupogen®), lenograstim (Neutrogin® and Granocyte®) and nartograstim (Neu-up®). Gran® and Neupogen® are non-glycosylated and produced in recombinant E. coli cells. Neutrogin® and Granocyte® are glycosylated and produced in recombinant CHO cells and Neu-up® is non-glycosylated with five amino acids substituted at the N-terminal region of intact rhG-CSF produced in recombinant E. coli cells.

As glycosylated protein, any glycosylated G-CSF such as Granocyte® may be employed. As non-glycosylated G-CSF, any non-glycosylated G-CSF such as Neupogen® may be employed in the methods and conjugate according to the present invention.

Furthermore, at position -1, G-CSF may contain a methionine amino acid residue, a serine residue, or a threonine residue.

In the context of the present invention, the term "hydroxyalkyl starch" (HAS) refers to a starch derivative which has been substituted by at least one hydroxyalkyl group. A preferred hydroxyalkyl starch of the present invention has a constitution according to formula (I) 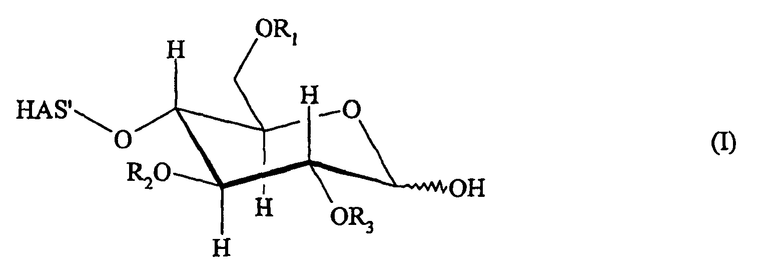 wherein the reducing end of the starch molecule is shown in the non-oxidized form and the terminal saccharide unit is shown in the acetal form which, depending on e.g. the solvent, may be in equilibrium with the aldehyde form.

The term hydroxyalkyl starch as used in the present invention is not limited to compounds where the terminal carbohydrate moiety comprises hydroxyalkyl groups R₁, R₂, and/or R₃ as depicted, for the sake of brevity, in formula (I), but also refers to compounds in which at least one hydroxy group present anywhere, either in the terminal carbohydrate moiety and/or in the remaining part of the starch molecule, HAS', is substituted by a hydroxyalkyl group R₁, R₂, or R₃.

Hydroxyalkyl starch comprising two or more different hydroxyalkyl groups are also possible.

The at least one hydroxyalkyl group comprised in HAS may contain two or more hydroxy groups. According to a preferred embodiment, the at least one hydroxyalkyl group comprised in HAS contains one hydroxy group.

The expression "hydroxyalkyl starch" also includes derivatives wherein the alkyl group is mono- or polysubstituted. In this context, it is preferred that the alkyl group is substituted with a halogen, especially fluorine, or with an aryl group. Furthermore, the hydroxy group of a hydroxyalkyl group may be esterified or etherified.

Furthermore, instead of alkyl, also linear or branched substituted or unsubstituted alkene groups may be used.

Hydroxyalkyl starch is an ether derivative of starch. Besides of said ether derivatives, also other starch derivatives can be used in the context of the present invention. For example, derivatives are useful which comprise esterified hydroxy groups. These derivatives may be e.g. derivatives of unsubstituted mono- or dicarboxylic acids with 2-12 carbon atoms or of substituted derivatives thereof. Especially useful are derivatives of unsubstituted monocarboxylic acids with 2-6 carbon atoms, especially derivatives of acetic acid. In this context, acetyl starch, butyl starch and propyl starch are preferred.

Furthermore, derivatives of unsubstituted dicarboxylic acids with 2-6 carbon atoms are preferred.

In the case of derivatives of dicarboxylic acids, it is useful that the second carboxy group of the dicarboxylic acid is also esterified. Furthermore, derivatives of monoalkyl esters of dicarboxylic acids are also suitable in the context of the present invention.

For the substituted mono- or dicarboxylic acids, the substitute groups may be preferably the same as mentioned above for substituted alkyl residues.

Techniques for the esterification of starch are known in the art (see e.g. Klemm D. et al, Comprehensive Cellulose Chemistry Vol. 2, 1998, Whiley-VCH, Weinheim, New York, especially chapter 4.4, Esterification of Cellulose (ISBN 3-527-29489-9).

According to a preferred embodiment of the present invention, hydroxyalkyl starch according to formula (I) is employed. In formula (I), the saccharide ring described explicitly and the residue denoted as HAS' together represent the preferred hydroxyalkyl starch molecule. The other saccharide ring structures comprised in HAS' may be the same as or different from the explicitly described saccharide ring.

As far as the residues R₁, R₂ and R₃ according to formula (I) are concerned there are no specific limitations. According to a preferred embodiment, R₁, R₂ and R₃ are independently hydrogen or a hydroxyalkyl group, a hydroxyaryl group, a hydroxyaralkyl group or a hydroxyalkaryl group having of from 2 to 10 carbon atoms in the respective alkyl residue. Hydrogen and hydroxyalkyl groups having of from 2 to 10 are preferred. More preferably, the hydroxyalkyl group has from 2 to 6 carbon atoms, more preferably from 2 to 4 carbon atoms, and even more preferably from 2 to 4 carbon atoms. "hydroxyalkyl starch" therefore preferably comprises hydroxyethyl starch, hydroxypropyl starch and hydroxybutyl starch, wherein hydroxyethyl starch and hydroxypropyl starch are particularly preferred and hydroxyethyl starch is most preferred.

The alkyl, aryl, aralkyl and/or alkaryl group may be linear or branched and suitably substituted.

Therefore, the present invention also relates to a method as described above wherein R₁, R₂ and R₃ are independently hydrogen or a linear or branched hydroxyalkyl group with from 1 to 6 carbon atoms.

Thus, R₁, R₂ and R₃ preferably may be hydroxyhexyl, hydroxypentyl, hydroxybutyl, hydroxypropyl such as 2-hydroxypropyl, 3-hydroxypropyl, 2-hydroxyisopropyl, hydroxyethyl such as 2-hydroxyethyl, hydrogen and the 2-hydroxyethyl group being especially preferred.

Therefore, the present invention also relates to a method and a conjugate as described above wherein R₁, R₂ and R₃ are independently hydrogen or a 2-hydroxyethyl group, an embodiment wherein at least one residue R₁, R₂ and R₃ being 2-hydroxyethyl being especially preferred.

Hydroxyethyl starch (HES) is most preferred for all embodiments of the present invention.

Therefore, the present invention relates to the method and the conjugate as described above, wherein the polymer is hydroxyethyl starch and the polymer derivative is a hydroxyethyl starch derivative.

Hydroxyethyl starch (HES) is a derivative of naturally occurring amylopectin and is degraded by alpha-amylase in the body. HES is a substituted derivative of the carbohydrate polymer amylopectin, which is present in corn starch at a concentration of up to 95 % by weight. HES exhibits advantageous biological properties and is used as a blood volume replacement agent and in hemodilution therapy in the clinics (Sommermeyer et al., 1987, Krankenhauspharmazie, 8(8), 271-278; and Weidler et al., 1991, Arzneim.-Forschung/Drug Res., 41, 494-498).

Amylopectin consists of glucose moieties, wherein in the main chain alpha-1,4-glycosidic bonds are present and at the branching sites alpha-1,6-glycosidic bonds are found. The physical-chemical properties of this molecule are mainly determined by the type of glycosidic bonds. Due to the nicked alpha-1,4-glycosidic bond, helical structures with about six glucose-monomers per turn are produced. The physico-chemical as well as the biochemical properties of the polymer can be modified via substitution. The introduction of a hydroxyethyl group can be achieved via alkaline hydroxyethylation. By adapting the reaction conditions it is possible to exploit the different reactivity of the respective hydroxy group in the unsubstituted glucose monomer with respect to a hydroxyethylation. Owing to this fact, the skilled person is able to influence the substitution pattern to a limited extent.

HES is mainly characterized by the molecular weight distribution and the degree of substitution. There are two possibilities of describing the substitution degree:
1. The degree can be described relatively to the portion of substituted glucose monomers with respect to all glucose moieties.
2. The degree of substitution can be described as the molar substitution, wherein the number of hydroxyethyl groups per glucose moiety are described.

In the context of the present invention, the degree of substitution, denoted as DS, relates to the molar substitution, as described above.

HES solutions are present as polydisperse compositions, wherein each molecule differs from the other with respect to the polymerisation degree, the number and pattern of branching sites, and the substitution pattern. HES is therefore a mixture of compounds with different molecular weight Consequently, a particular HES solution is determined by average molecular weight with the help of statistical means. In this context, Mₙ is calculated as the arithmetic mean depending on the number of molecules. Alternatively, M_{w} (or MW), the weight mean, represents a unit which depends on the mass of the HES.

In the context of the present invention, hydroxyethyl starch may preferably have a mean molecular weight (weight mean) of from 1 to 300 kD. Hydroxyethyl starch can further exhibit a preferred molar degree of substitution of from 0.1 to 0.8 and a preferred ratio between C₂ : C₆ substitution in the range of from 2 to 20 with respect to the hydroxyethyl groups.

The term "mean molecular weight" as used in the context of the present invention relates to the weight as determined according to Sommermeyer et al., 1987, Krankenhauspharmazie, 8(8), 271-278; and Weidler et al., 1991, Arzneim.-Forschung/Drug Res., 41, 494-498.

According to a preferred embodiment of the present invention, the mean molecular weight of hydroxyethyl starch employed is from 1 to 300 kD, more preferably from 2 to 200 kD, more preferably of from 4 to 130 kD, more preferably of from 4 to 70 kD.

An example for HES with a mean molecular weight of about 130 kD is Voluven® from Fresenius. Voluven® is an artifical colloid, employed, e.g., for volume replacement used in the therapeutic indication for therapy and prophylaxis of hypovolaemia. The characteristics of Voluven® are a mean molecular weight of 130,000 +/- 20,000 D, a molar substitution of 0.4 and a C2 : C6 ratio of about 9:1.

Therefore, the present invention also relates to a method and to conjugates as described above wherein the hydroxyalkyl starch is hydroxyethyl starch having a mean molecular weight of from 4 to 70 kD.

Preferred ranges of the mean molecular weight are, e.g., 4 to 70 kD or 10 to 70 kD or 12 to 70 kD or 18 to 70 kD or 50 to 70 kD or 4 to 50 kD or 10 to 50 kD or 12 to 50 kD or 18 to 50 kD or 4 to 18 kD or 10 to 18 kD or 12 to 18 kD or 4 to 12 kD or 10 to 12 kD or 4 to 10 kD.

According to particularly preferred embodiments of the present invention, the mean molecular weight of hydroxyethyl starch employed is in the range of from more than 4 kD and below 70 kD, such as about 10 kD, or in the range of from 9 to 10 kD or from 10 to 11 kD or from 9 to 11 kD, or about 12 kD, or in the range of from 11 to 12 kD or from 12 to 13 kD or from 11 to 13 kD, or about 18 kD, or in the range of from 17 to 18 kD or from 18 to 19 kD or from 17 to 19 kD, or about 50 kD, or in the range of from 49 to 50 kD or from 50 to 51 kD or from 49 to 51 kD.

As far as the degree of substitution (DS) is concerned, DS is preferably at least 0.1, more preferably at least 0.2, and more preferably at least 0.4. Preferred ranges of DS are from 0.1 to 0.8, more preferably from 0.2 to 0.8, more preferably from 0.3 to 0.8 and even more preferably from 0.4 to 0.8, still more preferably from 0.1 to 0.7, more preferably from 0.2 to 0.7, more preferably from 0.3 to 0.7 and more preferably from 0.4 to 0.7. Particularly preferred values of DS are, e.g., 0.1, 0.2, 0.3, 0.4, 0.5, 0.6, 0.7 or 0.8, with 0.2, 0.3, 0.4, 0.5, 0.6, 0.7 or 0.8 being more preferred, 0.3, 0.4, 0.5, 0.6, 0.7 or 0.8 being even more preferred, 0.4, 0.5, 0.6, 0.7 or 0.8 being still more preferred and, e.g. 0.4 and 0.7 being particularly preferred.

Particularly preferred combinations of molecular weight of the hydroxyalkyl starch, preferably hydroxyethyl starch, and its degree of substitution DS are, e.g., 10 kD and 0.4 or 10 kD and 0.7 or 12 kD and 0.4 or 12 kD and 0.7 or 18 kD and 0.4 or 18 kD and 0.7 or 50 kD and 0.4 or 50 kD and 0.7.

In another preferred embodiment of the present invention, the hydroxyethyl starch (as employed as well as contained in the conjugates described herein) has a molecular weight of from 20 kD to 130 kD (i.e. 40 kD, 50 kD, 60 kD, 70 kD, 80 kD, 90 kD, 100 kD, 110 kD, 120 kD, 130 kD) preferably a mean molecular weight of 30 kD to 100 kD, more preferably from 40 to 70 kD and a degree of substitution from 0.4 to 0.8, more preferred from 0.5 to 0.8.

In this context the term "30kD" is understood to relate to a mean molecular weight in the range of from 25 kD to 34 kD, i.e. including also starches having a mean molecular weight of 26, 27, 28, 29, 31, 32, 33 or 34 kD.

In this context the term "40kD" is understood to relate to a mean molecular weight in the range of from 35 kD to 44 kD, i.e. including also starches-having a mean molecular weight of 36, 37, 38, 39, 41, 42, 43 or 44 kD.

In this context the term "50kD" is understood to relate to a mean molecular weight in the range of from 45 kD to 54 kD, i.e. including also starches having a mean molecular weight of 46, 47, 48, 49, 51, 52, 53 or 54 kD.

In this context the term "60kD" is understood to relate to a mean molecular weight in the range of from 55 kD to 64 kD, i.e. including also starches having a mean molecular weight of 56, 57, 58, 59, 61, 62, 63 or 64 kD.

In this context the term "70kD" is understood to relate to a mean molecular weight in the range of from 65 kD to 74 kD, i.e. including also starches having a mean molecular weight of 66, 67, 68, 69, 71, 72, 73 or 74 kD.

In this context the term "80kD" is understood to relate to a mean molecular weight in the range of from 75 kD to 84 kD, i.e. including also starches having a mean molecular weight of 76, 77, 78, 79, 81, 82, 83 or 84 kD.

In this context the term "90kD" is understood to relate to a mean molecular weight in the range of from 85 kD to 94 kD, i.e. including also starches having a mean molecular weight of 86, 87, 88, 89, 91, 92, 93 or 94 kD.

In this context the term " 100kD" is understood to relate to a mean molecular weight in the range of from 95 kD to 104 kD, i.e. including also starches having a mean molecular weight of 96, 97, 98, 99, 101, 102, 103 or 104 kD.

In this context the term "110kD" is understood to relate to a mean molecular weight in the range of from 105 kD to 114 kD, i.e. including also starches having a mean molecular weight of 106, 107, 108, 109, 111, 112, 113 or 114 kD.

In this context the term "120kD" is understood to relate to a mean molecular weight in the range of from 115 kD to 124 kD, i.e. including also starches having a mean molecular weight of 116, 117; 418; 119; 121; 122; 123 or 124 kD.

In this context the term "130kD" is understood to relate to a mean molecular weight in the range of from 125 kD to 134 kD, i.e. including also starches having a mean molecular weight of 126, 127, 128, 129, 131, 132, 133 or 134 kD.

Accordingly the embodiment described above comprises a hydroxethyl starch (and the conjugates described herein comprising the hydroxyl ethyl starch as well as the methods described herein employing hydroxylethyl starch) that has a mean molecular weight of 30 kD and a degree of substitution of 0.4 or 0.5 or 0.6 or 0.7 or 0.8, preferably 0.6, 0.7 or 0.8.

Accordingly the embodiment described above also comprises a hydroxethyl starch (and the conjugates described herein comprising the hydroxyl ethyl starch as well as the methods described herein employing hydroxyethyl starch) that has a mean molecular weight of 40 kD and a degree of substitution of 0.4 or 0.5 or 0.6 or 0.7 or 0.8, preferably 0.6, 0.7 or 0.8.

Accordingly the embodiment described above also comprises a hydroxethyl starch (and the conjugates described herein comprising the hydroxyl ethyl starch as well as the methods described herein employing hydroxyethyl starch) that has a mean molecular weight of 50 kD and a degree of substitution of 0.4 or 0.5 or 0.6 or 0.7 or 0.8, preferably 0.6, 0.7 or 0.8.

Accordingly the embodiment described above also comprises a hydroxethyl starch (and the conjugates described herein comprising the hydroxyl ethyl starch as well as the methods described herein employing hydroxyethyl starch) that has a mean molecular weight of 60 kD and a degree of substitution of 0.4 or 0.5 or 0.6 or 0.7 or 0.8, preferably 0.6, 0.7 or 0.8.

Accordingly the embodiment described above also comprises a hydroxethyl starch (and the conjugates described herein comprising the hydroxyl ethyl starch as well as the methods described herein employing hydroxyethyl starch) that has a mean molecular weight of 70 kD and a degree of substitution of 0.4 or 0.5 or 0.6 or 0.7 or 0.8, preferably 0.6, 0.7 or 0.8.

Accordingly the embodiment described above also comprises a hydroxethyl starch (and the conjugates described herein comprising the hydroxyl ethyl starch as well as the methods described herein employing hydroxyethyl starch) that has a mean molecular weight of 80 kD and a degree of substitution of 0.4 or 0.5 or 0.6 or 0.7 or 0.8, preferably 0.6, 0.7 or 0.8.

Accordingly the embodiment described above also comprises a hydroxethyl starch (and the conjugates described herein comprising the hydroxyl ethyl starch as well as the methods described herein employing hydroxyethyl starch) that has a mean molecular weight of 90 kD and a degree of substitution of 0.4 or 0.5 or 0.6 or 0.7 or 0.8, preferably 0.6, 0.7 or 0.8.

Accordingly the embodiment described above also comprises a hydroxethyl starch (and the conjugates described herein comprising the hydroxyl ethyl starch as well as the methods described herein employing hydroxyethyl starch) that has a mean molecular weight of 100 kD and a degree of substitution of 0.4 or 0.5 or 0.6 or 0.7 or 0.8, preferably 0.6, 0.7 or 0.8.

Accordingly the embodiment described above also comprises a hydroxethyl starch (and the conjugates described herein comprising the hydroxyl ethyl starch as well as the methods described herein employing hydroxyethyl starch) that has a mean molecular weight of 110 kD and a degree of substitution of 0.4 or 0.5 or 0.6 or 0.7 or 0.8, preferably 0.6, 0.7 or 0.8.

Accordingly the embodiment described above comprises a hydroxethyl starch (and the conjugates described herein comprising the hydroxyl ethyl starch as well as the methods described herein employing hydroxyethyl starch) that has a mean molecular weight of 120 kD and a degree of substitution of 0.4 or 0.5 or 0.6 or 0.7 or 0.8, preferably 0.6, 0.7 or 0.8.

Accordingly the embodiment described above comprises a hydroxethyl starch (and the conjugates described herein comprising the hydroxyl ethyl starch as well as the methods described herein employing hydroxyethyl starch) that has a mean molecular weight of 130 kD and a degree of substitution of 0.4 or 0.5 or 0.6 or 0.7 or 0.8, preferably 0.6, 0.7 or 0.8.

An example of HES having a mean molecular weight of about 130 kD is a HES with a degree of substitution of 0.2 to 0.8 such as 0.2, 0.3, 0.4, 0.5, 0.6, 0.7, or 0.8, preferably of 0.4 to 0.7 such as 0.4, 0.5, 0.6, or 0.7.

As far as the ratio of C₂ : C₆ substitution is concerned, said substitution is preferably in the range of from 2 to 20, more preferably in the range of from 2 to 15 and even more preferably in the range of from 3 to 12.

According to a further embodiment of the present invention, also mixtures of hydroxyethyl starches may be employed having different mean molecular weights and/or different degrees of substitution and/or different ratios of C₂ : C₆ substitution. Therefore, mixtures of hydroxyethyl starches may be employed having different mean molecular weights and different degrees of substitution and different ratios of C₂ : C₆ substitution, or having different mean molecular weights and different degrees of substitution and the same ratio of C₂ : C₆ substitution, or having different mean molecular weights and the same degree of substitution and different ratios of C₂ : C₆ substitution, or having the same mean molecular weight and different degrees of substitution and different ratios of C₂ : C₆ substitution, or having different mean molecular weights and the same degree of substitution and the same ratio of C₂ : C₆ substitution, or having the same mean molecular weights and different degrees of substitution and the same ratio of C₂ : C₆ substitution, or having the same mean molecular weight and the same degree of substitution and different ratios of C₂ : C₆ substitution, or having the same mean molecular weight and the same degree of substitution and the same ratio of C₂ : C₆ substitution.

In different conjugates and/or different methods according to the present invention, different hydroxyalkyl starches, preferably different hydroxyethyl starches and/or different hydroxyalkyl starch mixtures, preferably different hydroxyethyl starch mixtures, may be employed.

The reductive amination reaction according to the invention, wherein the polymer or polymer derivative is covalently linked via at least one aldehyde group or keto group or hemiacetal group to at least one amino group of the protein, is preferably carried out at a temperature of from 0 to 40 °C, more preferably of from 0 to 25 °C and especially preferably of from 4 to 21 °C. The reaction time preferably ranges of from 0.5 to 72 h, more preferably of from 2 to 48 h and especially preferably of from 4 to 7 h. As solvent for the reaction, an aqueous medium is preferred.

Thus, the present invention also relates to a method and a conjugate as described above, wherein the reductive amination is carried out at a temperature of from 4 to 21 °C.

Therefore, the present invention also relates to a method and a conjugate as described above, wherein reductive amination is carried out in an aqueous medium.

Thus, the present invention also relates to a method and conjugate as described above, wherein the reductive amination is carried out at a temperature of from 4 to 21 °C in an aqueous medium.

The term "aqueous medium" as used in the context of the present invention relates to a solvent or a mixture of solvents comprising water in the range of from at least 10 % per weight, more preferably at least 20 % per weight, more preferably at least 30 % per weight, more preferably at least 40 % per weight, more preferably at least 50 % per weight, more preferably at least 60 % per weight, more preferably at least 70 % per weight, more preferably at least 80 % per weight, even more preferably at least 90 % per weight or up to 100 % per weight, based on the weight of the solvents involved. The preferred reaction medium is water.

The pH value of the reaction medium is generally in the range of from 4 to 9 or from 4 to 8 or from 4 to 7.3.

According to a preferred embodiment of the present invention, the pH at which the reductive amination reaction is carried out, is below 7.3, more preferably smaller or equal 7 and most preferably below 7, i.e. in the acidic range. Preferred ranges are therefore of from 3 to below 7, more preferably of from 3.5 to 6.5, still more preferably of from 4 to 6, still more preferably of from 4.5 to 5.5 and especially preferably about 5.0, i.e. 4.6 or 4.7 or 4.8 or 4.9 or 5.0. or 5.1 or 5.2 or 5.3 or 5.4. Preferred ranges, are among others, 3 to 6.9 or 3 to 6.5 or 3 to 6 or 3 to 5.5 or 3 to 5 or 3 to 4.5 or 3 to 4 or 3 to 3.5 or 3.5 to 6.9 or 3.5 to 6.5 or 3.5 to 6 or 3.5 to 5.5 or 3.5 to 5 or 3.5 to 4.5 or 3. 5 to 4 or 4 to 6.9 or 4 to 6.5 or 4 to 6. or 4 to 5.5 or 4 to 5 or 4 to 4.5 or 4.5 to 6.9 or 4.5 to 6.5 or 4.5 to 6 or 4.5 to 5.5 or 4.5 to 5 or 5 to 6.9 or 5 to 6.5 or 5 to 6 or 5 to 5.5 or 5.5 to 6.9 or 5.5 to 6.5 or 5.5 to 6 or 6 to 6.9 or 6 to 6.5 or 6.5 to 6.9.

Therefore, the present invention also relates to a method and a conjugate as described above, wherein the reductive amination is carried out at a pH of 7 or less, more preferably at a pH of 6 or less.

Thus, the present invention also relates to a method and conjugate as described above, wherein the reductive amination is carried out at a temperature of from 4 to 21 °C at a pH of 7 or less, preferably of 6 or less.

Hence, the present invention also relates to a method and conjugate as described above, wherein the reductive amination is carried out in an aqueous medium at a pH of 7 or less, preferably of 6 or less.

Accordingly, the present invention also relates to a method and conjugate as described above, wherein the reductive amination is carried out at a temperature of from 4 to 21 °C in an aqueous medium at a pH of 7 or less, preferably of 6 or less.

The molar ratio of polymer derivative : protein used for the reaction is preferably in the range of from 200:1 to 5:1, more preferably of from 100:1 to 10:1 and especially preferably of from 75:1 to 20:1 .

It was surprisingly found that it was possible, especially at the preferred pH ranges given above, particularly at a pH below 7 and greater or equal 4, to react the polymer derivative predominantly with the amino group located at the N terminus of the protein. The term "predominantly" as used in the context of the present invention relates to an embodiment where at least 80 %, preferably at least 85 % of the N-terminal amino groups available are reacted via reductive amination. It is also possible to react at least 90 % or at least 95 % or at least 96 % or at least 97 % or at least 98 % or at least 99 % of the N-terminal amino groups available. Although coupling to amino groups other than the N-terminal amino group could not be ruled out completely, it is believed that coupling via reductive amination according to the present invention at a pH of below 7, preferably below 6, took place essentially selectively at the N-terminal amino group.

Therefore, the present invention also relates to a method and a conjugate as described above, wherein the protein comprises the N-terminal amino group and at least one further amino group, said conjugate comprises the polymer being predominantly coupled to the N-terminal amino group.

According to an especially preferred embodiment, the present invention relates to a method of linking aldehyde or keto or hemiacetal functionalized hydroxyalkyl starch or an aldehyde or keto or hemiacetal functionalized hydroxyalkyl starch derivative predominantly to the N-terminal amino group of a protein, said method comprising subjecting said hydroxyalkyl starch or derivative thereof to a reductive amination reaction, at a pH of 7 or less, preferably at a pH of 6 or less, said reductive amination reaction being carried out preferably in an aqueous medium.

According to the present invention, aldehyde functionalized hydroxyalkyl starch or an aldehyde functionalized hydroxyalkyl starch derivative is preferred.

According to a still further preferred embodiment, the present invention relates to a method of linking aldehyde or keto or hemiacetal functionalized hydroxyethyl starch or an aldehyde or keto or hemiacetal functionalized hydroxyethyl starch derivative selectively to the N-terminal amino group of a protein, said method comprising subjecting said hydroxyalkyl starch or derivative thereof to a reductive amination reaction, at a pH of 7 or less, preferably at a pH of 6 or less, said reductive amination reaction being carried out preferably in an aqueous medium, the hydroxyethyl starch employed preferably being hydroxethyl starch having a mean molecular weight of about 10 kD and a DS of about 0.4 or hydroxethyl starch having a mean molecular weight of 10 kD and a DS of 0.7 or hydroxethyl starch having a mean molecular weight of 12 kD and a DS of 0.4 or hydroxethyl starch having a mean molecular weight of 12 kD and a DS of 0.7 or hydroxethyl starch having a mean molecular weight of 18 kD and a DS of 0.4 or hydroxethyl starch having a mean molecular weight of 18 kD and a DS of 0.7 or hydroxethyl starch having a mean molecular weight of 50 kD and a DS of 0.4 or hydroxethyl starch having a mean molecular weight of 50 kD and a DS of 0.7.

The reaction of the polymer derivative and the protein between the aldehyde group or keto group or hemiacetal group and the amino group is a reductive amination wherein a Schiff's base is produced. Subsequently after the reaction, this base may be reduced by at least one reductive agent to give a stable linkage between the polymer derivative and the protein. It is also possible to carry out the reaction in the presence of at least one reductive agent. According to a preferred embodiment, the reductive amination reaction is carried out in the presence of at least one reductive agent.

Preferred reductive agents are sodium borohydride, sodium cyanoborohydride, organic borane complex compounds such as a 4-(dimethylamin)pyridine borane complex, N-ethyldiisopropylamine borane complex, N-ethylmorpholine borane complex, N-methylmorpholine borane complex, N-phenylmorpholine borane complex, lutidine borane complex, triethylamine borane complex, or trimethylamine borane complex. Particularly preferred is sodium cyanoborohydride.

Therefore, the present invention also relates to a method and a conjugate as described above, wherein the reductive amination is carried out in the presence of NaCNBH₃.

Hence, the present invention also relates to a method and conjugate as described above, wherein the reductive amination is carried out in an aqueous medium at a pH of 7 or less, preferably of 6 or less in the presence of reductive agent, preferably NaCNBH₃.

Accordingly, the present invention also relates to a method and conjugate as described above, wherein the reductive amination is carried out at a temperature of from 4 to 21 °C in an aqueous medium at a pH of 7 or less, preferably of 6 or less in the presence of reductive agent, preferably NaCNBH₃.

The molar ratio of polymer derivative : protein used for the reaction is preferably in the range of from 200:1 to 10:1 more preferably of from 100:1 to 10:1 and especially preferably of from 75:1 to 20:1.

Therefore, the present invention also relates to a method of producing a conjugate, said method comprising reacting a polymer or a polymer derivative comprising an aldehyde group in an aqueous medium with an amino group of the protein in the presence of a reductive agent, said reductive agent preferably being NaCNBH₃.

According to the first preferred embodiment of the present invention, according to which the polymer comprises at least two aldehyde groups which are introducing in the polymer by a ring-opening oxidation reaction, the polymer preferably comprises at least one structure according to formula

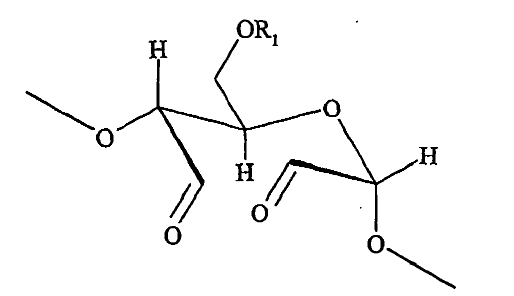

According to this embodiment of the present invention, each oxidation agent or combination of oxidation agents may be employed which is capable of oxidizing at least one saccharide ring of the polymer to give an opened saccharide ring having at least one, preferably at least two aldehyde groups. This reaction is illustrated by the following reaction scheme which shows a saccharide ring of the polymer which is oxidized to give an opened ring having two aldehyde groups:

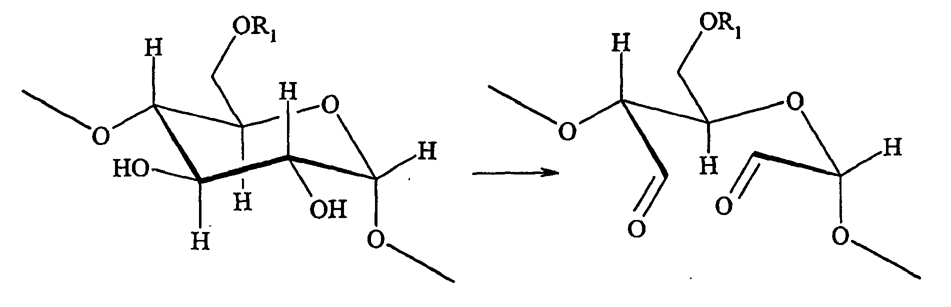

Suitable oxidating agents are, among others, periodates such as alkaline metal periodates or mixtures of two or more thereof, with sodium periodate and potassium periodate being preferred.

Therefore, the present invention also relates to a method and a conjugate as described above, wherein the polymer is subjected to a ring-opening oxidation reaction using a periodate to give a polymer derivative having at least one, preferably at least two aldehyde groups.

For this oxidation reaction, the polymer may be employed with its reducing end either in the oxidized or in the non-oxidized form, the non-oxidized form being preferred.

Therefore, the present invention also relates to a method and a conjugate as described above, wherein the polymer is employed with its reducing end in the non-oxidized form.

The reaction temperature is in a preferred range of from 0 to 40°C, more preferably of from 0 to 25 °C and especially preferably of from 0 to 5 °C. The reaction time is in a preferred range of from 1 min to 5 h and especially preferably of from 10 min to 4 h. Depending on the desired degree of oxidiation, i.e. the number of aldehyde groups resulting from the oxidation reaction, the molar ratio of periodate : polymer may be appropriately chosen.

Therefore, the present invention also relates to a method and a conjugate as described above, wherein the ring-opening oxidation reaction is carried out at a temperature of from 0 to 5 °C.

The oxidation reaction of the polymer with periodate is preferably carried out in an aqueous medium, most preferably in water.

Therefore, the present invention also relates to a method and a conjugate as described above, wherein the ring-opening oxidation reaction is carried out in an aqueous medium. The suitable pH value of the reaction mixture may be adjusted by adding at least one suitable buffer. Among the preferred buffers, sodium acetate buffer, phosphate or borate buffers may be mentioned.

The hydroxyethyl starch subjected to said ring-opening oxidation reaction is preferably hydroxethyl starch having a mean molecular weight of 10 kD and a DS of 0.4 or hydroxethyl starch having a mean molecular weight of 10 kD and a DS of 0.7 or hydroxethyl starch having a mean molecular weight of 12 kD and a DS of 0.4 or hydroxethyl starch having a mean molecular weight of 12 kD and a DS of 0.7 or hydroxethyl starch having a mean molecular weight of 18 kD and a DS of 0.4 or hydroxethyl starch having a mean molecular weight of 18 kD and a DS of 0.7 or hydroxethyl starch having a mean molecular weight of 50 kD and a DS of 0.4 or hydroxethyl starch having a mean molecular weight of 50 kD and a DS of 0.7.

The resulting polymer derivative may be purified from the reaction mixture by at least one suitable method. If necessary, the polymer derivative may be precipitated prior to the isolation by at least one suitable method.

If the polymer derivative is precipitated first, it is possible, e.g., to contact the reaction mixture with at least one solvent or solvent mixture other than the solvent or solvent mixture present in the reaction mixture at suitable temperatures. According to a particularly preferred embodiment of the present invention where an aqueous medium, preferably water is used as solvent, the reaction mixture is contacted with 2-propanol or with am mixture of acetone and ethanol, preferably a 1:1 mixture (v/v), indicating equal volumes of said compounds, at a temperature, preferably in the range of from -20 to +50 °C and especially preferably in the range of from -20 to 25 °C.

Isolation of the polymer derivative may be carried out by a suitable process which may comprise one or more steps. According to a preferred embodiment of the present invention, the polymer derivative is first separated off the reaction mixture or the mixture of the reaction mixture with, e.g., aqueous 2-propanol mixture, by a suitable method such as centrifugation or filtration. In a second step, the separated polymer derivative may be subjected to a further treatment such as an after-treatment like dialysis, centrifugal filtration or pressure filtration, ion exchange chromatography, reversed phase chromatography, HPLC, MPLC, gel filtration and/or lyophilisation. According to an even more preferred embodiment, the separated polymer derivative is first dialysed, preferably against water, and then lyophilized until the solvent content of the reaction product is sufficiently low according to the desired specifications of the product. Lyophilisation may be carried out at temperature of from 20 to 35 °C, preferably of from 20 to 30 °C.

According to a preferred embodiment, the oxidized polymer resulting from the oxidation reaction is purified using at least one suitable method such as ultrafiltration and/or dialysis in order to, e.g., remove undesirable low molecular weight salts and polymer components, thereby also offering a means of controlling the molecular weight range of oxidized polymer.

The oxidized polymer can be used directly for the reaction with the protein or is suitably recovered in a first step, e.g. by lyophilization, and redissolved in water for conjugation to the protein in a second step. As to the coupling of at least one amino group of the protein with at least one aldehyde group of the polymer by reductive amination, reference is made to the detailed disclosure above concerning the specific reaction parameters of the reductive amination reaction such as pH or temperature.

According to the second preferred embodiment, the polymer is reacted with an at least bifunctional compound comprising at least one functional group M capable of being reacted with the polymer and at least one functional group Q which is an aldehyde group or a keto group or a hemiacetal group and which is reacted with an amino group of the protein by reductive amination.

The oxidized polymer can be used directly for the reaction with the protein or is suitably recovered in a first step, e.g. by lyophilization, and redissolved in water for conjugation to the protein in a second step. As to the coupling of at least one amino group of the protein with at least one aldehyde group of the polymer by reductive amination, reference is made to the detailed disclosure above concerning the specific reaction parameters of the reductive amination reaction such as pH or temperature. According to especially preferred embodiments of the present invention, the reductive amination is preferably carried out at a temperature of from 0 to 5 °C such as about 4 °C at a pH of about 4.5 to 5.5 such as about 5.0 and for a reaction time of about 20 to 30 h such as about 24 h.

According to the second preferred embodiment, the polymer is reacted with an at least bifunctional compound comprising at least one functional group M capable of being reacted with the polymer and at least one functional group Q which is an aldehyde group, a keto group or a hemiacetal group and which is reacted with an amino group of the protein by reductive amination.

It is preferred to employ a compound having, apart from the aldehyde group or keto group or hemiacetal group, at least one carboxy group or at least one reactive carboxy group, preferably one carboxy group or one reactive carboxy group. The aldehyde group or keto group or hemiacetal group and the carboxy group or the reactive carboxy group may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 2 to 10, more preferably from 2 to 6 and especially preferably from 2 to 4 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to an even more preferred embodiment, the hydrocarbon residue is an aryl residue having 5 to 7 and preferably 6 carbon atoms. Most preferably, the hydrocarbon residue is the benzene residue. According to this preferred embodiment, the carboxy group and the aldehyde group may be located at the benzene ring in 1,4-position, 1,3-position or 1,2-position, the 1,4-position being preferred.

As reactive carboxy group, a reactive ester, isothiocyanates or isocyanate may be mentioned. Preferred reactive esters are derived from N-hydroxy succinimides such as N-hydroxy succinimide or Sulfo-N-hydroxy succinimide, suitably substituted phenols such as p-nitrophenol, o,p-dinitrophenol, o,o'-dinitrophenol, trichlorophenol such as 2,4,6-trichlorophenol or 2,4,5-trichlorophenol, trifluorophenol such as 2,4,6-trifluorophenol or 2,4,5-trifluorophenol, pentachlorophenol, pentafluorophenol, or hydroxyazoles such as hydroxy benzotriazole. Especially preferred are N-hydroxy succinimides, with N-hydroxy succinimide and Sulfo-N-hydroxy succinimide being especially preferred. All alcohols may be employed alone or as suitable combination of two or more thereof. As reactive ester, pentafluorophenyl ester and N-hydroxy succinimide ester are especially preferred.

Thus, according to a preferred embodiment, the present invention relates to a method and a conjugate as described above, wherein the polymer is reacted with formylbenzoic acid.

According to another preferred embodiment, the present invention relates to a method and a conjugate as described above, wherein the polymer is reacted with formylbenzoic acid pentafluorophenyl ester.

According to yet another preferred embodiment, the present invention relates to a method and a conjugate as described above, wherein the polymer is reacted with formylbenzoic acid N-hydroxysuccinimide ester.

According to yet another embodiment, the present invention relates to a method and a conjugate as described above, wherein the polymer is reacted with 4-(4-formyl-3,5-dimethoxyphenoxy)butyric acid.

The hydroxyethyl starch subjected to the reaction with the compound comprising M, M preferably being a carboxy group or a reactive carboxy group and Q being an aldehyde group or a keto group or a hemiacetal group, is most preferably hydroxethyl starch having a mean molecular weight of 10 kD and a DS of 0.7. Also possible are hydroxethyl starches having a mean molecular weight of 10 kD and a DS of 0.4 or hydroxethyl starch having a mean molecular weight of 12 kD and a DS of 0.4 or hydroxethyl starch having a mean molecular weight of 12 kD and a DS of 0.7 or hydroxethyl starch having a mean molecular weight of 18 kD and a DS of 0.4 or hydroxethyl starch having a mean molecular weight of 18 kD and a DS of 0.7 or hydroxethyl starch having a mean molecular weight of 50 kD and a DS of 0.4 or hydroxethyl starch having a mean molecular weight of 50 kD and a DS of 0.7. Particularly preferably, the hydroxyalkyl starch and even more preferably the hydroxyethyl starch is employed with its reducing end in the oxidized form.

The resulting polymer derivative with the aldehyde group or the keto group or the hemiacetal group is subsequently reacted with an amino group of the protein via reductive amination. As to the coupling of at least one amino group of the protein with at least one aldehyde group or keto group or hemiacetal group of the polymer by reductive amination, reference is made to the detailed disclosure above concerning the specific reaction parameters of the reductive amination reaction such as pH or temperature. According to an especially preferred embodiment of the present invention, the reaction with the amino group of the protein is preferably carried out at a temperature of from 0 to 40 °C, more preferably of from 0 to 25 °C and especially preferably of from 4 to 21 °C. The reaction time preferably ranges of from 30 min to 72 h, more preferably of from 2 to 48 h and especially preferably of from 4 h to 17 h. As solvent for the reaction, an aqueous medium is preferred. The pH value of the reaction medium is preferably in the range of from 4 to 9, more preferably of from 4 to 8 and especially preferably of from 4.5 to 5.5.

According to the third preferred embodiment, the polymer is reacted at its optionally oxidized reducing end with an at least bifunctional compound comprising an amino group M and a functional group Q, wherein said amino group M is reacted with the optionally oxidized reducing end of the polymer and wherein the functional group Q is chemically modified to give an aldehyde functionalized polymer derivative which is reacted with an amino group of the protein by reductive amination.

As to functional group Q, the following functional groups are to be mentioned, among others:
- C-C-double bonds or C-C-triple bonds or aromatic C-C-bonds;
- the thio group or the hydroxy groups;
- alkyl sulfonic acid hydrazide, aryl sulfonic acid hydrazide;
- 1,2-dioles;
- 1,2 amino-thioalcohols;
- azides;
- 1,2-aminoalcohols;
- the amino group -NH₂ or derivatives of the amino groups comprising the structure unit -NH- such as aminoalkyl groups, aminoaryl group, aminoarakyl groups, or alkarlyaminogroups;
- the hydroxyl amino group -O-NH₂, or derivatives of the hydroxylamino group comprising the structure unit -O-NH-, such as hydroxylalkylamino groups, hydroxylarylamino groups, hydroxylaralkylamino groups, or hydroxalalkarylamino groups;
- alkoxyamino groups, aryloxyamino groups, aralkyloxyamino groups, or alkaryloxyamino groups, each comprising the structure unit -NH-O-;
- residues having a carbonyl group, -Q-C(=G)-M, wherein G is O or S, and M is, for example,
   -- -OH or -SH;
   -- an alkoxy group, an aryloxy group, an aralkyloxy group, or an alkaryloxy group;
   -- an alkylthio group, an arylthio group, an aralkylthio group, or an alkarylthio group;
   -- an alkylcarbonyloxy group, an arylcarbonyloxy group, an aralkylcarbonyloxy group, an alkarylcarbonyloxy group;
   -- activated esters such as esters of hydroxylamines having imid structure such as N-hydroxysuccinimide or having a structure unit O-N where N is part of a heteroaryl compound or, with G = O and Q absent, such as aryloxy compounds with a substituted aryl residue such as pentafluorophenyl, paranitrophenyl or trichlorophenyl;
   wherein Q is absent or NH or a heteroatom such as S or O;
- -NH-NH₂, or -NH-NH-;
- -NO₂;
- the nitril group;
- carbonyl groups such as the aldehyde group or the keto group;
- the carboxy group;
- the -N=C=O group or the -N=C=S group;
- vinyl halide groups such as the vinyl iodide or the vinyl bromide group or triflate;
- -C=C-H;
- -(C=NH₂Cl)-OAlkyl
- groups -(C=O)-CH₂-Hal wherein Hal is Cl, Br, or I;
- -CH=CH-SO₂-;
- a disulfide group comprising the structure -S-S-;
- the group 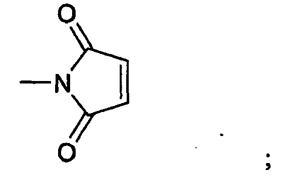
- the group

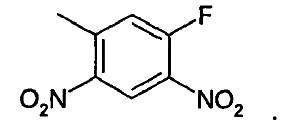

According to a preferred embodiment of the present invention, the term "functional group Q" relates to a functional group Q which comprises the chemical structure -NH-.

According to one preferred embodiment of the present invention, the functional group M is a group having the structure R'-NH- where R' is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogenes such as F, Cl or Br may be mentioned. Especially preferred residues R' are hydrogen, alkyl and alkoxy groups, and even more preferred are hydrogen and unsubstituted alkyl and alkoxy groups.

Among the alkyl and alkoxy groups, groups with 1, 2, 3, 4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

According to another embodiment of the present invention, the functional group M has the structure R'-NH-R"- where R" preferably comprises the structure unit -NH- and/or the structure unit -(C=G)- where G is O or S, and/or the structure unit -SO₂-. Specific examples for the functional group R" are 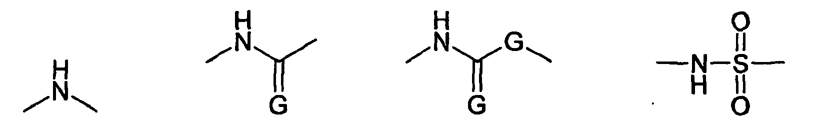 and 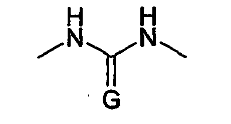 where, if G is present twice, it is independently O or S.

Therefore, the present invention also relates to a method and a conjugate as mentioned above wherein the functional group M is selected from the group consisting of 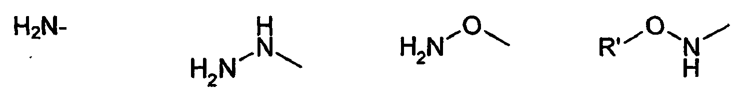 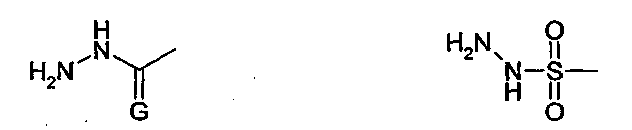 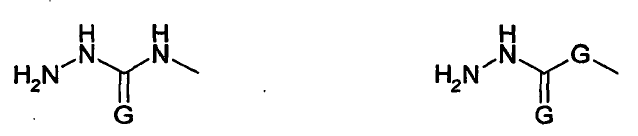 wherein G is O or S and, if present twice, independently O or S, and R' is methyl.

According to a particularly preferred embodiment of the present invention, the functional group M is an amino group -NH₂.

The term "amino group Q" relates to a functional group Q which comprises the chemical structure -NH-.

According to a preferred embodiment of the present invention, the functional group Q is a group having the structure R'-NH- where R' is hydrogen or a alkyl, cycloalkyl, aryl, aralkyl, arylcycloalkyl, alkaryl or cycloalkylaryl residue where the cycloalkyl, aryl, aralkyl, arylcycloalkyl, alkaryl or cycloalkylaryl residue may be linked directly to the NH group or, according to another embodiment, may be linked by an oxygen bridge to the NH group. The alkyl, cycloalkyl, aryl, aralkyl, arylcycloalkyl, alkaryl, or cycloalkylaryl residues may be suitably substituted. As preferred substituents, halogenes such as F, Cl or Br may be mentioned. Especially preferred residues R' are hydrogen, alkyl and alkoxy groups, and even more preferred are hydrogen and unsubstituted alkyl and alkoxy groups.

Among the alkyl and alkoxy groups, groups with 1, 2, 3, 4, 5, or 6 C atoms are preferred. More preferred are methyl, ethyl, propyl, isopropyl, methoxy, ethoxy, propoxy, and isopropoxy groups. Especially preferred are methyl, ethyl, methoxy, ethoxy, and particular preference is given to methyl or methoxy.

According to another embodiment of the present invention, the functional group Q has the structure R'-NH-R"- where R" preferably comprises the structure unit -NH- and/or the structure unit -(C=G)- where G is O or S, and/or the structure unit -SO₂-. According to more preferred embodiments, the functional group R" is selected from the group consisting of 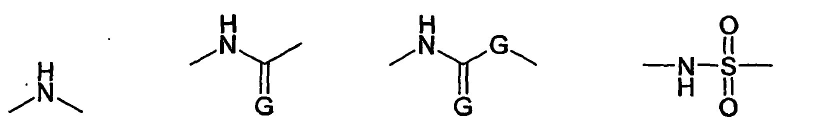 and 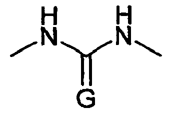 where, if G is present twice, it is independently O or S.

Therefore, the present invention also relates to a method and a conjugate as mentioned above wherein the functional group Q is selected from the group consisting of 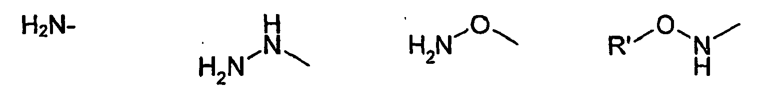 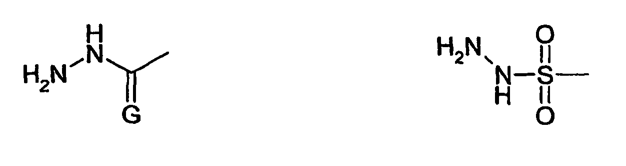 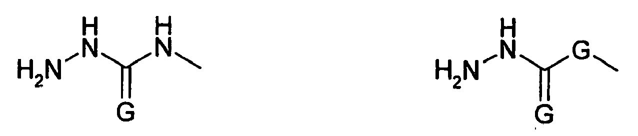 wherein G is O or S and, if present twice, independently O or S, and R' is methyl.

According to a particularly preferred embodiment of the present invention, the functional group Q is an amino group -NH₂.

According to a still further preferred embodiment of the present invention, both M and Q comprise an amino group -NH-. According to a particularly preferred embodiment, both M and Q are an amino group NH₂.

According to a preferred embodiment of the present invention, the compound comprising M and Q is a homobifunctional compound, more preferably a homobifunctional compound comprising, as functional groups M and Q, most preferably the amino group -NH₂, or according to other embodiments, the hydroxylamino group -O-NH₂ or the group 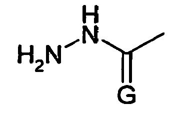 with G preferably being O. Specific examples for these compounds comprising M and Q are 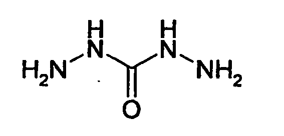 or 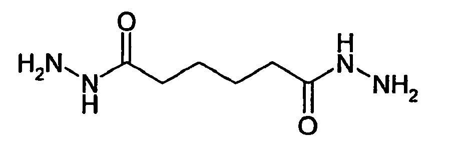 or

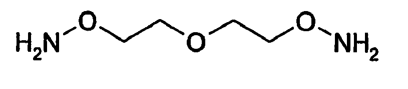

The hydroxyethyl starch subjected to the reaction with the compound comprising M, M preferably being an amino group -NH- and more preferably being an amino group -NH₂, still more preferably both M and Q comprising an amino group -NH- and particularly preferably both M and Q comprising an amino group -NH₂, is preferably hydroxethyl starch having a mean molecular weight of 10 kD and a DS of 0.4 or hydroxethyl starch having a mean molecular weight of 10 kD and a DS of 0.7. Also possible are or hydroxethyl starches having mean molecular weight of 12 kD and a DS of 0.4 or hydroxethyl starch having a mean molecular weight of 12 kD and a DS of 0.7 or hydroxethyl starch having a mean molecular weight of 18 kD and a DS of 0.4 or hydroxethyl starch having a mean molecular weight of 18 kD and a DS of 0.7 or hydroxethyl starch having a mean molecular weight of 50 kD and a DS of 0.4 or hydroxethyl starch having a mean molecular weight of 50 kD and a DS of 0.7.

In case both M and Q are an amino group -NH₂, M and Q may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 2 to 10, more preferably from 2 to 6 and especially preferably from 2 to 4 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to an even more preferred embodiment, the hydrocarbon residue is an alkyl chain of from 1 to 20, preferably from 2 to 10, more preferably from 2 to 6, and especially preferably from 2 to 4 carbon atoms.

Therefore, the present invention also relates to a method and a conjugate as described above, wherein the polymer is reacted with 1,4 diaminobutane, 1,3-diaminopropane or 1,2-diaminoethane to give a polymer derivative.

The reaction of the at least bifunctional compound comprising M and Q with the polymer is preferably carried out at a temperature of from 0 to 100 °C, more preferably of from 4 to 80 °C and especially preferably of from 20 to 80 °C; the reaction time preferably ranges of from 4 h to 7 d, more preferably of from 10 h to 5 d and especially preferably of from 17 to 4 h. The molar ratio of at least bifunctional compound : polymer is preferably in the range of from 10 to 200, specially from 50 to 100.

As solvent for the reaction of the at least bifunctional compound with the polymer, at least one aprotic solvent, particularly preferably an anhydrous aprotic solvent having a water content of not more than 0.5 percent by weight, preferably of not more than 0.1 percent by weight is preferred. Suitable solvents are, among others, dimethyl sulfoxide (DMSO), N-methyl pyrrolidone, dimethyl acetamide (DMA), dimethyl formamide (DMF) and mixtures of two or more thereof.

As solvent for the reaction of the at least bifunctional compound with the polymer, also an aqueous medium may be used.

According to a preferred embodiment, the polymer derivative comprising the polymer and the at least bifunctional compound is chemically modified at the free functional group Q to give a polymer derivative comprising an aldehyde group or keto group or hemiacetal group. According to this embodiment, it is preferred to react the polymer derivative with at least one at least bifunctional compound which comprises a functional group capable of being reacted with the functional group Q and an aldehyde group or keto group or hemiacetal group.

As at least bifunctional compound, each compound is suitable which has an aldeyhde group or keto group or hemiacetal group and at least one functional group which is capable of forming a linkage with the functional group Q of the polymer derivative. The at least one functional group is selected from the same pool of functional groups as Q and is chosen to be able to be reacted with Q. In the preferred case that Q is an amino group - NH₂, it is preferred to employ a compound having, apart from the aldehyde group or keto group or hemiacetal group, at least one carboxy group or at least one reactive carboxy group, preferably one carboxy group or one reactive carboxy group. The aldehyde group group or keto group or hemiacetal group and the carboxy group or the reactive carboxy group may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 2 to 10, more preferably from 2 to 6 and especially preferably from 2 to 4 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to an even more preferred embodiment, the hydrocarbon residue is an aryl residue having 5 to 7 and preferably 6 carbon atoms. Most preferably, the hydrocarbon residue is the benzene residue. According to this preferred embodiment, the carboxy group and the aldehyde group may be located at the benzene ring in 1,4-position, 1,3-position or 1,2-position, the 1,4-position being preferred.

As reactive carboxy group, a reactive ester, isothiocyanates or isocyanate may be mentioned. Preferred reactive esters are derived from N-hydroxy succinimides such as N-hydroxy succinimide or Sulfo-N-hydroxy succinimide, suitably substituted phenols such as p-nitrophenol, o,p-dinitrophenol, o,o'-dinitrophenol, trichlorophenol such as 2,4,6-trichlorophenol or 2,4,5-trichlorophenol, trifluorophenol such as 2,4,6-trifluorophenol or 2,4,5-trifluorophenol, pentachlorophenol, pentafluorophenol, or hydroxyazoles such as hydroxy benzotriazole. Especially preferred are N-hydroxy succinimides, with N-hydroxy succinimide and Sulfo-N-hydroxy succinimide being especially preferred. All alcohols may be employed alone or as suitable combination of two or more thereof. As reactive ester, pentafluorophenyl ester and N-hydroxy succinimide ester are especially preferred.

Thus, according to a preferred embodiment, the present invention relates to a method and a conjugate as described above, wherein the polymer derivative comprising Q, Q being an amino group -NH₂, is further reacted with formylbenzoic acid.

According to another embodiment, the present invention relates to a method and a conjugate as described above, wherein the polymer derivative comprising Q, Q being an amino group, is further reacted with formylbenzoic acid pentafluorophenyl ester.

According to yet another embodiment, the present invention relates to a method and a conjugate as described above, wherein the polymer derivative comprising Q, Q being an amino group, is further reacted with formylbenzoic acid N-hydroxysuccinimide ester.

According to yet another embodiment, the present invention relates to a method and a conjugate as described above, wherein the polymer derivative comprising Q, Q being an amino group, is further reacted with 4-(4-formyl-3,5-dimethoxyphenoxy)butyric acid.

As solvent for the reaction of the polymer derivative comprising an amino group and, e.g., formylbenzoic acid, at least one aprotic solvent, particularly preferably an anhydrous aprotic solvent having a water content of not more than 0.5 percent by weight, preferably of not more than 0.1 percent by weight is preferred. Suitable solvents are, among others, dimethyl sulfoxide (DMSO), N-methyl pyrrolidone, dimethyl acetamide (DMA), dimethyl formamide (DMF) and mixtures of two or more thereof.

The reaction is preferably carried out at a temperature of from 0 to 40 °C, more preferably of from 0 to 25 °C and especially preferably of from 15 to 25 °C for a reaction time preferably of from 0.5 to 24 h and especially preferably of from 1 to 17 h.

According to a preferred embodiment, the reaction is carried out in the presence of an activating agent. Suitable activating agents are, among others, carbodiimides such as diisopropyl carbodiimde (DIC), dicyclohexyl carbodiimides (DCC), 1-ethyl-3-(3-dimethylaminopropyl) carbodiimide (EDC), with diisopropyl carbodiimde (DIC) being especially preferred.

The resulting polymer derivative may be purified from the reaction mixture by at least one suitable method. If necessary, the polymer derivative may be precipitated prior to the isolation by at least one suitable method.

If the polymer derivative is precipitated first, it is possible, e.g., to contact the reaction mixture with at least one solvent or solvent mixture other than the solvent or solvent mixture present in the reaction mixture at suitable temperatures. According to a particularly preferred embodiment of the present invention where an aqueous medium, preferably water is used as solvent, the reaction mixture is contacted with 2-propanol or with am mixture of acetone and ethanol, preferably a 1:1 mixture (v/v), indicating equal volumes of said compounds, at a temperature, preferably in the range of from -20 to +50 °C and especially preferably in the range of from -20 to 25 °C.

Isolation of the polymer derivative may be carried out by a suitable process which may comprise one or more steps. According to a preferred embodiment of the present invention, the polymer derivative is first separated off the reaction mixture or the mixture of the reaction mixture with, e.g., aqueous 2-propanol mixture, by a suitable method such as centrifugation or filtration. In a second step, the separated polymer derivative may be subjected to a further treatment such as an after-treatment like dialysis, centrifugal filtration or pressure filtration, ion exchange chromatography, reversed phase chromatography, HPLC, MPLC, gel filtration and/or lyophilisation. According to an even more preferred embodiment, the separated polymer derivative is first dialysed, preferably against water, and then lyophilized until the solvent content of the reaction product is sufficiently low according to the desired specifications of the product. Lyophilisation may be carried out at temperature of from 20 to 35 °C, preferably of from 20 to 30 °C.

The resulting polymer derivative with the aldehyde group or keto group or hemiacetal group is subsequently reacted with an amino group of the protein via reductive amination. As to the coupling of at least one amino group of the protein with at least one aldehyde group or keto group or hemiacetal group of the polymer by reductive amination, reference is made to the detailed disclosure above concerning the specific reaction parameters of the reductive amination reaction such as pH or temperature. According to an especially preferred embodiment of the present invention, the reductive amination is carried out at a temperature of from 0 to 10 °C such as from 1 to 8 °C or from 2 to 6 °C such as about 4 °C at a pH of about 4.5 to 5.5 such as about 5.0. The reaction time is 10 to 20 h such as from 12 to 19 h or from 14 to 18 h such as 17 h or 20 to 30 h such as 24 h.

Thus, according to the above-mentioned preferred embodiments, the present invention also relates, in case the polymer was reacted via its oxidized reducing end, to a conjugate according to the formula

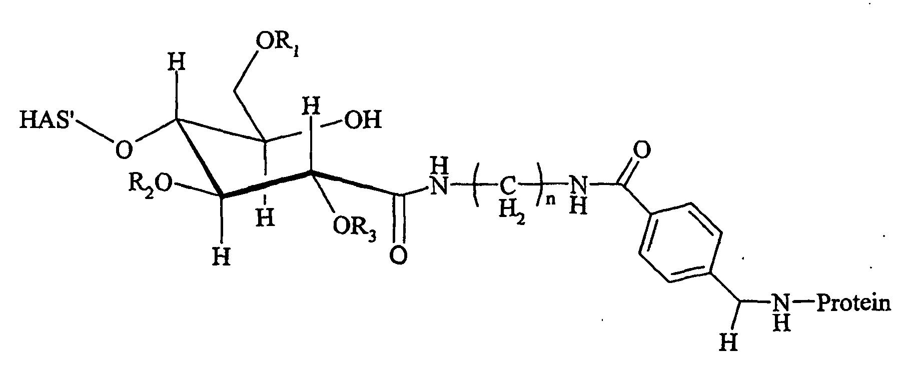

According to an especially preferred embodiment, the polymer is hydroxyethyl starch, i.e. HAS' is HES', and n = 2, 3, or 4, most preferably 4, as described above. Therefore, in case the polymer was reacted via its oxidized reducing end, the present invention also relates to a conjugate according to the formula

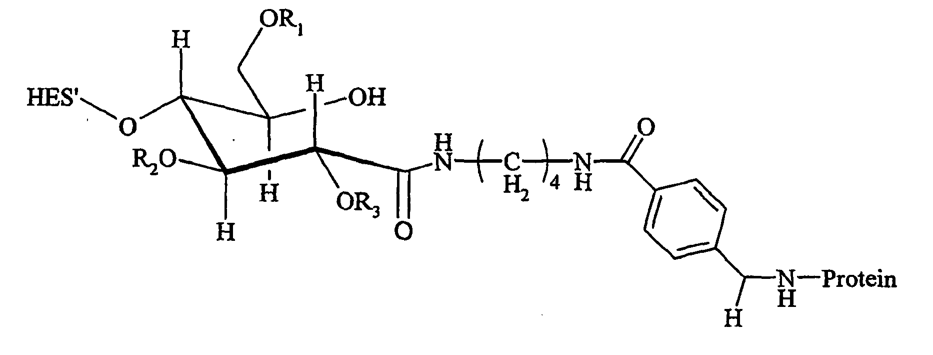

According to another preferred embodiment, the present invention also relates, in case the polymer was reacted via its oxidized reducing end, to a conjugate according to the formula 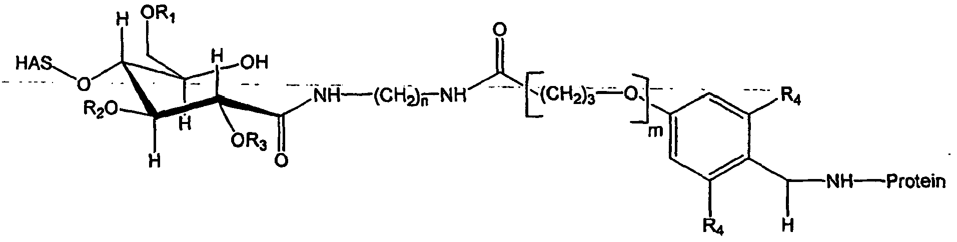 wherein n = 2, 3, or 4, R₄ being independently hydrogen or a methoxy group, and m = 0 in case R₄ is hydrogen and m =1 in case R₄ is methoxy, HAS preferably being HES'.

In each of the formulae above, the nitrogen attached to the protein derives from the amino group of the protein the polymer derivative is linked to via the aldehyde group.

With respect to the above-mentioned embodiments according to which the functional groups M and Q comprise an amino group -NH₂, it is also possible that M is an amino group -NH₂ and Q comprises a beta hydroxy amino group -CH(OH)-CH₂-NH₂ and preferably is a beta hydroxy amino group.

Therefore, the present invention also relates to a method and a conjugate as described above, wherein the amino group Q of the compound comprising two amino groups M and Q, is a beta hydroxy amino group -CH(OH)-CH₂-NH₂.

In this case, M and Q may be separated by any suitable spacer. Among others, the spacer may be an optionally substituted, linear, branched and/or cyclic hydrocarbon residue. Generally, the hydrocarbon residue has from 1 to 60, preferably from 1 to 40, more preferably from 1 to 20, more preferably from 2 to 10, more preferably from 1 to 6 and especially preferably from 1 to 2 carbon atoms. If heteroatoms are present, the separating group comprises generally from 1 to 20, preferably from 1 to 8 and especially preferably from 1 to 4 heteroatoms. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group. According to an even more preferred embodiment, the hydrocarbon residue is an alkyl chain of from 1 to 20, preferably from 1 to 10, more preferably from 1 to 6, more preferably from 1 to 4 carbon atoms and especially preferably from 1 to 2 carbon atoms. Still more preferably, M and Q are separated by a methylene group.

Therefore, the present invention also relates to a method and a conjugate as described above, wherein the polymer is reacted with 1,3-diamino-2-hydroxypropane.

In case the polymer is reacted via its oxidized reducing end, a polymer derivative according to the formula results 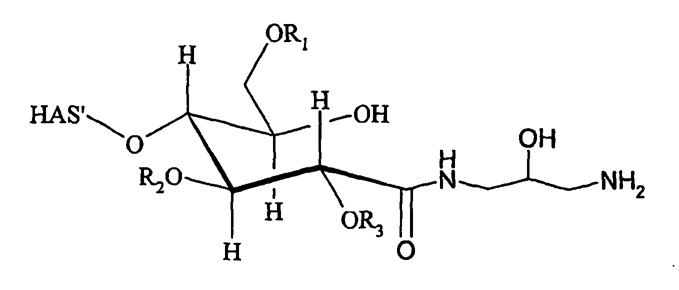 especially preferably with HAS' = HES'

The reaction of the at least bifunctional compound comprising M and Q, particularly preferably 1,3-diamino-2-hydroxypropane, with the polymer is preferably carried out at a temperature of from 40 to 120 °C, more preferably of from 40 to 90 °C and especially preferably of from 60 to 80 °C. The reaction time preferably ranges from 17 to 168 h, more preferably from 17 to 96 h and especially preferably from 48 to 96 h. The molar ratio of at least bifunctional compound : polymer is preferably in the range of from 200:1 to 10:1, specially from 50:1 to 100:1.

As solvent for the reaction of the at least bifunctional compound with the polymer, at least one aprotic solvent, preferably an anhydrous aprotic solvent having a water content of not more than 0.5 percent by weight, preferably of not more than 0.1 percent by weight is preferred. Suitable solvents are, among others, dimethyl sulfoxide (DMSO), N-methyl pyrrolidone, dimethyl acetamide (DMA), dimethyl formamide (DMF) and mixtures of two or more thereof.

The beta hydroxy amino group Q of the polymer derivative generally may be reacted with an at least bifunctional compound comprising at least one functional group capable of being reacted with Q and further comprising at least one functional group being an aldehyde group or keto group or hemiacetal group or a functional group capable of being modified to give an aldehyde group or keto group or hemiacetal group. According to another embodiment of the present invention, the beta hydroxy amino group is directly chemically modified to give an aldehyde group by chemical oxidation.

This oxidation may be carried with all suitable oxidation agents which are capable of converting the beta hydroxy amino group to an aldehyde group. Preferred oxidation reagents are periodates such as alkaline metal periodates. Especially preferred is sodium periodate which is preferably employed as aqueous solution. This solution has a preferred iodate concentration of from 1 to 50 mM, more preferably from 1 to 25 mM and especially preferably of from 1 to 10 mM. Oxidation is carried out at a temperature of from 0 to 40 °C, preferably from 0 to 25 °C and especially preferably from 4 to 20 °C.

The resulting polymer derivative may be purified from the reaction mixture by at least one suitable method. If necessary, the polymer derivative may be precipitated prior to the isolation by at least one suitable method.

If the polymer derivative is precipitated first, it is possible, e.g., to contact the reaction mixture with at least one solvent or solvent mixture other than the solvent or solvent mixture present in the reaction mixture at suitable temperatures. According to a particularly preferred embodiment of the present invention where an aqueous medium, preferably water is used as solvent, the reaction mixture is contacted with 2-propanol or with am mixture of acetone and ethanol, preferably a 1:1 mixture (v/v), indicating equal volumes of said compounds, at a temperature, preferably in the range of from -20 to +50 °C and especially preferably in the range of from -20 to 25 °C.

Isolation of the polymer derivative may be carried out by a suitable process which may comprise one or more steps. According to a preferred embodiment of the present invention, the polymer derivative is first separated off the reaction mixture or the mixture of the reaction mixture with, e.g., aqueous 2-propanol mixture, by a suitable method such as centrifugation or filtration. In a second step, the separated polymer derivative may be subjected to a further treatment such as an after-treatment like dialysis, centrifugal filtration or pressure filtration, ion exchange chromatography, reversed phase chromatography, HPLC, MPLC, gel filtration and/or lyophilisation. According to an even more preferred embodiment, the separated polymer derivative is first dialysed, preferably against water, and then lyophilized until the solvent content of the reaction product is sufficiently low according to the desired specifications of the product. Lyophilisation may be carried out at temperature of from 20 to 35 °C, preferably of from 20 to 30 °C.

Therefore, the present invention also relates to a method and a conjugate as described above, wherein the oxidation of the beta hydroxy amino group Q is carried out using a periodate.

Therefore, the present invention also relates to a method of producing a conjugate, wherein, in case the polymer was employed with oxidized reducing end, a polymer derivative having a beta hydroxy amino group, especially preferably 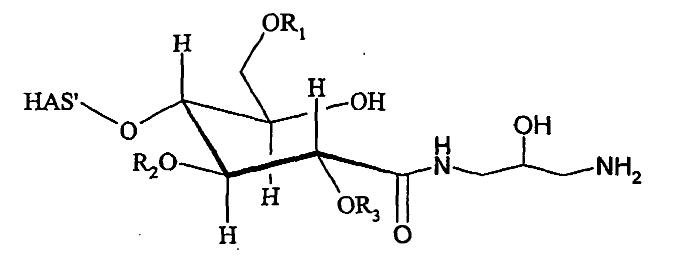 and particularly with HAS' = HES', is oxidized, preferably with a periodate, to a polymer derivative having an aldehyde group, especially preferably 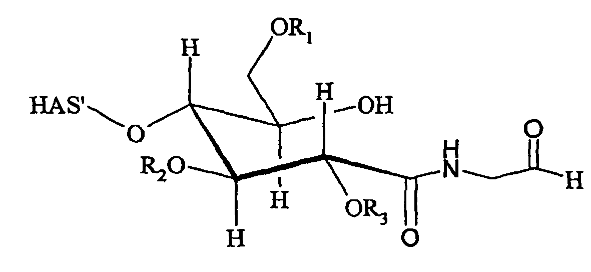 and particularly with HAS' = HES'.

The resulting polymer derivative with the aldehyde group A is subsequently reacted with the protein. Therefore, the present invention also relates to as method of producing a conjugate, said method comprising reacting a polymer derivative having a beta hydroxy amino group, in case the polymer was employed with oxidized reducing end especially preferably according to the formula 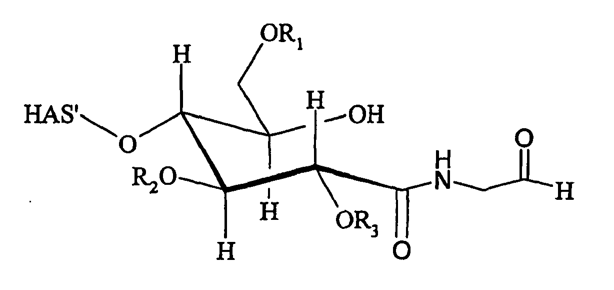 and particularly with HAS' = HES', with an amino group of the protein.

The resulting polymer derivative with the aldehyde group is subsequently reacted with an amino group of the protein via reductive amination. As to the coupling of at least one amino group of the protein with at least one aldehyde group of the polymer by reductive amination, reference is made to the detailed disclosure above.

Thus, according to the above-mentioned preferred embodiment, the present invention also relates to a conjugate according to the formula 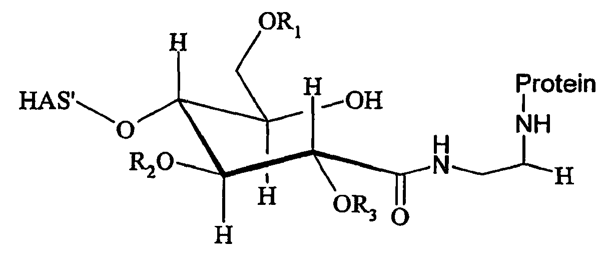 particularly with HAS' = HES', in case the polymer was employed with oxidized reducing end. In the formula above, the nitrogen attached to the protein derives from the amino group of the protein the polymer derivative is linked to via the aldehyde group.

According to a further embodiment of the present invention, the polymer is first reacted with a suitable compound to give a first polymer derivative comprising at least one reactive carboxy group. This first polymer derivative is then reacted with a further, at least bifunctional compound wherein at least one functional group of this further compound is reacted with at least one reactive carboxy group of the polymer derivative and at least one other functional group of the further compound is an aldehyde group or keto group or hemiacetal group or is a functional group which is chemically modified to give an aldehyde group or keto group or hemiacetal group, and wherein the resulting polymer derivative comprising said aldehyde group or keto group or hemiacetal group is reacted via reductive amination, as described above, with at least one amino group of the protein. It is also possible to alter the sequence of reacting the respective compounds with each other.

According to a first alternative of said further embodiment, the polymer comprising at least one reactive carboxy group is prepared by selectively oxidizing the polymer at its reducing end and subsequently reacting the oxidized polymer being a lactone 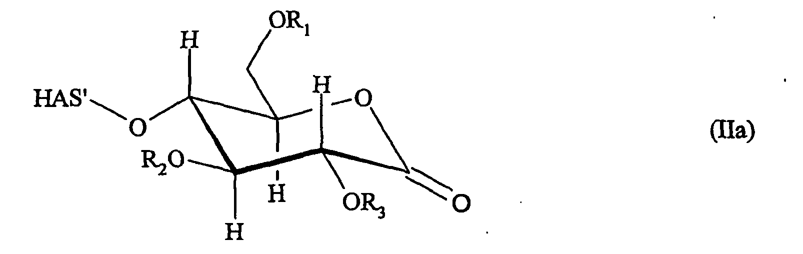 and/or a carboxylic acid 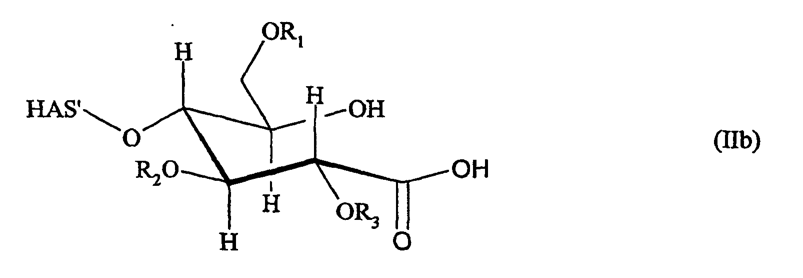 or a suitable salt of the carboxylic acid such as alkali metal salt, preferably as sodium and/or potassium salt, and HAS' preferably being HES', with a suitable compound to give the polymer comprising at least one reactive carboxy group.

Oxidation of the polymer, preferably hydroxyethyl starch, may be carried out according to each method or combination of methods which result in compounds having the above-mentioned structures (IIa) and/or (IIb).

Although the oxidation may be carried out according to all suitable method or methods resulting in the oxidized reducing end of hydroxyalkyl starch, it is preferably carried out using an alkaline iodine solution as described, e.g., in DE 196 28 705 A1 (example A, column 9, lines 6 to 24).

Introducing the reactive carboxy group into the polymer which is selectively oxidized at its reducing end may carried out by all conceivable methods and all suitable compounds.

According to a specific method of the present invention, the polymer which is selectively oxidized at its reducing end is reacted at the oxidized reducing end with at least one alcohol, preferably with at least one acidic alcohol such as acidic alcohols having a pK_{A} value in the range of from 6 to 12 or of from 7 to 11 at 25 °C. The molecular weight of the acidic alcohol may be in the range of from 80 to 500 g/mole, such as of from 90 to 300 g/mole or of from 100 to 200 g/mole.

Suitable acidic alcohols are all alcohols H-O-R_{A} having an acidic proton and are capable of being with reacted with the oxidized polymer to give the respective reactive polymer ester, preferably according to the formula 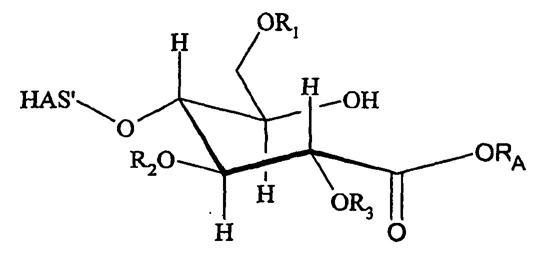 still more preferably according to formula

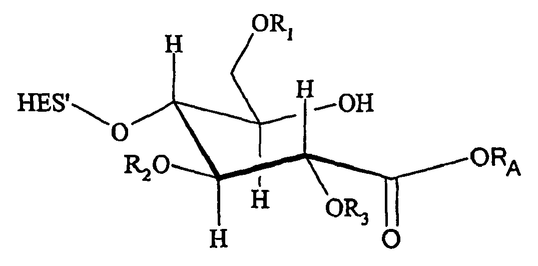

Preferred alcohols are N-hydroxy succinimides such as N-hydroxy succinimde or Sulfo-N-hydroxy succinimide, suitably substituted phenols such as p-nitrophenol, o,p-dinitrophenol, o,o'-dinitrophenol, trichlorophenol such as 2,4,6-trichlorophenol or 2,4,5-trichlorophenol, trifluorophenol such as 2,4,6-trifluorophenol or 2,4,5-trifluorophenol, pentachlorophenol, pentafluorophenol, or hydroxyazoles such as hydroxy benzotriazole. Especially preferred are N-hydroxy succinimides, with N-hydroxysuccinimide and Sulfo-N-hydroxysuccinimide being especially preferred. All alcohols may be employed alone or as suitable combination of two or more thereof. In the context of the present invention, it is also possible to employ a compound which releases the respective alcohol, e.g. by adding diesters of carbonic acid.

Therefore, the present invention also relates to a method as described above, wherein the polymer which is selectively oxidised at its reducing end is activated by reacting the oxidised polymer with an acidic alcohol, preferably with N-hydroxy succinimide and/or Sulfo-N-hydroxy succinimide.

According to a preferred embodiment of the present invention, the polymer which is selectively oxidized at its reducing end is reacted at the oxidized reducing end with at least one carbonic diester R_{B}-O-(C=O)-O-R_{C}, wherein R_{B} and R_{C} may be the same or different. Preferably, this method gives reactive polymers according to the formula 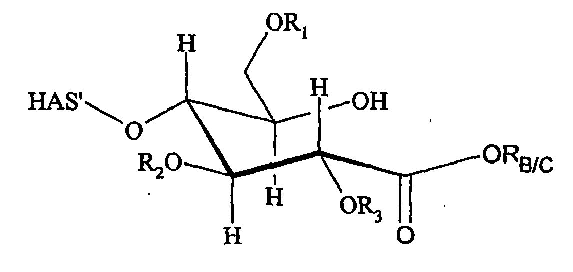 wherein HAS' is preferably HES'.

As suitable carbonic diester compounds, compounds may be employed whose alcohol components are independently N-hydroxy succinimides such as N-hydroxy succinimde or Sulfo-N-hydroxy succinimide, suitably substituted phenols such as p-nitrophenol, o,p-dinitrophenol, o,o'-dinitrophenol, trichlorophenol such as 2,4,6-trichlorophenol or 2,4,5-trichlorophenol, trifluorophenol such as 2,4,6-trifluorophenol or 2,4,5-trifluorophenol, pentachlorophenol, pentafluorophenol, or hydroxyazoles such as hydroxy benzotriazole. Especially preferred are N,N'-disuccinimidyl carbonate and Sulfo-N,N'-disuccinimidyl carbonate, with N,N'-disuccinimidyl carbonate being especially preferred.

Therefore, the present invention also relates a method as described above, wherein the polymer which is selectively oxidised at its reducing end is activated by reacting the oxidised polymer with N,N'-disuccinimidyl carbonate.

The acidic alcohol is reacted with the oxidized polymer or the salt of the oxidized polymer at a molar ratio of acidic alcohol : polymer preferably of from 5:1 to 50:1, more preferably of from 8:1 to 20:1, at a preferred reaction temperature of from 2 to 40 °C, more preferably of from 10 to 30 °C and especially preferably of from 15 to 25 °C. The reaction time is preferably in the range of from 1 to 10 h, more preferably of from 2 to 5 h, more preferably of from 2 to 4 h and particularly of from 2 to 3 h.

The carbonic diester compound is reacted with the oxidized polymer or the salt of the oxidized polymer at a molar ratio of diester compound : polymer generally of from 1:1 to 3:1, such as of from 1:1 to 1.5:1. The reaction time is generally in the range of from 0.1 to 12 h, like of from 0.2 to 6 h, or of from 0.5 to 2 h or of from 0.75 to 1.25 h.

According to a preferred embodiment of the present invention, reacting the oxidized polymer with acidic alcohol and/or carbonic diester is carried out in at least one aprotic solvent, such as in an anhydrous aprotic solvent having a water content of not more than 0.5 percent by weight, preferably of not more than 0.1 percent by weight. Suitable solvents are, among others, dimethyl sulfoxide (DMSO), N-methyl pyrrolidone, dimethyl acetamide (DMA), dimethyl formamide (DMF) and mixtures of two or more thereof. The reaction temperatures are preferably in the range of from 2 to 40 °C, more preferably of from 10 to 30 °C.

For reacting the oxidized polymer with the at least one acidic alcohol, at least one additional activating agent is employed.

Suitable activating agents are, among others, carbonyldiimidazole, carbodiimides such as diisopropyl carbodiimde (DIC), dicyclohexyl carbodiimides (DCC), I-ethyl-3-(3-dimethylaminopropyl) carbodiimide (EDC), with dicyclohexyl carbodiimides (DCC) and 1-ethyl-3-(3-dimethylaminopropyl) carbodiimide (EDC) being especially preferred.

Therefore, the present invention also relates to the method as described above, where the Polymer which is oxidized at its reducing end, is reacted with an acidic alcohol in the presence of an additional activating agent to give the reactive polymer ester.

According to one embodiment of the present invention, the reaction of the oxidized polymer with carbonic diester and/or acidic alcohol is carried out at a low base activity which may be determined by adding the reaction mixture to water with a volume ratio of water to reaction mixture of 10:1. Prior to the addition, the water which comprises essentially no buffer, has a pH value of 7 at 25 °C. After the addition of the reaction mixture and by measuring the pH value, the base activity of the reaction mixture is obtained, having a value of preferably not more than 9.0, more preferably of nor more than 8.0 and especially preferably of not more than 7.5.

According to another embodiment of the present invention, the oxidized polymer is reacted with N-hydroxy succinimide in dry DMA in the absence of water with EDC to selectively give the polymer N-hydroxy succinimide ester according to the formula 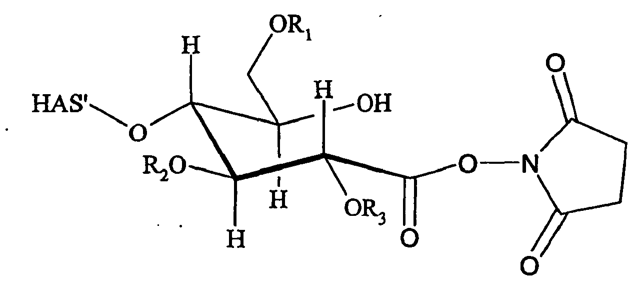 more preferably with HAS' being HES'.

Surprisingly, this reaction does not give by-products resulting from reactions of EDC with OH groups of HES, and the rearrangement reaction of the O-acyl isourea formed by EDC and the oxidized polymer to the respective N-acyl urea is surprisingly suppressed.

According to another preferred embodiment of the present invention, the oxidized polymer is reacted with N,N'-disuccinimidyl carbonate in dry DMF in the absence of water and in the absence of an activating agent to selectively give the polymer N-hydroxy succinimide ester according to the formula 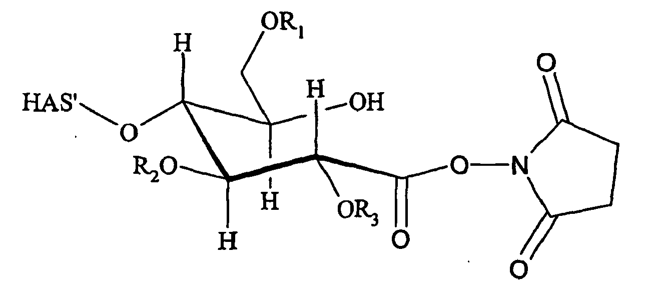 more preferably with HAS' being HES'.

According to another embodiment of the present invention, the polymer which is selectively oxidized at its reducing end is reacted at the oxidized reducing end with an azolide such as carbonyldiimidazole or carbonyl dibenzimidazole to give a polymer having a reactive carboxy group. In the case of carbonyldiimidazole, a reactive imidazolide polymer derivative according to formula 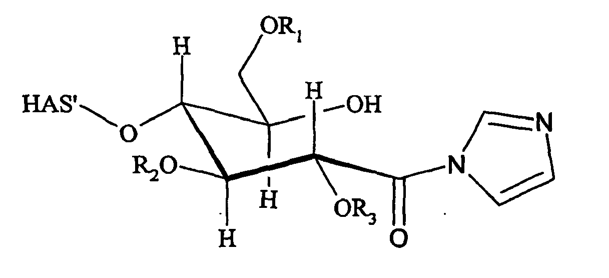 results, wherein HAS' is preferably HES'.

According to a second alternative of said further embodiment of the present invention regarding the introduction of at least one reactive carboxy group into the polymer, the reactive carboxy group is introduced into the polymer whose-reducing end is not oxidized, by reacting at least one hydroxy group of the polymer with a carbonic diester.

Therefore, the present invention also relates to a method and conjugates wherein the reactive carboxy group is introduced in the polymer whose reducing end is not oxidized, by reacting at least one hydroxy group of the polymer with at least one carbonic diester carbonic diester R_{B}-O-(C=O)-O-R_{C}, wherein R_{B} and R_{C} may be the same or different.

According to another embodiment of the present invention, the polymer whose reducing end is not oxidized, is reacted at at least one hydroxy group with an azolide such as carbonyldiimidazole, carbonyl-di-(1,2,4-triazole) or carbonyl dibenzimidazol to give a polymer having a reactive carboxy group.

As suitable carbonic diester compounds, compounds may be employed whose alcohol components are independently N-hydroxy succinimides such as N-hydroxy succinimde or Sulfo-N-hydroxy succinimide, suitably substituted phenols such as p-nitrophenol, o,p-dinitrophenol, o,o'-dinitrophenol, trichlorophenol such as 2,4,6-trichlorophenol or 2,4,5-trichlorophenol, trifluorophenol such as 2,4,6-trifluorophenol or 2,4,5-trifluorophenol, pentachlorophenol, pentafluorophenol, or hydroxyazoles such as hydroxy benzotriazole.

Especially preferred are symmetrical carbonic diester compounds, R_{B} and R_{C} thus being the same. The alcohol component of the carbonic diester is preferably selected from the group consisting of N-hydroxy succinimide, sulfonated N-hydroxy succinimide, N-hydroxy benzotriazole, and nitro- and halogen-substituted phenols. Among others, nitrophenol, dinitrophenol, trichlorophenol, trifluorophenol, pentachlorophenol, and pentafluorophenol are preferred. Especially preferred are N,N'-disuccinimidyl carbonate and Sulfo-N,N'-disuccinimidyl carbonate, with N,N'-disuccinimidyl carbonate being especially preferred.

Therefore, the present invention also relates to a hydroxyalkyl starch derivative, preferably a hydroxyethyl starch derivative, wherein at least one hydroxy group, preferably at least two hydroxy groups of said starch have been reacted with a carbonic diester compound to give the-respective reactive ester.

According to one embodiment of the present invention, the reaction of the polymer whose reducing end is not oxidized, with the at least one carbonic diester compound is carried out at a temperature of from 2 to 40 °C, more preferably of from 10 to 30 °C and especially of from 15 to 25 °C. A preferred reaction time ranges from 0,5 to 5 h, more preferably from 1 to 3 h, and especially preferably from 2 to 3 h.

The molar ratio of carbonic diester compound : polymer depends on the degree of substitution of the polymer regarding the number of hydroxy groups reacted with carbonic diester compound relative to the number of hydroxy groups present in the non-reacted polymer.

According to one embodiment of the present invention, the molar ratio of carbonic diester compound : anhydroglucose units of the polymer is in the range of from 1:2 to 1:1000, more preferably of from 1:3 to 1:100 and especially preferably of from 1:10 to 1:50, to give a degree of substitution in the range of from 0.5 to 0.001, preferably of from 0.33 to 0.01 and especially preferably of from 0.1 to 0.02

According to one embodiment of the present invention, reacting the polymer whose reducing end is not oxidized, with carbonic diester is carried out in at least one aprotic solvent, particularly preferably in an anhydrous aprotic solvent having a water content of not more than 0.5 percent by weight, preferably of not more than 0.1 percent by weight. Suitable solvents are, among others, dimethyl sulfoxide (DMSO), N-methyl pyrrolidone, dimethyl acetamide (DMA), dimethyl formamide (DMF) and mixtures of two or more thereof.

Therefore, the present invention also relates to a method as described above wherein the reaction of the at least one hydroxy group of the polymer whose reducing end is not oxidised, with the carbonic diester to give a reactive carboxy group is carried out in an anhydrous aprotic polar solvent, the solvent preferably being dimethyl acetamide, dimethyl formamide or a mixture thereof.

The reactive polymer derivative comprising at least one reactive carboxy group, preferably resulting from the reaction of the polymer with the acidic alcohol, the carbonate and/or the azolide, as described above, is further reacted with a further, at least bifunctional compound wherein at least one functional group F₁ of this further compound is reacted with at least one reactive carboxy group of the polymer derivative. As at least one functional group F₁ of the further compound no specific limitations exist given that a reaction with the at least one reactive carboxy group of the polymer is possible. Preferred functional groups F₁ are, e.g., an amino group or a hydroxy group or a thio group or a carboxy group.

The further, at least bifunctional compound comprises at least one other functional group F₂ being an aldehyde group or a functional group F₂ being capable of being chemically modified to give an aldehyde group. The chemical modification may be, e.g., a reaction of the functional group F₂ with a functional group F₃ a further linker compound or an oxidation or a reduction of a suitable functional group F₂.

In case F₂ is reacted with a functional group F₃ of a further compound, the functional group F₂ may be selected from, among others,
- C-C-double bonds or C-C-triple bonds or aromatic C-C-bonds;
- the thio group or the hydroxy group;
- alkyl sulfonic acid hydrazide, aryl sulfonic acid hydrazide;
- 1,2-dioles;
- 1,2-aminoalcohols;
- 1,2 amino-thioalcohols;
- azides;
- the amino group -NH₂ or derivatives of the amino groups comprising the structure unit -NH- such as aminoalkyl groups, aminoaryl group, aminoaralkyl groups, or alkarlyaminogroups;
- the hydroxylamino group -O-NH₂, or derivatives of the hydroxylamino group comprising the structure unit -O-NH-, such as hydroxylalkylamino groups, hydroxylarylamino groups, hydroxylaralkylamino groups, or hydroxalalkarylamino groups;
- alkoxyamino groups, aryloxyamino groups, aralkyloxyamino groups, or alkaryloxyamino groups, each comprising the structure unit NH-O-;
- residues having a carbonyl group, -Q-C(=G)-M, wherein G is O or S, and M is, for example,
   -OH or -SH;
   -- an alkoxy group, an aryloxy group, an aralkyloxy group, or an alkaryloxy group;
   -- an alkylthio group, an arylthio group, an aralkylthio group, or an alkarylthio group;
   -- an alkylcarbonyloxy group, an arylcarbonyloxy group, an aralkylcarbonyloxy group, an alkarylcarbonyloxy group;
   -- activated esters such as esters of hydroxylamines having imid structure such as N-hydroxysuccinimide or having a structure unit O-N where N is part of a heteroaryl compound or, with G = O and Q absent, such as aryloxy compounds with a substituted aryl residue such as pentafluorophenyl, paranitrophenyl or trichlorophenyl;
   wherein Q is absent or NH or a heteroatom such as S or O;
- -NH-NH₂, or -NH-NH-;
- -NO₂;
- the nitril group;
- carbonyl groups such as the aldehyde group or the keto group;
- the carboxy group;
- the -N=C=O group or the -N=C=S group;
- vinyl halide groups such as the vinyl iodide or the vinyl bromide group or triflate;
- -C=C-H;
- -(C=NH₂Cl)-OAlkyl
- groups -(C=O)-CH₂-Hal wherein Hal is Cl, Br, or I;
- -CH=CH-SO₂-;
- a disulfide group comprising the structure -S-S-;
- the group 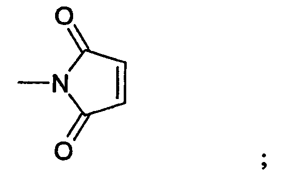
- the group 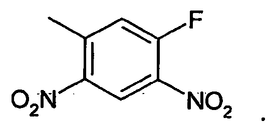
wherein F₃ is a group capable of forming a chemical linkage with one of the above-mentioned groups and is preferably selected from the above-mentioned groups. Moreover, the second linker compound preferably has at least one aldehyde group or keto group or hemiacetal group which is capable of being reacted with an amino group of the protein via reductive amination.

The functional group F₁ and the aldehyde group or keto group or hemiacetal group of the at least bifunctional linking compound which is reacted with the polymer, and/or the functional groups F₁ and F₂ of the at least bifunctional linking compound which is reacted with the polymer, and/or the functional group F₃ and the aldehyde group or keto group or hemiacetal group of the further, at least bifunctional linking compound, may be independently separated by any suitable spacer. Among others, the spacer may be an optionally substituted, linear, branched and/or cyclic, aliphatic and/or aromatic hydrocarbon residue. Generally, the hydrocarbon residue has up to 60, preferably up to 40, more preferably up to 20, more preferably up to 10 carbon atoms. If heteroatoms are present, the separating group comprises generally from 1 to 20, preferably from 1 to 8, more preferably 1 to 6, more preferably 1 to 4 and especially preferably from 1 to 2 heteroatoms. As heteroatom, O is preferred. The hydrocarbon residue may comprise an optionally branched alkyl chain or an aryl group or a cycloalkyl group having, e.g., from 5 to 7 carbon atoms, or be an aralkyl group, an alkaryl group where the alkyl part may be a linear and/or cyclic alkyl group.

Examples of a compound with functional groups F₁ and F₂ are, e.g., optionally substituted diaminoalkane having from 2 to 20 carbon atoms, especially preferably 1,2-diaminoethane, 1,3-diaminopropane, and 1,4-diaminobutane. Preferred examples of a compound with functional groups F₃ and an aldehyde group or a keto group or a hemiacetal group are, e.g., formylbenzoic acid, 4-formylbenzoic acid pentafluorophenyl ester, 4-formylbenzoic acid-N-hydroxysuccinimide ester and 4-(4-formyl-3,5-dimethoxyphenoxy)butyric acid.

Therefore, the present invention also relates to a method of producing a conjugate, said method comprising reacting the polymer, preferably hydroxyethyl starch, at its optionally oxidized reducing end with a compound, selected from the group consisting of acidic alcohols, carbonic diesters and azolides, to give a polymer derivative comprising at least one reactive carboxy group, reacting said polymer derivative with at least one at least bifunctional compound to give a polymer derivative comprising an aldehyde group or a keto group or a hemiacetal group or a functional group capable of being chemically modified to give an aldehyde group or a keto group or a hemiacetal group, optionally chemically modifying said functional group to give a polymer derivative comprising an aldehyde group or a keto group or a hemiacetal group, and reacting the polymer derivative comprising an aldehyde group or a keto group or a hemiacetal group with an amino group of a protein via reductive amination.

Accordingly, the present invention also relates to a conjugate comprising a polymer, preferably hydroxyethyl starch, and a protein covalently linked to each other, obtainable by a method of producing a conjugate, said method comprising reacting the polymer, at its optionally oxidized reducing end with a compound, selected from the group consisting of acidic alcohols, carbonic diesters and azolides, to give a polymer derivative comprising at least one reactive carboxy group, reacting said polymer derivative with at least one at least bifunctional compound to give a polymer derivative comprising an aldehyde group or a keto group or a hemiacetal group or a functional group capable of being chemically modified to give an aldehyde group or a keto group or a hemiacetal group, optionally chemically modifying said functional group to give a polymer derivative comprising an aldehyde group or a keto group or a hemiacetal group, and reacting the polymer derivative comprising an aldehyde group or a keto group or a hemiacetal group with an amino' group of a protein via reductive amination.

A specific example of a compound having a functional group F₁ and a functional group F₂ which is oxidized to give an aldehyde group is, e.g., a compound having an amino group as F₁ and a beta hydroxy amino group as F₂. An especially preferred example is 1,3-diamino-2-hydroxypropane. This oxidation may be carried with all suitable oxidation agents which are capable of converting the beta hydroxy amino group to an aldehyde group. Preferred oxidation reagents are periodates such as alkaline metal periodates. Especially preferred is sodium periodate which is preferably employed as aqueous solution. This solution has a preferred iodate concentration of from 1 to 50 mM, more preferably from 1 to 25 mM and especially preferably of from 1 to 10 mM. Oxidation is carried out at a temperature of from 0 to 40 °C, preferably from 0 to 25 °C and especially preferably from 4 to 20 °C.

The resulting polymer derivative may be purified from the reaction mixture by at least one suitable method. If necessary, the polymer derivative may be precipitated prior to the isolation by at least one suitable method.

If the polymer derivative is precipitated first, it is possible, e.g., to contact the reaction mixture with at least one solvent or solvent mixture other than the solvent or solvent mixture present in the reaction mixture at suitable temperatures. According to a particularly preferred embodiment of the present invention where an aqueous medium, preferably water is used as solvent, the reaction mixture is contacted with 2-propanol or with am mixture of acetone and ethanol, preferably a 1:1 mixture (v/v), indicating equal volumes of said compounds, at a temperature, preferably in the range of from -20 to +50 °C and especially preferably in the range of from -20 to 25 °C.

Isolation of the polymer derivative may be carried out by a suitable process which may comprise one or more steps. According to a preferred embodiment of the present invention, the polymer derivative is first separated off the reaction mixture or the mixture of the reaction mixture with, e.g., aqueous 2-propanol mixture, by a suitable method such as centrifugation or filtration. In a second step, the separated polymer derivative may be subjected to a further treatment such as an after-treatment like dialysis, centrifugal filtration or pressure filtration, ion exchange chromatography, reversed phase chromatography, HPLC, MPLC, gel filtration and/or lyophilisation. According to an even more preferred embodiment, the separated polymer derivative is first dialysed, preferably against water, and then lyophilized until the solvent content of the reaction product is sufficiently low according to the desired specifications of the product Lyophilisation may be carried out at temperature of from 20 to 35 °C, preferably of from 20 to 30 °C.

The present invention also relates to a conjugate, comprising a protein and a polymer or a derivative thereof, wherein the polymer is a hydroxyalkyl starch (HAS) and the protein is a granulocyte colony stimulating factor (G-CSF), having a structure according to the formula 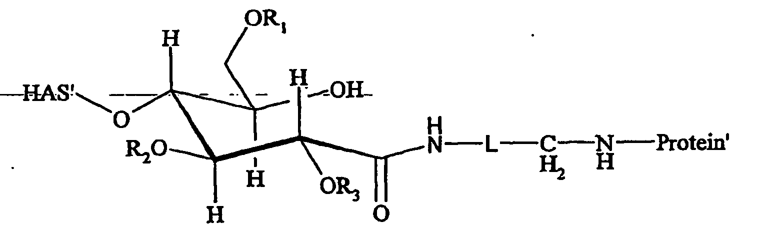 wherein R₁, R₂ and R₃ are independently hydrogen or a hydroxyalkyl group, a hydroxyaryl group, a hydroxyaralkyl group or a hydroxyalkaryl group having of from 1 to 10 carbon atoms, preferably hydrogen or a hydroxyalkyl group, more preferably hydrogen or a hydroxyethyl group, and
wherein L is an optionally substituted, linear, branched and/or cyclic hydrocarbon residue, optionally comprising at least one heteroatom, having from 1 to 60 preferably from 1 to 40, more preferably from 1 to 20, more preferably from 1 to 10, more preferably from 1 to 6 more preferably from 1 to 2 carbon atoms and especially preferably 1 carbon atom, L being in particular CH₂.

The abbreviation "Protein' " as used in the formula above refers to the G-CSF molecule used for the reaction without the nitrogen atom of the amino group which is part the aminomethyl linkage.

The present invention also relates to a conjugate, comprising a protein and a polymer or a derivative thereof, wherein the polymer is a hydroxyalkyl starch (HAS) and the protein is a granulocyte colony stimulating factor (G-CSF), having a structure according to the formula 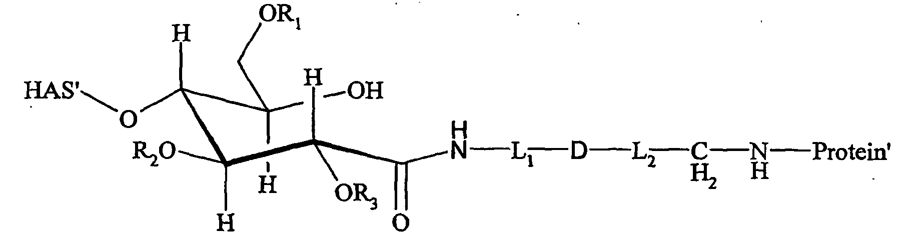 wherein R₁, R₂ and R₃ are independently hydrogen or a hydroxyalkyl group, a hydroxyaryl group, a hydroxyaralkyl group or a hydroxyalkaryl group having of from 1 to 10 carbon atoms, preferably hydrogen or a hydroxyalkyl group, more preferably hydrogen or a hydroxyethyl group, and
wherein L₁ and L₂ are independently an optionally substituted, linear, branched and/or cyclic hydrocarbon residue, optionally comprising at least one heteroatom, comprising an alkyl, aryl, aralkyl heteroalkyl, and/or heteroaralkyl moiety, said residue having from 1 to 60 preferably from 1 to 40, more preferably from 1 to 20, more preferably from 1 to 10 carbon atoms, and
wherein D is a linkage, preferably a covalent linkage which was formed by a suitable functional group F² linked to L₁ and a suitable functional group F₃ linked to L₂.

The abbreviation "Protein' " as used in the formulae above refers to the G-CSF molecule used for the reaction without the nitrogen atom of the amino group which is part of the aminomethyl linkage.

The present invention also relates to a conjugate as describe above, wherein L₁ is - (CH₂)n- with n = 2, 3, 4, 5, 6, 7, 8, 9, 10, preferably 2, 3, 4, 5, 6, more preferably 2, 3, 4, and especially preferably 4.

The present invention also relates to a conjugate as described above, wherein L₂ comprises an optionally suitably substituted aryl moiety, preferably an aryl moiety containing 6 carbon atoms, L₂ being especially preferably C₆H₄, or wherein L₂ is -(CH₂)n- with n = 2, 3, 4, 5, 6, 7, 8, 9, 10, preferably 2, 3, 4, 5, 6, more preferably 2, 3, 4.

The present invention also relates to a conjugate as described above, wherein is selected from the group consisting of
- C-C-double bonds or C-C-triple bonds or aromatic C-C-bonds;
- the thio group or the hydroxy groups;
- alkyl sulfonic acid hydrazide, aryl sulfonic acid hydrazide;
- 1,2-dioles;
- 1,2 amino-thioalcohols;
- azides;
- 1,2-aminoalcohols;
- the amino group -NH₂ or derivatives of the amino groups comprising the structure unit -NH- such as aminoalkyl groups, aminoaryl group, aminoaralkyl groups, or alkarlyaminogroups;
- the hydroxylamino group -O-NH₂, or derivatives of the hydroxylamino group comprising the structure unit -O-NH-, such as hydroxylalkylamino groups, hydroxylarylamino groups, hydroxylaralkylamino groups, or hydroxalalkarylamino groups;
- alkoxyamino groups, aryloxyamino groups, aralkyloxyamino groups, or alkaryloxyamino groups, each comprising the structure unit -NH-O-;
- residues having a carbonyl group, -Q-C(=G)-M, wherein G is O or S, and M is, for example,
   -- -OH or -SH;
   -- an alkoxy group, an aryloxy group, an aralkyloxy group, or an alkaryloxy group;
   -- an alkylthio group, an arylthio group, an aralkylthio group, or an alkarylthio group;
   -- an alkylcarbonyloxy group, an arylcarbonyloxy group, an aralkylcarbonyloxy group, an alkarylcarbonyloxy group;
   -- activated esters such as esters of hydroxylamines having imid structure such as N-hydroxysuccinimide or having a structure unit O-N where N is part of a heteroaryl compound or, with G = O and Q absent, such as aryloxy compounds with a substituted aryl residue such as pentafluorophenyl, paranitrophenyl or trichlorophenyl;
   wherein Q is absent or NH or a heteroatom such as S or O;
- -NH-NH₂, or -NH-NH-;
- -NO₂;
- the nitril group;
- carboxyl groups such as the aldehyde group or the keto group;
- the carboxy group;
- the -N=C=O group or the -N=C=S group;
- vinyl halide groups such as the vinyl iodide or the vinyl bromide group or triflate;
- -C=C-H;
- -(C=NH₂Cl)-OAlkyl
- groups -(C=O)-CH₂-Hal wherein Hal is Cl, Br, or I;
- -CH=CH-SO₂-;
- a disulfide group comprising the structure -S-S-;
- the group 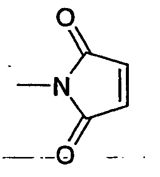
- the group 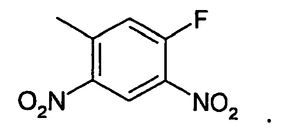
and wherein F₃ is a functional group capable of forming a chemical linkage with F₂ and is preferably selected from the above-mentioned group, F₂ preferably comprising the moiety -NH-, more preferably comprising an amino group, F₃ preferably comprising the moiety -(C=G)-, more preferably -(C=O)-, more preferably the moiety -(C=G)-G-, still more preferably -(C=O)-G-, and especially preferably - (C=O)-O, D being particularly preferably an amide linkage.

The present invention also relates to any conjugate as described above, wherein the hydroxyalkyl starch is hydroxyethyl starch.

The present invention also relates to any conjugate as described above, wherein the hydroxyethyl starch has a molecular weight of from 2 to 200 kD, preferably of from 4 to 130 kD, more preferably of from 4 to 70 kD.

According to a further aspect, the present invention relates to a conjugate as described above, or a conjugate, obtainable by a method as described above, for use in a method for the treatment of the human or animal body.

Furthermore, the present invention relates to a pharmaceutical composition comprising in a therapeutically effective amount a conjugate as described above or a conjugate, obtainable by a method as described above.

The term "in a therapeutically effective amount" as used in the context of the present invention refers to that amount which provides therapeutic effect for a given condition and administration regimen. The administration is preferably by routes. The specific route chosen will depend upon the condition being treated. The administration is preferably done as part of a formulation containing a suitable carrier, such as polysorbat, a suitable diluent, such as water and/or a suitable adjuvant such as sorbitol. The required dosage will depend upon the severity of the condition being treated, the patients individual response, the method of administration used, and the like.

Thus, in a preferred embodiment, the pharmaceutical composition further comprises at least one pharmaceutically acceptable diluent, adjuvant and/or carrier, especially preferably useful in G-CSF therapy.

The pharmaceutical composition is preferably used for the treatment of a disorder characterized by a reduced hematopoietic or immune function or diseases related thereto.

Therefore, the present invention also relates to the use of a pharmaceutical composition as described above, comprising a conjugate as described above or a conjugate, obtainable by a method as described above, for the preparation of a medicament for the treatment of a disorder characterized by a reduced hematopoietic or immune function.

According to a preferred embodiment, the disorder characterized by a reduced hematopoietic or immune function, is a result of chemotherapy, radiation therapy, infectious disease, severe chronic neutropenia, or leukemia. Therefore, the present invention also relates to the use of a pharmaceutical composition as described above, comprising a conjugate as described above or a conjugate, obtainable by a method as described above, for the preparation of a medicament for the treatment of a disorder characterized by a reduced hematopoietic or immune function, wherein the disorder is a result of chemotherapy, radiation therapy, infectious disease, severe chronic neutropenia, or leukemia.

The object of the treatment with the pharmaceutical composition according to the invention is preferably administered by i.v. or s.c. routes. For this, the pharmaceutical composition may be administered as a sterile solution.

The invention is further illustrated by the following figures, tables and examples.

### Short description of the Figures

### Figure 1a

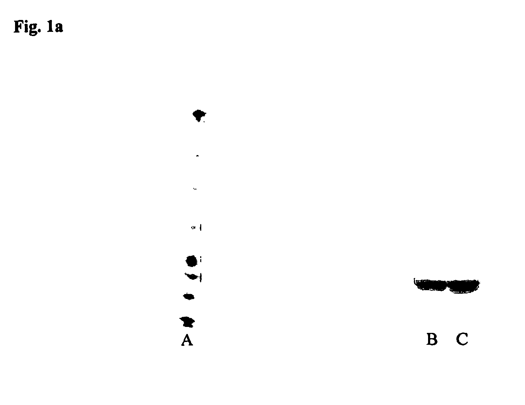

Figure 1a shows an SDS page analysis of the HES-G-CSF conjugate, produced according to Example 2.1 (a), Neupogen®. For gel electrophoresis, a XCell Sure Lock Mini Cell (Invitrogen GmbH, Karlsruhe, D) and a Consort E143 power supply (CONSORTnv, Turnhout, B) were employed. A 12% Bis-Tris gel together with a MOPS SDS running buffer at reducing conditions (both Invitrogen GmbH, Karlsruhe, D) were used according to the manufactures instruction.
- Lane A:: Protein marker SeeBlue®Plus2 (Invitrogen GmbH, Karlsruhe, D) Molecular weight marker from top to bottom: 188 kD, 98 kD, 62 kD, 49 kD, 38 kD, 28 kD, 17 kD, 14 kD, 6 kD, 3 kD.
- Lane B:: Crude product after conjugation of G-CSF (Neupogen®) with HES as described in Example 2.1 (a).
- Lane C:: G-CSF starting material.

### Figure 1b

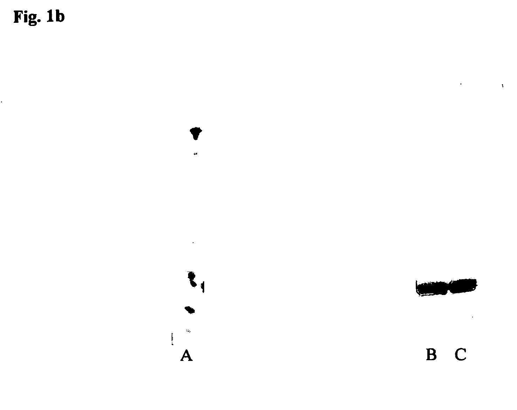

Figure 1b shows an SDS page analysis of the HES-G-CSF conjugate, produced according to Example 2.1 (a), Granocyte®. For gel electrophoresis, a XCell Sure Lock Mini Cell (Invitrogen GmbH, Karlsruhe, D) and a Consort E143 power supply (CONSORTnv, Turnhout, B) were employed. A 12% Bis-Tris gel together with a MOPS SDS running buffer at reducing conditions (both Invitrogen GmbH, Karlsruhe, D) were used according to the manufactures instruction.
- Lane A:: Protein marker SeeBlue®Plus2 (Invitrogen GmbH, Karlsruhe, D). Molecular weight marker from top to bottom: 188 kD, 98 kD, 62 kD, 49 kD, 38 kD, 28 kD, 17 kD, 14 kD, 6 kD, 3 kD.
- Lane B:: Crude product after conjugation of G-CSF (Granocyte®) with HES as described in Example 2.1(a).
- Lane C:: G-CSF starting material.

### Figure 2

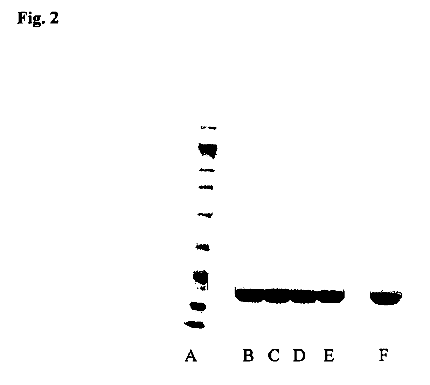

Figure 2 shows an SDS page analysis of the HES-G-CSF conjugate, produced according to Example 2.1(b), G-CSF from Strathmann Biotec AG, Hamburg, D. For gel electrophoresis a XCell Sure Lock Mini Cell (Invitrogen GmbH, Karlsruhe, D) and a Consort E143 power supply (CONSORTnv, Turnhout, B) were employed. A 12% Bis-Tris gel together with a MOPS SDS running buffer at reducing conditions (both Invitrogen GmbH, Karlsruhe, D) were used according to the manufactures instruction.
- Lane A:: Protein marker SeeBlue®Plus2 (Invitrogen GmbH, Karlsruhe, D). Molecular weight marker from top to bottom: 188 kD, 98 kD, 62 kD, 49 kD, 38 kD, 28 kD, 17 kD, 14 kD, 6 kD, 3 kD
- Lane B:: Crude product after conjugation of G-CSF with HES10/0.4 in 0.1M NaOAc buffer pH 5.0.
- Lane C:: Crude product after conjugation of G-CSF with HES10/0.7 in 0.1M NaOAc buffer pH 5.0.
- Lane D:: Crude product after conjugation of G-CSF with HES50/0.4 in 0.1M NaOAc buffer pH 5.0.
- Lane E:: Crude product after conjugation of G-CSF with HES50/0.7 in 0.1M NaOAc buffer pH 5.0.
- Lane F:: G-CSF starting material.

### Figure 3

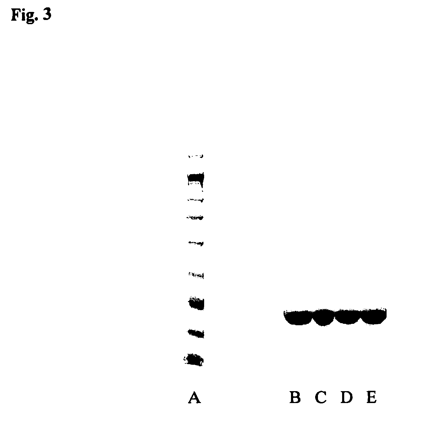

Figure 3 shows an SDS page analysis of the HES-G-CSF conjugates, produced according to Example 2.2, G-CSF from Strathmann Biotec AG, Hamburg, D. For gel electrophoresis a XCell Sure Lock Mini Cell (Invitrogen GmbH, Karlsruhe, D) and a Consort E143 power supply (CONSORTnv, Turnhout, B) were employed. A 12% Bis-Tris gel together with a MOPS SDS running buffer at reducing conditions (both Invitrogen GmbH, Karlsruhe, D) were used according to the manufactures instruction.
- Lane A:: Protein marker SeeBlue®Plus2 (Invitrogen GmbH, Karlsruhe, D). Molecular weight marker from top to bottom: 188 kD, 98 kD, 62 kD, 49 kD, 38 kD, 28 kD, 17 kD, 14 kD, 6 kD, 3 kD.
- Lane B:: Crude product after conjugation of G-CSF with oxidized HES10/0.7 in 0.1M NaOAc buffer pH 5.0.
- Lane C:: Crude product after conjugation of G-CSF with oxidized HES50/0.4 in 0.1M NaOAc buffer pH 5.0.
- Lane D:: Crude product after conjugation of G-CSF with oxidized HES50/0.7 in 0.1M NaOAc buffer pH 5.0.
- Lane E:: G-CSF starting material.

### Figure 4

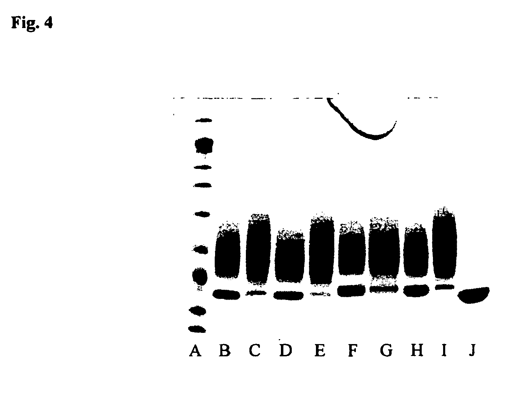

Figure 4 shows an SDS page analysis of the HES-G-CSF conjugates, produced according to Example 2.3, G-CSF is Neupogen® or Granocyte®. For gel electrophoresis a XCell Sure Lock Mini Cell (Invitrogen GmbH, Karlsruhe, D) and a Consort E143 power supply (CONSORTnv, Turnhout, B) were employed. A 12% Bis-Tris gel together with a MOPS SDS running buffer at reducing conditions (both Invitrogen GmbH, Karlsruhe, D) were used according to the manufactures instruction.
- Lane A:: Protein marker SeeBlue®Plus2 (Invitrogen GmbH, Karlsruhe, D). Molecular weight marker from top to bottom: 188 kD, 98 kD, 62 kD, 49 kD, 38 kD, 28 kD, 17 kD, 14 kD, 6 kD, 3 kD.
- Lane B:: Crude product (i-N) according to Example 2.3.
- Lane C:: Crude product (ii-N) according to Example 2.3.
- Lane D:: Crude product (iii-N) according to Example 2.3.
- Lane E:: Crude product (iv-N) according to Example 2.3.
- Lane F:: Crude product (i-G) according to Example 2.3.
- Lane G:: Crude product (ii-G) according to Example 2.3.
- Lane H:: Crude product (iii-G) according to Example 2.3.
- Lane I:: Crude product (iv-G) according to Example 2.3.
- Lane J:: Neupogen®.

### Figure 5

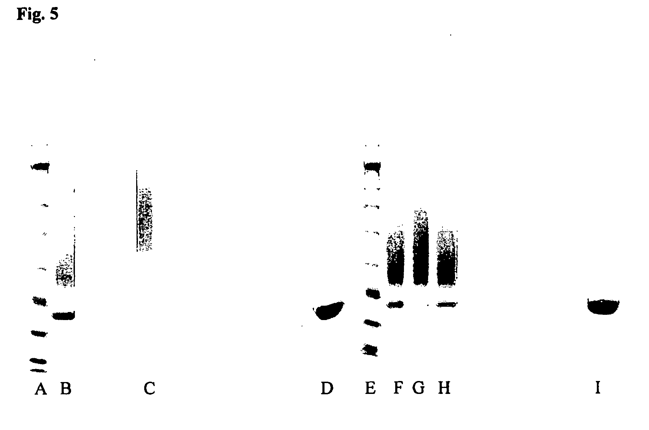

Figure 5 shows an SDS page analysis of the HES-G-CSF conjugates, produced according to Example 2.4, G-CSF from Strathmann Biotec AG, Hamburg, D. For gel electrophoresis a XCell Sure Lock Mini Cell (Invitrogen GmbH, Karlsruhe, D) and a Consort E143 power supply (CONSORTnv, Turnhout, B) were employed. A 12% Bis-Tris gel together with a MOPS SDS running buffer at reducing conditions (both Invitrogen GmbH, Karlsruhe, D) were used according to the manufactures instruction.
- Lane A:: Protein marker SeeBlue®Plus2 (Invitrogen GmbH, Karlsruhe, D). Molecular weight marker from top to bottom: 188 kD, 98 kD, 62 kD, 49 kD, 38 kD, 28 kD, 17 kD, 14 kD, 6 kD, 3 kD.
- Lane B:: Crude product (vi) according to Example 2.4.
- Lane C:: Crude product (v) according to Example 2.4.
- Lane D:: G-CSF starting material.
- Lane E:: Protein marker SeeBlue®Plus2 (Invitrogen GmbH, Karlsruhe, D). Molecular weight marker from top to bottom: 188 kD, 98 kD, 62 kD, 49 kD, 38 kD, 28 kD, 17 kD, 14 kD, 6 kD, 3 kD.
- Lane F:: Crude product (ix) according to Example 2.4.
- Lane G:: Crude product (viii) according to Example 2.4.
- Lane H:: Crude product (vii) according to Example 2.4.
- Lane I:: G-CSF starting material.

### Figure 6

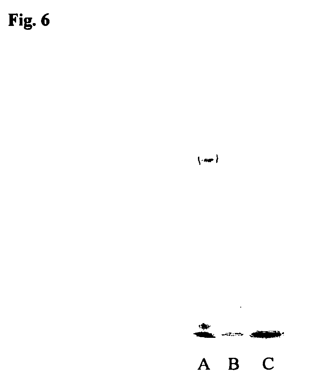

Figure 6 shows an SDS page analysis of the HES-G-CSF conjugate, produced according to Example 2.5, G-CSF from Strathmann Biotec AG, Hamburg, D. For gel electrophoresis a XCell Sure Lock Mini Cell (Invitrogen GmbH, Karlsruhe, D) and a Consort E143 power supply (CONSORTnv, Turnhout, B) were employed. A 10% Bis-Tris gel together with a MOPS SDS running buffer at reducing conditions (both Invitrogen GmbH, Karlsruhe, D) were used according to the manufactures instruction.
- Lane A:: Protein marker SeeBlue®Plus2 (Invitrogen GmbH, Karlsruhe, D). Molecular weight marker from top to bottom: 188 kD, 98 kD, 62 kD, 49 kD, 38 kD, 28 kD, 17 kD, 14 kD, 6 kD, 3 kD.
- Lane B:: Crude product according to Example 2.5.
- Lane C:: G-CSF starting material.

### Figure 7

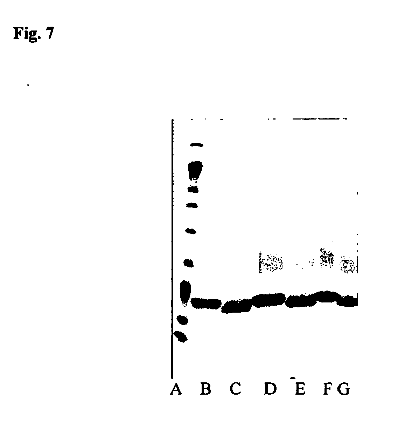

Figure 7 shows an SDS page analysis of the HES-G-CSF conjugate, produced according to Example 3, G-CSF is Neupogen® or Granocyte®. For gel electrophoresis a XCell Sure Lock Mini Cell (Invitrogen GmbH, Karlsruhe, D) and a Consort E143 power supply (CONSORTnv, Turnhout, B) were employed. A 12% Bis-Tris gel together with a MOPS SDS running buffer at reducing conditions (both Invitrogen GmbH, Karlsruhe, D) were used according to the manufactures instruction.
- Lane A:: Protein marker SeeBlue®Plus2 (Invitrogen GmbH, Karlsruhe, D). Molecular weight marker from top to bottom: 188 kD, 98 kD, 62 kD, 49 kD, 38 kD, 28 kD, 17 kD, 14 kD, 6 kD, 3 kD
- Lane B:: Crude product (x) according to Example 3.
- Lane C:: Crude product (xi) according to Example 3.
- Lane D:: Crude product (xii) according to Example 3.
- Lane E:: Crude product (xiii) according to Example 3.
- Lane F:: Crude product (xiv) according to Example 3.
- Lane G:: Crude product (xv) according to Example 3.

### Figure 8

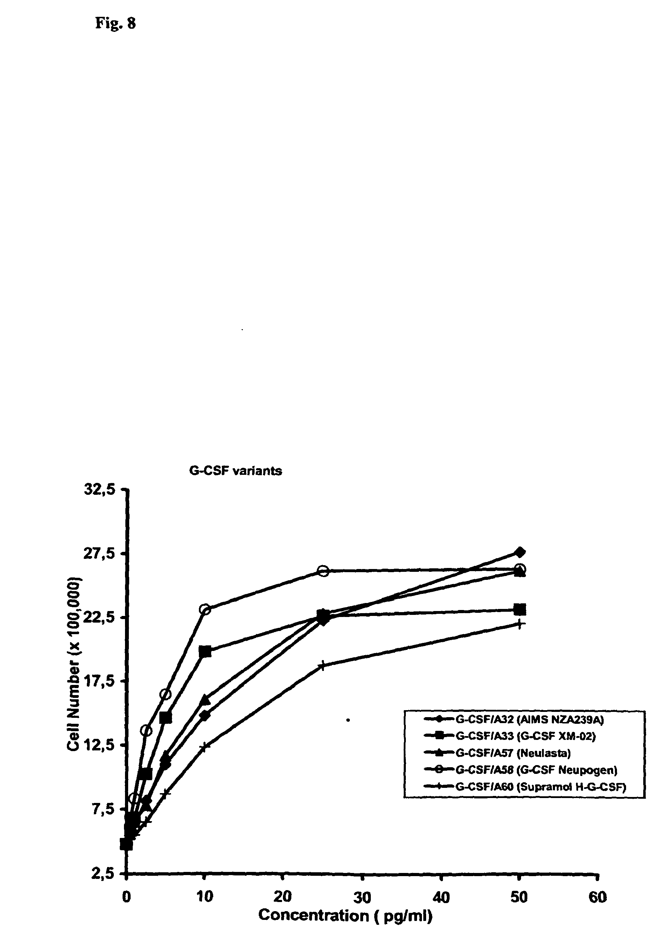

Figure 8 shows the in vitro results of Example 6.

In the diagram, the x axis shows the concentration in pg/ml, the y axis refers to the number of cells / 100,000. In the diagram, the following abbreviations refer to
- G-CSF/A32: G-CSF conjugate as prepared according to Example 2.5
- G-CSF/A33: G-CSF starting material, used for the conjugate of Example 2.5
- G-CSF/A57: non-modified Neulasta®
- G-CSF/A58: non-modified Neupogen®
- G-CSF/A60: G-CSF conjugate as prepared according to Example 4.2

### Figure 9

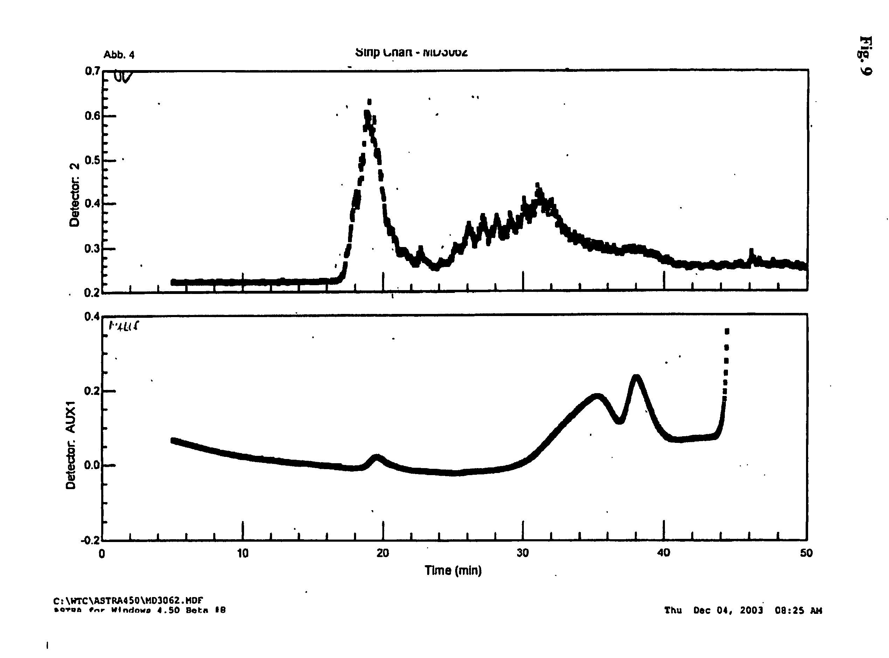

Figure 9 shows the HPGPC chromatogram with regard to the crude conjugation reaction product according to Example 4.1. The following parameters were used in the HPGPC analysis:
- Column:: Superose 12 HR 10/30 300 x 10 mm I.D. (Pharmacia)
- Eluent:: 27.3 8 mM Na₂HPO₄; 12.62 mM NaH₂PO₄; 0.2 M NaCl; 0,005 % NaN₃ in 11 of demineralized water
- Flux:: 0,24 ml/h
- Detector 1:: MALLS detector
- Detector 2:: UV (280 nm)
- Detector 3:: RI (Refractive Index detector)

A represents the result from detector 1, B represents the result from detector 2.

### Figure 10

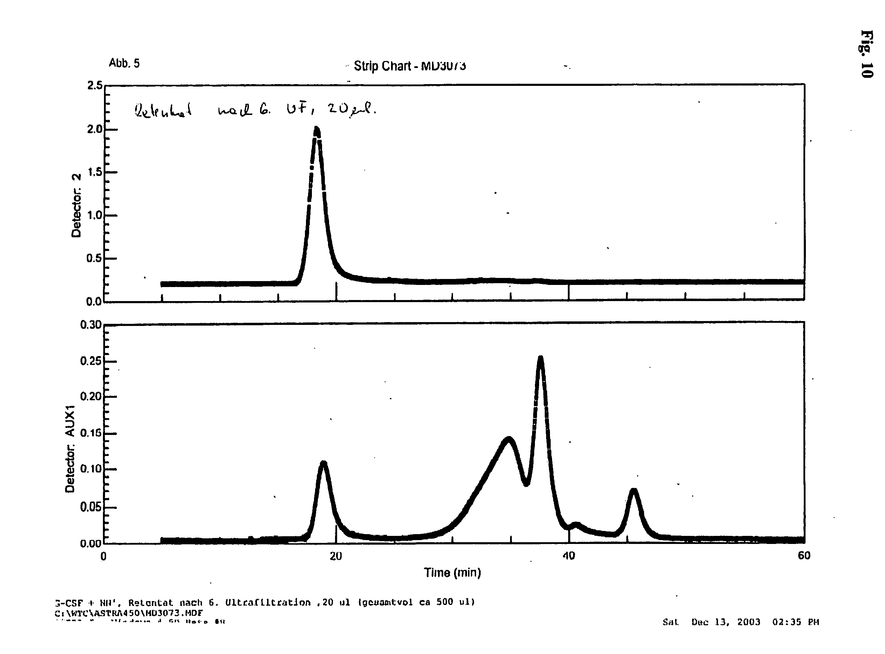

Figure 10 shows the HPGPC chromatogram with regard to the conjugation reaction product according to Example 4.1 where the content of the mixture regarding reaction by-products such as non-reacted oxo-HES and free N-hydroxysuccinimide as well as solvent was downgraded using a 10 kD ultrafiltration membrane in a cooling centrifuge.

The following parameters were used in the HPGPC analysis:
- Column:: Superose 12 HR 10/30 300 x 10 mm I.D. (Pharmacia)
- Eluent:: 27.38 mM Na₂HPO₄; 12.62 mM NaH₂PO₄; 0.2 M NaCl; 0,005 % NaN₃ in 11 of demineralized water
- Flux:: 0,24 ml/h
- Detector 1:: MALLS detector
- Detector 2:: UV (280 nm)
- Detector 3:: RI (Refractive Index detector)

A represents the result from detector 1, B represents the result from detector 2.

### Figure 11

The following parameters were used in the HPGPC analysis:
- Column:: Superose 12 HR 10/30 300 x 10 mm Lid. (Pharmacia)
- Eluent:: 27.38 mM Na₂HPO₄; 12.62 mM NaH₂PO₄; 0.2 M NaCl; 0,005 % NaN₃ in 11 of demineralized water
- Flux:: 0,24 ml/h
- Detector 1:: MALLS detector
- Detector: 2: UV (280 nm).
- Detector 3:: RI (Refractive Index detector)

A represents the result from detector 1, B represents the result from detector 2.

### Figure 12

The following parameters were used in the HPGPC analysis:
- Column:: Superose 12 HR 10/30 300 x 10 mm I.D. (Pharmacia)
- Eluent:: 27.3 8 mM Na₂HPO₄; 12.62 mM NaH₂PO₄; 0.2 M NaCl; 0,005 % NaN₃ in 11 of demineralized water
- Flux:: 0,24 ml/h

Detector 1: MALLS detector
Detector 2: UV (280 nm)
Detector 3: RI (Refractive index detector)

A represents the result from detector 1, B represents the result from detector 2.

### Figure 13

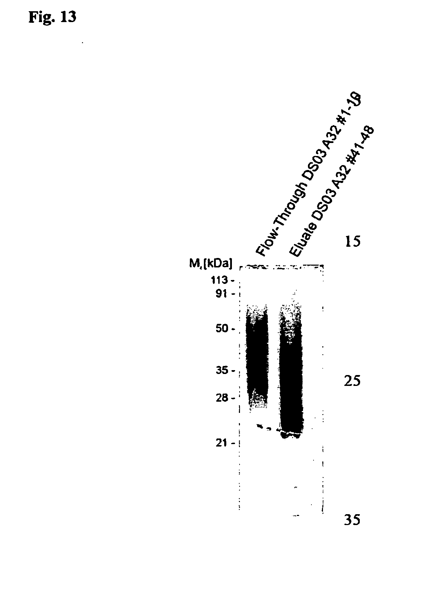

Figure 13 shows the SDS-PAGE analysis of the flow-through and the eluate of HES-modified G-CSF (A32) after chromatography on DEAE-Sepharose CL-6B. 1.5% of the indicated fractions were desalted by ultrafiltration, dried in a SpeedVac and were applied onto a 12.5% polyacrylamide gel.

### Figure 14

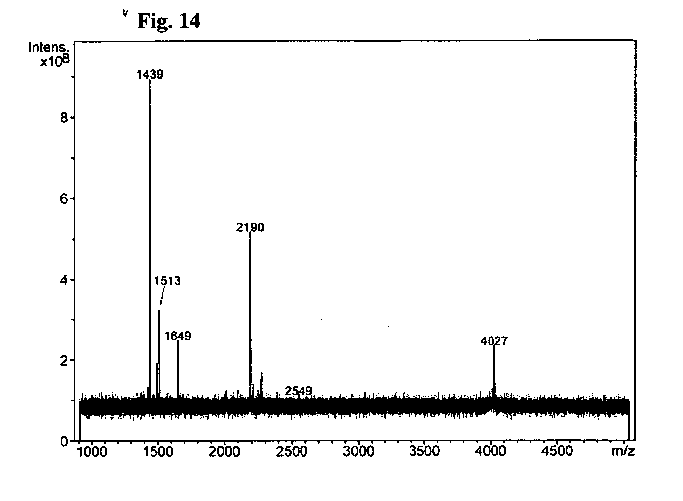

Figure 14 shows a MALDI/TOF spectrum of the G-CSF starting material (sample A33)

### Figure 15

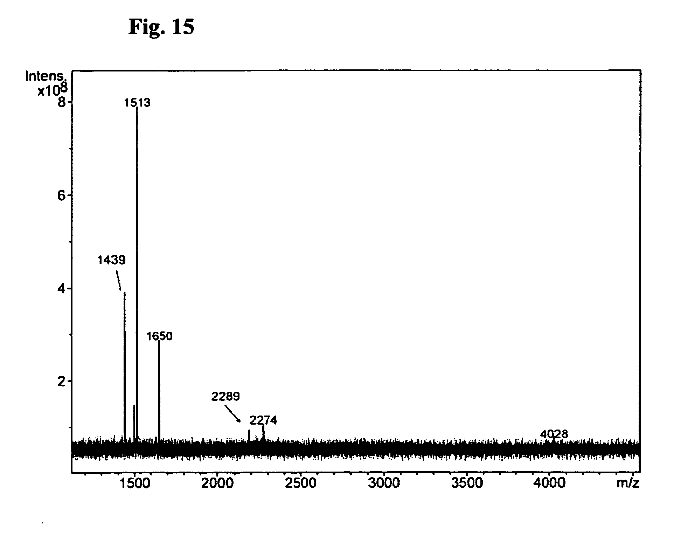

Figure 15 shows a MALDI/TOF spectrum of HES-modified G-CSF (sample A32)

### Figure 16

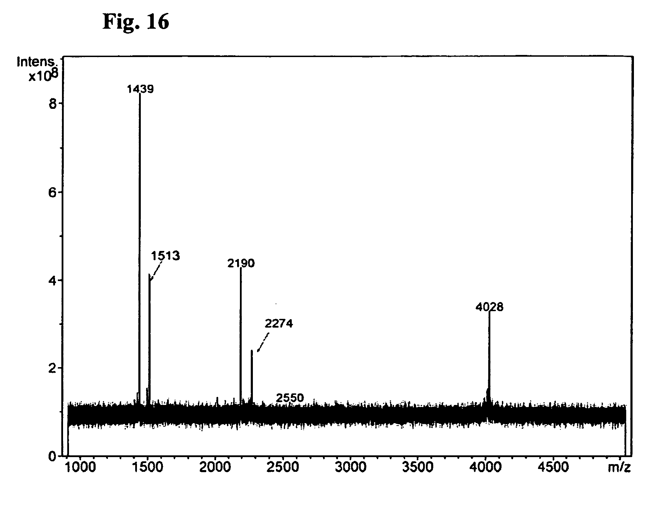

Figure 16 shows a MALDI/TOF spectrum of HES-modified G-CSF (sample A60)

### Figure 17

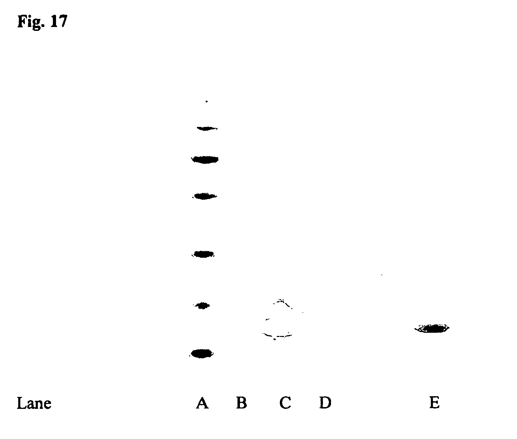

Figure 17 shows the gel eletrophoresis of the reaction mixtures of example 7.2(b).

For gel electrophoresis a XCell Sure Lock Mini Cell (Invitrogen GmbH, Karlsruhe, D) and a Consort E143 power supply (CONSORTnv, Turnhout, B) were employed. A 12% Bis-Tris gel together with a MOPS SDS running buffer at reducing conditions (both Invitrogen GmbH, Karlsruhe, D) were used according to the manufactures instruction. The gel was stained with Roti-Blue (Carl Roth GmbH + Co.KG, Karlsruhe, D) according to the manufacturer's instruction.
- Lane A:: Protein marker Roti-Mark STANDARD (Carl Roth GmbH + Co.KG, Karlsruhe, D) Molecular weight marker from top to bottom: 200 KD, 119 KD, 66 KD, 43 KD, 29 KD, 20 KD, 14.3 KD
- Lane B:: Crude product after conjugation of hG-CSF with the HES derivative prepared in example 7.1 (d)
- Lane C:: Crude product after conjugation of hG-CSF with the HES derivative prepared in example 7.1(b)
- Lane D:: Crude product after conjugation of hG-CSF with the HES derivative prepared in example 7.1 (j)
- Lane E:: Reaction control: HES 50/07

### Figure 18

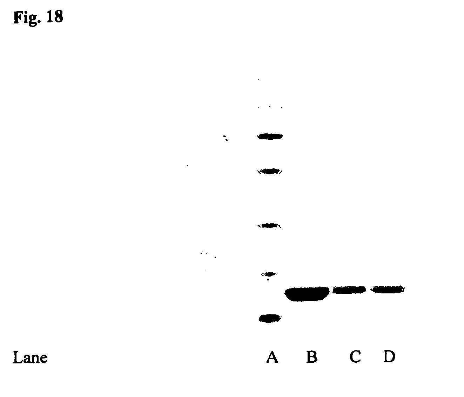

Figure 18 shows the gel electrophoresis of the reaction mixtures of example 7.2(d). For gel electrophoresis a XCell Sure Lock Mini Cell (Invitrogen GmbH, Karlsruhe, D) and a Consort E143 power supply (CONSORTnv, Turnhout, B) were employed. A 12% Bis-Tris gel together with a MOPS SDS running buffer at reducing conditions (both Invitrogen GmbH, Karlsruhe, D) were used according to the manufactures instruction. The gel was stained with Roti-Blue (Carl Roth GmbH + Co.KG, Karlsruhe, D) according to the manufacturer's instruction.
- Lane A:: Protein marker Roti-Mark STANDARD (Carl Roth GmbH + Co.KG, Karlsruhe, D) Molecular weight marker from top to bottom: 200 KD, 119 KD, 66 KD, 43 KD, 29 KD, 20 KD, 14.3 KD.
- Lane B:: hG-CSF after buffer exchange as described in Example 7.2(c).
- Lane C:: Crude product after conjugation of hG-CSF with the HES derivative prepared as described in Example 7.1(f).
- Lane D:: Crude product after conjugation of hG-CSF with the HES derivative prepared as described in Example 7.1 (h).

### Figure 19

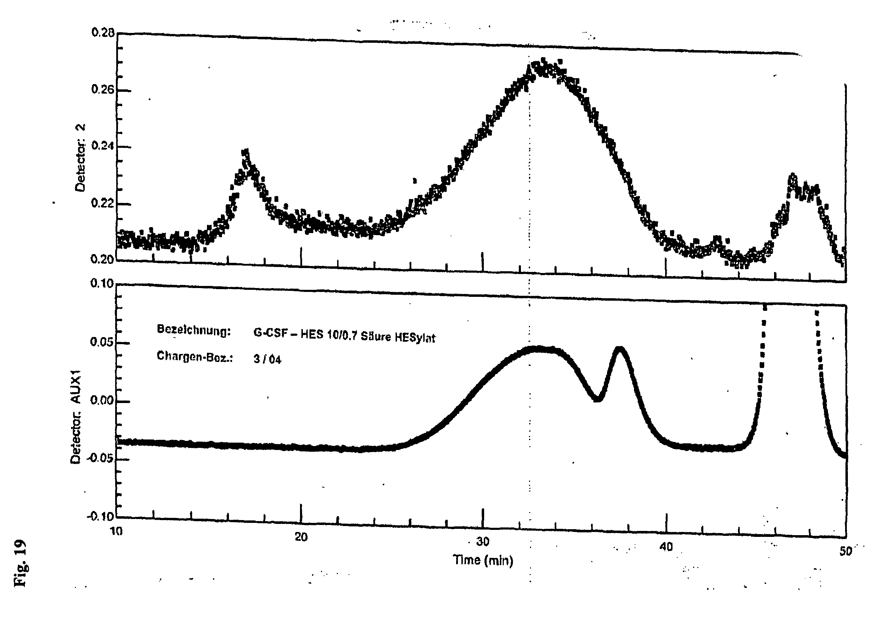

Figure 19 shows the HPGPC chromatogram with regard to the conjugation reaction product according to Example 7.3 (MALLS detector: upper chart; UV detector: lower chart). The following parameters were used in the HPGPC analysis: Column: Superose 12 HR 10/30 300 x 10 mm I.D. (Pharmacia)
- Eluent:: 27.38 mM Na₂HPO₄; 12.62 mM NaH₂PO₄; 0.2 M NaCl; 0,005 % NaN₃ in 11 of demineralized water
- Flux:: 0,24 ml/h
- Detector 1:: MALLS detector
- Detector 2:: UV (280 nm)
- Detector 3:: RI (Refractive Index detector)

### Figure 20

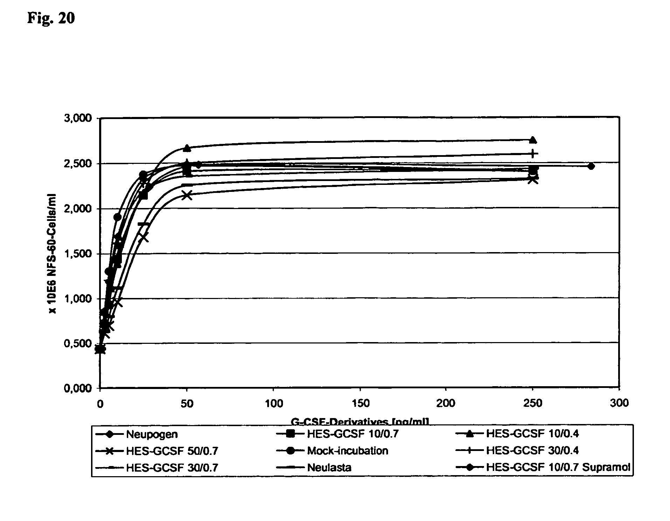

Figure 20 shows the results of the mitogenicity assay of example 7.4. The Y axis indicates number of NFS-60-Cells/ml and the X-axis the concentration in pg/ml.

### Figure 21

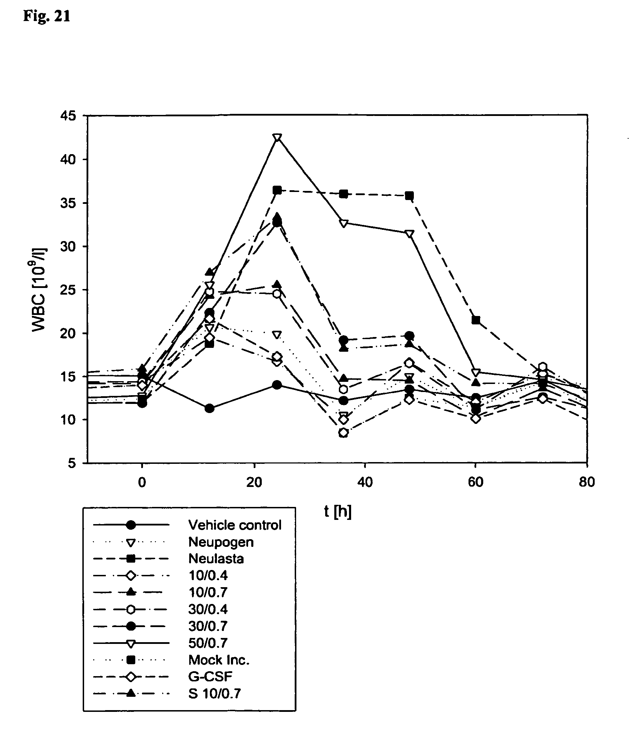

Figure 21 shows the results of the in vivo assay of example 7.5.

### Examples

### Example 1: Synthesis of aldehyde functionalized hydroxyethyl starch

### Example 1.1(a): Synthesis by periodate oxidation of hydroxyethyl starch selectively oxidized at its reducing end and incubation at 0 °C

100 mg of Oxo-HES 10/0.4 (MW = 10 kD, DS = 0.4, prepared by Supramol Parenteral Colloids GmbH, Rosbach-Rodheim, D; according to DE 196 28 705. A1, were dissolved in 5 ml 20 mM sodium phosphate buffer, pH 7.2 and cooled to 0 °C. 21.4 mg sodium periodate (Fluka, Sigma-Aldrich Chemie GmbH, Taufkirchen, D) were dissolved in 5 ml of the same buffer and cooled to 0°C. Both solutions were mixed and after incubation for 10 min at 0°C, 0.73 ml glycerol were added and the reaction mixture was incubated at 21 °C for 10 min. The reaction mixture was dialysed for 24 h against water (SnakeSkin dialysis tubing, 3.5 kD cut off, Perbio Sciences Deutschland GmbH, Bonn, D) and lyophilized.

### Example 1.1(b) Synthesis by periodate oxidation of hydroxyethyl starch selectively oxidized at its reducing end and incubation at 21 °C

100 mg of Oxo-HES10/0.4 (MW = 10 kD, DS = 0.4, prepared by Supramol Parenteral Colloids GmbH, Rosbach-Rodheim, D; according to DE 196 28 705 A1) were dissolved in 5 ml 20 mM sodium phosphate buffer, pH 7.2. 21.4 mg sodium periodate (Fluka, Sigma-Aldrich Chemie GmbH, Taufkirchen, D) were dissolved in 5 ml of the same buffer. Both solutions were mixed and after incubation for 10 min at 21 °C 0.73 ml glycerol were added and the reaction mixture was incubated at 21°C for 10 min. The reaction mixture was dialysed for 24 h against water (SnakeSkin dialysis tubing, 3.5 kD cut off, Perbio Sciences Deutschland GmbH, Bonn, D) and lyophilized.

### Example 1.2(a): Synthesis of aldehyde functionalized hydroxyethyl starch by periodate oxidation of hydroxyethyl starch with non-oxidized reducing end and incubation at 0 °C

100 mg of HES10/0.4 (MW = 10 kD, DS = 0.4, Supramol Parenteral Colloids GmbH, Rosbach-Rodheim, D) were dissolved in 5 ml 20 mM sodium phosphate buffer, pH 7.2 and cooled to 0 °C. 21.4 mg sodium periodate (Fluka, Sigma-Aldrich Chemie GmbH, Taufkirchen, D) were dissolved in 5 ml of the same buffer and cooled to 0°C. Both solutions were mixed and after incubation for 10 min at 0°C 0.73 ml glycerol were added and the reaction mixture was incubated at 21°C for 10 min. The reaction mixture was dialysed for 24 h against water (SnakeSkin dialysis tubing, 3.5 kD cut off, Perbio Sciences Deutschland GmbH, Bonn, D) and lyophilized.

### Example 1.2(b): Synthesis of aldehyde functionalized hydroxyethyl starch by periodate oxidation of hydroxyethyl starch with non-oxidized reducing end and incubation at 21 °C

100 mg of HES 10/0.4 (MW = 10 kD, DS = 0.4, prepared by Supramol Parenteral Colloids GmbH, Rosbach-Rodheim, D) were dissolved in 5 ml 20 mM sodium phosphate buffer, pH 7.2. 21.4 mg sodium periodate (Fluka, Sigma-Aldrich Chemie GmbH, Taufkirchen, D) were dissolved in 5 ml of the same buffer. Both solutions were mixed and after incubation for 10 min at 21 °C 0.73 ml glycerol were added and the reaction mixture was incubated at 21 °C for 10 min. The reaction mixture was dialysed for 24 h against water (SnakeSkin dialysis tubing, 3.5 kD cut off, Perbio Sciences Deutschland GmbH, Bonn, D) and lyophilized.

### Example 1.3: Synthesis of aldehyde functionalized hydroxyethyl starch from amino functionalized hydroxyethyl starch and formylbenzoic acid

Oxo-HES 10/0.4 (MW = 10 kD, DS = 0.4) was prepared by Supramol Parenteral Colloids GmbH, Rosbach-Rodheim, D; according to DE 196 28 705 A1.

5.1 g (0.51 mmol) of oxo-HES10/0.4 were dissolved in 15 ml anhydrous dimethyl sulfoxide (DMSO, Fluka, Sigma-Aldrich Chemie GmbH, Taufkirchen, D)) and added dropwise under nitrogen to a solution of 5.1 ml (51 mmol) 1,4-diaminobutane in 10 ml anhydrous dimethyl sulfoxide and stirred at 40 °C for 19 h. The reaction mixture was added to a mixture of 80 ml ethanol and 80 .ml acetone. The resulting precipitate was separated by centrifugation, washed with a mixture of 20 ml ethanol and 20 ml acetone and re-dissolved in 80 ml water. The solution was dialyzed for 4 days against water (SnakeSkin dialysis tubing, 3.5 kD cut off, Perbio Science Deutschland GmbH, Bonn, D) and subsequently lyophilized. The yield was 67 % (3.4 g) amino-HES 10/0.4.

150 mg 4-formylbenzoic acid and 230 mg 1-hydroxy-1H-benzotriazole (both Aldrich, Sigma-Aldrich Chemie GmbH, Taufkirchen, D) were dissolved in 10 ml N,N-dimethylformamide (Peptide synthesis grade, Biosolve, Valkenswaard, NL) and 204 µl N,N'-diisopropylcarbodiimide were added. After incubation at 21 °C for 30 min, 1 g of the amino-HES10/0.4 were added. After shaking for 19 h at 22 °C, the reaction mixture was added to 84 mL of an ice-cold 1:1 mixture of acetone and ethanol (v/v). The precipitated product was collected by centrifugation at 4 °C, re-dissolved in 50 m water, dialysed for 2 d against water (SnakeSkin dialysis tubing, 3.5 kD cut off, Perbio Sciences Deutschland GmbH, Bonn, D) and lyophilized.

### Example 1.4: Synthesis of aldehyde functionalized hydroxyethyl starch from hydroxyethyl starch and formylbenzoic acid

Oxo-HES10/0.7 (MW = 10 kD, DS = 0.7) was prepared by Supramol Parenteral Colloids GmbH, Rosbach-Rodheim, D; according to DE 196 28 705 A1.

83 mg of 4-formylbenzoic acid and 180 mg 1-hydroxy-1H-benzotriazole (both Aldrich, Sigma-Aldrich Chemie GmbH, Taufkirchen, D) were dissolved in 5 mL N,N-dimethylformamide (DMF, Peptide synthesis grade, Biosolve, Valkenswaard, NL) and 78 µl N,N'-diisopropylcarbodiimide were added. After incubation at 21 °C for 30 min, 0.5 g of oxo-HES 10/0.7 were added. After shaking for 19 h at 22 °C, the reaction mixture was added to 37.5 ml of an ice-cold 1:1 mixture of acetone and ethanol (v/v). The precipitated product was collected by centrifugation at 4 °C, re-dissolved in a mixture of 2.5 ml water and 2.5 ml DMF and precipitated again as described above. The reaction product was collected by centrifugation as described, re-dissolved in 10 ml water, dialysed for 2 d against water (SnakeSkin dialysis tubing, 3.5 kD cut off, Perbio Sciences Deutschland GmbH, Bonn, D) and lyophilized.

### Example 1.5: Synthesis of aldehyde functionalized hydroxyethyl starch from hydroxyethyl starch and formylbenzoic acid

HES10/0.7 (MW = 10 kD, DS = 0.7) was prepared by Supramol Parenteral Colloids GmbH, Rosbach-Rodheim, D.

50 mg 4-formylbenzoic acid and 108 mg 1-hydroxy-1H-benzotriazole (both Aldrich, Sigma-Aldrich Chemie GmbH, Taufkirchen, D) were dissolved in 3 ml N,N-dimethylformamide (Peptide synthesis grade, Biosolve, Valkenswaard, NL) and 47 µl N,N'-diisopropylcarbodiimide were added. After incubation at 21 °C for 30 min, 0.3 g of HES10/0.7 were added. After shaking for 19 h at 22 °C, the reaction mixture was added to 23 ml of an ice-cold 1:1 mixture of acetone and ethanol (v/v). The precipitated product was collected by centrifugation at 4 °C, re-dissolved in a mixture of 1.5 ml water and 1.5 ml DMF and precipitated again as described above. The reaction product was collected by centrifugation as described, re-dissolved in 10 ml water, dialysed for 2 d against water (SnakeSkin dialysis tubing, 3.5 kD cut off, Perbio Sciences Deutschland GmbH, Bonn, D) and lyophilized.

### Example 1.6: Synthesis of aldehyde functionalized hydroxyethyl starch from amino functionalized hydroxyethyl starch and formylbenzoic acid pentafluorophenyl ester

Oxo-HES10/0.7 (MW = 10 kD, DS = 0.7) was prepared by Supramol Parenteral Colloids GmbH, Rosbach-Rodheim, D; according to DE 196 28 705 A1.

6.0 g (0.6 mmol) of oxo-HES 10/0.7 were dissolved in 20 ml anhydrous dimethyl sulfoxide (DMSO, Fluka, Sigma-Aldrich Chemie GmbH, Taufkirchen, D)) and added dropwise under nitrogen to a solution of 6 ml (60 mmol) 1,4-diaminobutane in 11 ml anhydrous dimethyl sulfoxide and stirred at 40 °C for 19 h. The reaction mixture was added to a mixture of 80 ml ethanol and 80 ml acetone. The resulting precipitate was separated by centrifugation, washed with a mixture of 20 ml ethanol and 20 ml acetone and re-dissolved in 80 ml water. The solution was dialyzed for 4 days against water (SnakeSkin dialysis tubing, 3.5 kD cut off, Perbio Science Deutschland GmbH, Bonn, D) and subsequently lyophilized. The yield was 52 % (3.15 g) amino-HES 10/0.7.

4-formylbenzoic acid pentafluorophenyl ester was synthesized as described in J. S. Lindsey at al., Tetrahedron 50 (1994) pp. 8941-68, especially p. 8956. 50 mg of amino-HES10/0.7 were dissolved in 0.5 ml N,N-dimethylformamide (Peptide synthesis grade, Biosolve, Valkenswaard, NL) and 15.3 mg 4-formylbenzoic acid pentafluorophenylester were added. After shaking for 22 h at 22 °C, the reaction mixture was added to 3.5 ml of ice-cold 2-propanol. The precipitated product was collected by centrifugation at 4 °C, washed with 4 ml ice-cold 2-propanol, re-dissolved in 50 ml water, dialysed for 2 d against water (SnakeSkin dialysis tubing, 3.5 kD cut off, Perbio Sciences Deutschland GmbH, Bonn, D) and lyophilized.

### Example 1.7: Synthesis of aldehyde functionalized hydroxyethyl starch from hydroxyethyl starch and formylbenzoic acid pentafluorophenyl ester

Oxo-HES10/0.7 (MW = 10 kD, DS = 0.7) was prepared by Supramol Parenteral Colloids GmbH, Rosbach-Rodheim, D; according to DE 196 28 705 A1.

4-formylbenzoic acid pentafluorophenyl ester was synthesized as described in J. S. Lindsey at al., Tetrahedron 50 (1994) pp. 8941-68, especially p. 8956. 200 mg oxo-HES10/0.7 were dissolved in 2 ml N,N-dimethylformamide (peptide synthesis grade, Biosolve, Valkenswaard, NL) and 61.2 mg 4-formylbenzoic acid pentafluorophenyl ester were added. After shaking for 22 h at 22 °C, the reaction mixture was added to 15 mL of ice-cold 1:1 mixture of acetone and ethanol (v/v). The precipitated product was collected by centrifugation at 4 °C, re-dissolved in a mixture of 1.4 ml water and 0.7 ml DMF and precipitated again as described above. The reaction product was collected by centrifugation as described, re-dissolved in 10 ml water, dialysed for 2 d against water (SnakeSkin dialysis tubing, 3.5 kD cut off, Perbio Sciences Deutschland GmbH, Bonn, D) and lyophilized.

### Example 1.8: Synthesis of aldehyde functionalized hydroxyethyl starch from amino functionalized hydroxyethyl starch and 4-(4-formyl-3,5-dimethoxyphenoxy)butyric acid

Oxo-HES10/0.4 (MW = 10 kD, DS = 0.4) was prepared by Supramol Parenteral Colloids GmbH, Rosbach-Rodheim, D; according to DE 196 28 705 A1.

5.1 g (0.51 mmol) of oxo-HES10/0.4 were dissolved in 15 ml anhydrous dimethyl sulfoxide (DMSO, Fluka, Sigma-Aldrich Chemie GmbH, Taufkirchen, D)) and added dropwise under nitrogen to a solution of 5.1 ml (51 mmol) 1,4-diaminobutane in 10 ml anhydrous dimethyl sulfoxide and stirred at 40 °C for 19 h. The reaction mixture was added to a mixture of 80 ml ethanol and 80 ml acetone. The resulting precipitate was separated by centrifugation, washed with a mixture of 20 ml ethanol and 20 ml acetone and re-dissolved in 80 ml water. The solution was dialyzed for 4 days against water (SnakeSkin dialysis tubing, 3.5 kD cut off, Perbio Science Deutschland GmbH, Bonn, D) and subsequently lyophilized. The yield was 67 % (3.4 g) amino-HES10/0.4.

80.5 mg 4-(4-formyl-3,5-dimethoxyphenoxy)butyric acid (Calbiochem-Novabiochem, Läufelfingen, CM and 61 mg 1-hydroxy-1H-benzotriazole (Aldrich, Sigma-Aldrich Chemie GmbH, Taufkirchen, D) were dissolved in 3 ml N,N-dimethylformamide (Peptide synthesis grade, Biosolve, Valkenswaard, NL) and 45.4 µl N,N'-diisopropylcarbodiimide were added. After incubation at 21 °C for 30 min, 0.3 g of amino-HES 10/0.4 were added. After shaking for 22 h at 22 °C, the reaction mixture was added to 23 ml of ice-cold 1:1 mixture of acetone and ethanol (v/v). The precipitated product was collected by centrifugation at 4 °C, re-dissolved in a mixture of 2 ml water and 1 ml DMF and precipitated again as described above. The reaction product was collected by centrifugation as described, re-dissolved in 10 ml water, dialysed for 1 d against water (SnakeSkin dialysis tubing, 3.5 kD cut off, Perbio Sciences Deutschland GmbH, Bonn, D) and lyophilized.

### Example 2: Synthesis of G-CSF conjugates by reductive amination

### Example 2.1(a): Synthesis of G-CSF-conjugates by reductive amination with hydroxyethyl starch with non-oxidized reducing end at pH = 7.4 (Comparative Example)

In Example 2.1, it was tried to use the synthesis method of WO 03/074087 (example 12, page 22 - 23) for the production of a HES-G-CSF conjugate.

To 3.33 µl of an aqueous solution of G-CSF (Neupogen® from Amgen, München, D, or Granocyte® from Aventis Pharma AG, Zurich, CH, respectively, 3 mg/ml) in 0.1 M sodium phosphate buffer with pH 7.4, 3.33 µl of a solution of HES10/0.4 (MW = 10 kD, DS = 0.4, Supramol Parenteral Colloids GmbH, Rosbach-Rodheim, D, 79 mg/ml) in the same buffer were added. To this mixture, 3.33 µl of a 60 mM solution of sodium cyanoborohydride in the same buffer was added, and the resulting mixture was incubated for 4 h at 22 °C. Subsequently, another 3.33 µl of the freshly prepared 60 mM sodium cyanoborohydride solution were added. During the incubation time of 30 h, altogether 5 portions of 3.33 µl of a freshly prepared 60 mM sodium cyanoborohydride solution were added. The reaction mixture was analysed by gel electrophoresis. No reaction was observed.

### Example 2.1(b): Synthesis of G-CSF-conjugates by reductive amination with hydroxyethyl starch with non-oxidized reducing end at a pH of from 5.0 to 9.2

### (Comparative Example)

To 3.33 µL of an aqueous solution of G-CSF (G-CSF from Strathmann Biotec AG, Hamburg, D, 3 mg/mL) in a given buffer, 3.33 µl of a HES solution (300 mg/ml) in the same buffer were added. The mixture was cooled to 4 °C, and 3.33 µl of a 60 mM solution of sodium cyanoborohydride in the same buffer at 4 °C were added, and the resulting mixture was incubated for 20 h at 4 °C.

The following HES preparations and buffer were employed:
a) Buffer: 0.1 M sodium acetate buffer pH 5.0
   - HES 10/0.4 (MW = 10 kD, DS = 0.4, Supramol Parenteral Colloids GmbH, Rosbach-Rodheim, D)
   - HES10/0.7 (MW = 10 kD, DS = 0.7, Supramol Parenteral Colloids GmbH, Rosbach-Rodheim, D)
   - HES50/0.4 (MW = 50 kD, DS = 0.4, Supramol Parenteral Colloids GmbH, Rosbach-Rodheim, D)
   - HES50/0.7 (MW = 50 kD, DS = 0.7, Supramol Parenteral Colloids GmbH, Rosbach-Rodheim, D)
b) Buffer: 0.1 M sodium phosphate buffer pH 7.2
   - HES 10/0.7 (MW = 10 kD, DS = 0.7, Supramol Parenteral Colloids GmbH, Rosbach-Rodheim, D)
c) Buffer: 0.1 M sodium borate buffer pH 8.3
   - HES10/0.7 (MW = 10 kD, DS = 0.7, Supramol Parenteral Colloids GmbH, Rosbach-Rodheim, D)
d) Buffer: 0.2 M potassium borate buffer pH 9.2
   - HES10/0.7 (MW = 10 kD, DS = 0.7, Supramol Parenteral Colloids GmbH, Rosbach-Rodheim, D)

Each reaction mixture was analysed by gel electrophoresis. No or negligible conjugation was observed (gel scans for reactions b) to d) not shown).

### Example 2.2: Synthesis of GCSF-conjugates by reductive amination with hydroxyethyl starch with oxidized reducing end at a pH of 5.0 from 9.2

### (Comparative Example)

To 3.33 µL of an aqueous solution of G-CSF (G-CSF from Strathmann Biotec AG, Hamburg, D, 3 mg/ml) in a given buffer, 3.33 µl of a solution of oxo-HES (300 mg/ml) in the same buffer were added. The mixture was cooled to 4°C, and 3.33 µl of a 60 mM solution of sodium cyanoborohydride in the same buffer at 4 °C were added, and the mixture was incubated for 17 h at 4°C.

The following HES preparations and buffer were employed:
a) Buffer: 0.1 M sodium acetate buffer pH 5.0
   - oxo-HES10/0.7 (MW = 10 kD, DS = 0.7, Supramol Parenteral Colloids GmbH, Rosbach-Rodheim, D)
   - oxo-HES50/0.4 (MW = 50 kD, DS = 0.4, Supramol Parenteral Colloids GmbH, Rosbach-Rodheim, D)
   - oxo-HES50/0.7 (MW = 50 kD, DS = 0.7, Supramol Parenteral Colloids GmbH, Rosbach-Rodheim, D)
b) Buffer: 0.1 M sodium phosphate buffer pH 7.2
   - HES10/0.7 (MW = 10 kD, DS = 0.7, Supramol Parenteral Colloids GmbH, Rosbach-Rodheim, D)
c) Buffer: 0.1 M sodium borate buffer pH 8.3
   - HES10/0.7 (MW = 10 kD, DS = 0.7, Supramol Parenteral Colloids GmbH, Rosbach-Rodheim, D)
d) Buffer: 0.2 M potassium borate buffer pH 9.2
   - HES10/0.7 (MW = 10 kD, DS = 0.7, Supramol Parenteral Colloids GmbH, Rosbach-Rodheim, D)

Each reaction mixture was analysed by gel electrophoresis. No or negligible conjugation was observed (gel scans for reactions b) to d) not shown).

Oxidation of HES10/0.4 (MW = 10 kD, DS = 0.4) was carried out by Supramol Parenteral Colloids GmbH, Rosbach-Rodheim, D; according to DE 196 28 705 A1.

### Example 2.3: Synthesis of G-CSF-conjugates by reductive amination with aldehyde functionalized hydroxyethyl starch synthesized by periodate oxidation

To 3.33 µl of an aqueous solution of G-CSF (Granocyte® from Aventis Pharma AG, Zurich, CH, and Neupogen® from Amgen, München, D, respectively, 3 mg/mL) in 0.1 M sodium acetate buffer pH 5.0, 3.33 µl of a solution of an aldehydo-HES (79 mg/mL) in the same buffer were added. To the mixture 3.33 µL of a 60 mM solution of sodium cyanoborohydride in the same buffer were added and the mixture was incubated for 25 h at 21°C. The reaction mixture was analysed by gel electrophoresis.

The following aldehyde functionalized HES conjugates were employed:
(i-N) prepared with Neupogen® according to Example 1.1 (a) hereinabove;
(ii-N) prepared with Neupogen® according to Example 1.1 (b) hereinabove;
(iii-N) prepared with Neupogen® according to Example 1.2(a) hereinabove;
(iv-N) prepared with Neupogen® according to Example 1.2(b) hereinabove;
(i-G) prepared with Granocyte® according to Example 1.1 (a) hereinabove;
(ii-G) prepared with Granocyte® according to Example 1.1(b) hereinabove;
(iii-G) prepared with Granocyte® according to Example 1.2(a) hereinabove;
(iv-G) prepared with Granocyte® according to Example 1.2(b) hereinabove.

### Example 2.4: Synthesis of GCSF-conjugates by reductive amination with aldehyde functionalized hydroxyethyl starch synthesized by conjugation of hydroxyethyl starch to a formyl-carboxylic acid

To 3.33 µl of an aqueous solution of G-CSF (G-CSF from Strathmann Biotec AG, Hamburg, D, 3 mg/ml) in 0.1 M sodium acetate buffer pH 5.0, 3.33 µl of a solution of an aldehydo-HES (118.5 mg/mL) in the same buffer were added and cooled to 4 °C. To the mixture 3.33 µl of a 60 mM solution of sodium cyanoborohydride in the same buffer at 4 °C were added and the mixture was incubated for 17 h at 4 °C. The reaction mixture was analysed by gel electrophoresis.

The following aldehyde functionalized HES conjugates were employed:
(v) prepared according to Example 1.4 hereinabove;
(vi) prepared according to Example 1.5 hereinabove;
(vii) prepared according to Example 1.6 hereinabove;
(viii) prepared according to Example 1.7 hereinabove;
(ix) prepared according to Example 1.8 hereinabove.

### Example 2.5: Synthesis of G-CSF-conjugates by reductive amination with aldehyde functionalized hydroxyethyl starch synthesized by conjugation of hydroxyethyl starch to formyl-carboxylic acid

To 2.5 ml of an aqueous solution of G-CSF (G-CSF from Strathmann Biotec AG, Hamburg, D, 227 mg/ml) in 0.1 M sodium acetate buffer pH 5.0, 136 mg aldehydo-HES 10/0.4, prepared as described in Example 1.3 hereinabove, were added, and the solution was cooled to 0 °C. To the mixture 2.5 ml of an ice-cold 40 mM solution of sodium cyanoborohydride in the same buffer were added and the mixture was incubated for 17 h at 4 °C. The reaction mixture was analysed by gel electrophoresis.

### Example 3: (not according to the invention) Synthesis of G-CSF conjugates by SH alkylation

Oxo-FIES10/0.7 (MW = 10 kD, DS = 0.7) was prepared by Supramol Parenteral Colloids GmbH, Rosbach-Rodheim, D; according to DE 196 28 705 A1.

6.0 g (0.6 mmol) of oxo-HES 10/0.7 were dissolved in 20 ml anhydrous dimethyl sulfoxide (DMSO, Fluka, Sigma-Aldrich Chemie GmbH, Tautkirchen, D)) and added dropwise under nitrogen to a solution of 6 ml (60 mmol) 1,4-diaminobutane in 11 ml anhydrous dimethyl sulfoxide and stirred at 40 °C for 19 h. The reaction mixture was added to a mixture of 80 ml ethanol and 80 ml acetone. The resulting precipitate was separated by centrifugation, washed with a mixture of 20 ml ethanol and 20 ml acetone and re-dissolved in 80 ml water. The solution was dialyzed for 4 days against water (SnakeSkin dialysis tubing, 3.5 kD cut off, Perbio Science Deutschland GmbH, Bonn, D) and subsequently lyophilized. The yield was 52 % (3.15 g) amino-HES 10/0.7.

To 132 µg amino-HES 10/0.7, dissolved in 100 µl sodium phosphate buffer (0.1 M, 0.15 M NaCl, 50 mM EDTA, pH 7.2), 10 µl of a solution of 17.5 mg/ml N-alpha(maleimidoacetoxy)succinimide ester (AMAS) in dry DMSO (both Fluka, Sigma-Aldrich, Chemie GmbH, Taufkirchen, D) were added, and the clear solution was incubated for 80 min at 25 °C and subsequently for 20 min at 40 °C. The excess of AMAS was removed by centrifugal filtration with a VIVASPIN 0.5 ml concentrator, 5KD MWCO (VIVASCIENCE, Hannover, D) at 13,000 rpm, washed 2 times for 30 min with 450 µl of the phosphate buffer and once with 450 µl of a buffer B. To the residual solution, 10 µg G-CSF (Neupogen® from Amgen, München, D, and Granocyte® from Aventis Pharma AG, Zürich, CH, respectively, 3 µg/µl in phosphate buffer) were added, and the mixture was incubated for 16h at 25 °C. The reaction mixture was analysed by gel electrophoresis after concentration in vacuo.

The following methods were chosen:
(x) G-CSF (Granocyte®) employing sodium phosphate buffer (0.1 M, 0.15 M NaCl, 50 mM EDTA, pH 7.2) as buffer B.
(xi) G-CSF (Neupogen®) employing sodium phosphate buffer (0.1 M, 0.15 M NaCl, 50 mM EDTA, pH 7.2) as buffer B.
(xii) G-CSF (Granocyte®) employing a 1 : 1 (v/v) mixture of sodium phosphate buffer (0.1 M, 0.15 M NaCl, 50 mM EDTA, pH 7.2) and 8 M urea, 1 % SDS, pH 7.4 as buffer B.
(xiii) G-CSF (Neupogen®) employing a 1 : 1 (v/v) mixture of sodium phosphate buffer (0.1 M, 0.15 M NaCl, 50 mM EDTA, pH 7.2) and 8 M urea, 1 % SDS, pH 7.4 as buffer B.
(xiv) G-CSF (Granocyte®) employing 8 M urea, 1 % SDS, pH 7.4 as buffer B.
(xv) G-CSF (Neupogen®) employing 8 M urea, 1 % SDS, pH 7.4 as buffer B.

### Example 4, not according to the invention Synthesis of G-CSF conjugates by reaction of hydroxyethyl starch having a reactive ester group with G-CSF

### Example 4.1:

Oxo-HES10/0.4 (MW = 10,559 D, DS = 0.4) was prepared by Supramol Parenteral Colloids GmbH, Rosbach-Rodheim, D; according to DE 196 28 705 A1. The degree of oxidation of oxo-HES was 95 %

66 mg of oxo-HES10/0.4 were dissolved in 0.5 ml anhydrous DMF. To this solution, 3.4 mg of N,N'-disuccinimidyl carbonate were added, and the mixture was stirred for 2 h at room temperature. The resulting solution had a reactive HES concentration of 13 percent by weight.

A solution of G-CSF (Strathmann Biotec AG, Hamburg, D), having a concentration of about 0.5 mg G-CSF/ml, was concentrated to a concentration of 10 mg/ml by ultracentrifugation at a cut-off of 10 kD using a cooling centrifuge.

To 0.5 ml of this concentrated G-CSF solution, 180 µl of a sodium bicarbonate solution were added. Subsequently, 3 portions (100 µl each) of the reactive HES solution were added dropwise to the protein solution, until, after about 30 min., the reaction had come to an end. Thus, the overall molar ratio of reactive HES : G-CSF was 20 : 1. Then, the pH of the mixture was adjusted to 4.0 using 0.1 N HCl.

A HPGPC analysis (High-Performance Gel Permeation Chromatography) gave a yield of about 70 %. This result is shown in Fig. 9.

The mixture could be stored at 4 °C at a pH of 4.0 for 4 d and, according to HPGPC analyses, remained stable, i.e. unchanged.

Downgrading the content of the mixture regarding reaction by-products such as non-reacted oxo-HES and free N-hydroxysuccinimide as well as solvent using a 10 kD ultrafiltration membrane in a cooling centrifuge was possible without difficulties. The results of this downgrading experiment is shown in Fig. 10.

### Example 4.2:

Oxo-HES10/0.4 (MW = 10,559 D, DS = 0.4) was prepared by Supramol Parenteral Colloids GmbH, Rosbach-Rodheim, D; according to DE 196 28 705 A1. The degree of oxidation of oxo-HES was 95 %

400 mg of oxo-HES 10/0.4 were dissolved in 1 ml anhydrous DMF. To this solution, 21 mg of N,N'-disuccinimidyl carbonate were added, and the mixture was stirred for 2 h at room temperature. The resulting solution had a reactive HES concentration of 40 percent by weight.

A solution of G-CSF (Strathmann Biotec AG, Hamburg, D), having a concentration of about 0.5 mg G-CSF/ml, was concentrated to a concentration of 10 mg/ml by ultracentrifugation at a cut-off of 10 kD using a cooling centrifuge.

To 0.5 ml of this concentrated G-CSF solution, 180 µl of a sodium bicarbonate solution were added. Subsequently, 3 portions (100 µl each) of the reactive HES solution were added dropwise to the protein solution, until, after about 30 min., the reaction had come to an end. Thus, the overall molar ratio of reactive HES : G-CSF was about 50 : 1. Then, the pH of the mixture was adjusted to 4.0 using 0.1 N HCl.

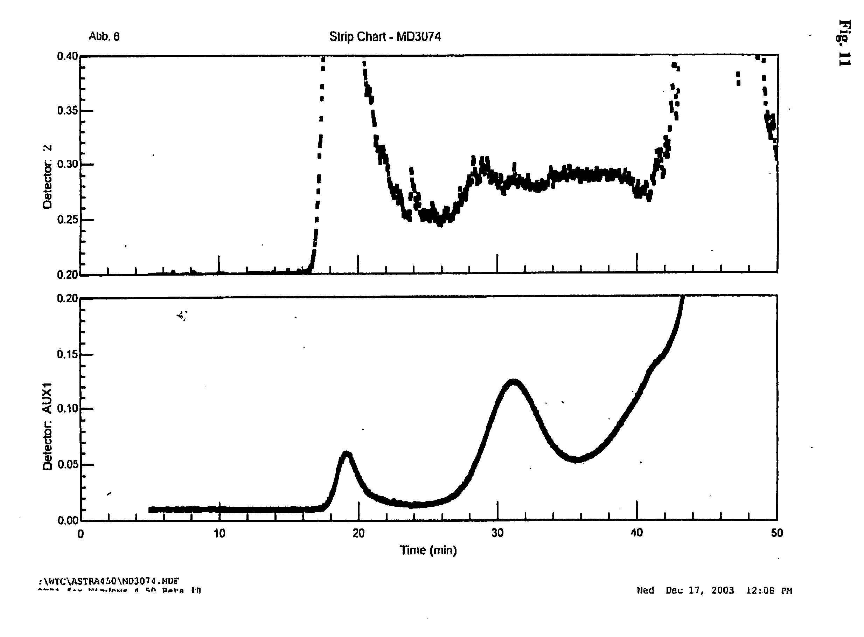

A HPGPC analysis (High-Performance Gel Permeation Chromatography) gave a yield of more than 95 %. No non-reacted G-CSF could be detected. This result is shown in Fig. 11.

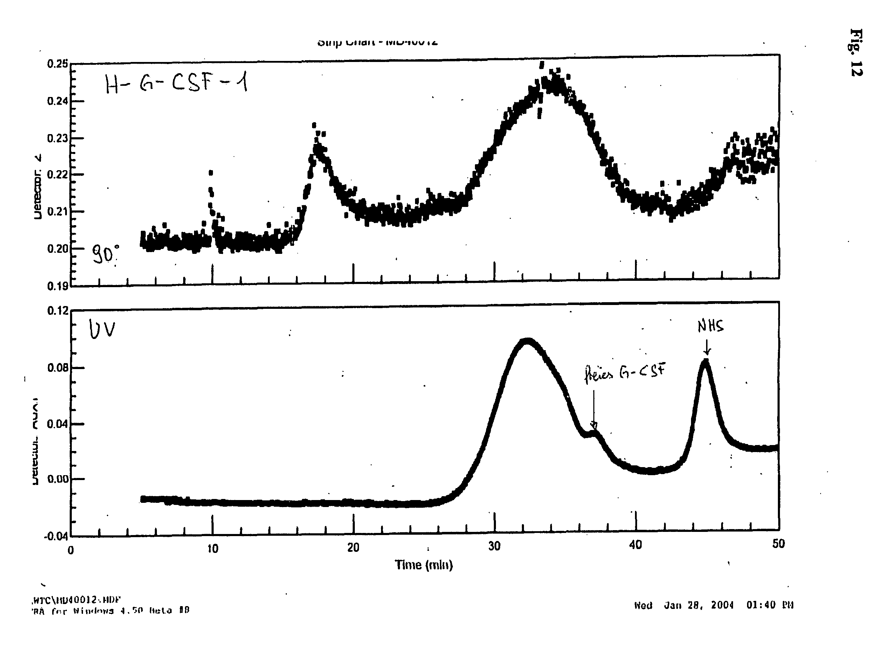

The mixture was purified using ultrafiltration technology without difficulties. The results of thes downgrading is shown in Fig. 12.

### Example 5

### 5.1. Purification

Purified G-CSF having essentially the same characteristics as the commercial product Neupogen ® (Amgen) was obtained and one aliquot was kept unmodified as a control.

### 5.2 Synthesis of conjugates of HES and G-CSF

Conjugates were synthesized essentially as described in Example 4.2, but with Oxo-HES50/0.7 (sample code A60), or as described in Example 2.5 (sample code A32), and used for further buffer exchange and purification.

### 5.3 Buffer exchange of G-CSF and HES-modified G-CSF samples before purification by anion-exchange chromatography

HES-modified G-CSF samples or unmodified G-CSF (as a control) (0.5 - 5 mg protein) were subjected to buffer exchange using Vivaspin 6 Concentrator units (10.000 MWCO PES, Vivascience, Cat. Nr. VS0602). Samples were concentrated to 0.5-0.7 ml and were diluted to 5 ml with 10 mM Na-phosphate buffer pH 7.2. Each sample was subjected 3 times to this concentration/buffer exchange cycle.

### 5.4 Anion exchange chromatography of G-CSF and HES-modified forms thereof on a DEAE-Sepharose column

G-CSF samples after HES-modification and, for comparison, samples of unmodified G-CSF were purified and analyzed by anion exchange chromatography at room temperature by using an ÄKTA explorer 10 system as described. Aliquots of G-CSF either before or after HESylation were dialyzed by ultrafiltration against said buffer A (10 mM Na-phosphate, pH 7.2) or were diluted with about 13 volumes of buffer A. The column containing 2 ml DEAE-Sepharose (DEAE-Sepharose CL-6B, Pharmacia Kat. Nr. 17-0710-01) was regenerated by applying 5.0 column volumes (CV) of 6.5 M guanidine/HCl, 5.0 CV buffer A, 5.0 CV of buffer C (1.5 M NaCl in 10 mM Na-phosphate, pH 7.2) and then 10 CV of buffer A. The samples (0.8 - 4.5 ml in 10 mM Na-phosphate buffer pH 7.2) were then injected by using a flow rate of 0.6 ml/min. Following washing of the sample loop with 10 ml (2 ml sample loop) or 20 ml (5 ml sample loop) buffer A, depending on the sample applied, the column was further washed with 0-22 CV of buffer A (flow rate = 0.8 ml/min). Elution was performed by applying a linear gradient from 0-100% buffer B over 5 CV and an isocratic run with 2.5 CV of 100% buffer B using a flow rate of 0.6 ml/min. The column was re-equilibrated with 5 CV of buffer A and was regenerated as detailed above by using a flow rate of 1 ml/min.

If required, samples were concentrated using a Vivaspin concentrator and buffer exchange was performed as described above. Samples were stored at 0-8°C in 10 mM Na-Acetat buffer pH 4.0 before or after sterile filtration using a 0.2 µm filtrations unit, Corning, Cat. No. 431215). The following samples were prepared for in-vitro bioassays and for further analytical analysis. Protein concentration was determined as described in section 6.1 below:
**I. 0401-15/A33**, 0.44 mg/ml, volume = 500 µl G-CSF (E.coli)
**II. 0402-03/A60**, 0.35 mg/ml, volume = 600 µl H-G-CSF (G-CSF HES modified, 10/0.4)
**III. 0401-13/A32**, 0.28 mg/ml, volume = 900 µl G-CSF (E.coli) HES modified, 10/0.4
**IV. 0401-28/A58**, 0.60 mg/ml, volume = 350 µl Neupogen
**V. 0401-28/A57**, 0.50 mg/ml, volume = 400 µl Neulasta

### 5.5. Further analysis of G-CSF samples

Aliquots of the samples were analyzed for their protein content and for modifications.

### 5.5(a) G-CSF protein quantitation by RIP-HPLC

G-CSF protein content of the samples was quantitated using the unmodified protein preparation (concentration : 0.453 mg/ml) as a standard.

A Dionex HPLC system consisting of a pump P 680 A HPG, degassing unit Degasys DG 1210, an autosampler and injector ASI-100, a sample loop 250 µl, a thermostatted column department TCC 100 along with a UV/Vis-Detektor UVD170U equipped with a Software Chromeleon Chromatography Management System was used. A precolumn CC 8/4 Nucleosil 120-5 C4, Macherey-Nagel, Cat. No. 721889, and a separation column 40 C-4 Nucleosil MPN, 5 µm, 125 x 4 mm RP-column (Macherey-Nagel, ordering No.7200 45.40) were used. Solvent A was H₂O plus 0,06 % (v/v) trifluoroacetic acid and solvent B was 90 % acetonitrile inH₂O, containing 0,06 % (v/v)trifluoroacetic acid; flow rate was : 1 ml/min. UV detection was at 214 ,221, 260 and at 280 nm wavelength.

Samples of approximately 10 - 20 µg were injected into a RP-HPLC column. The following gradient was used:
0-5 min: 0-10 % B

- 17 min: 10-45 % B
- 35 min: 45-80 % B
- 36 min: 80-100 % B
- 38 min: 100 % B
- 39 min: 10 % B
- 45 min: 10 % B

The resulting peak area at the elution position of the standard G-CSF preparation was used and compared to the reference standard by comparing the peak appearing at around 29min at 280 nm wavelength.

### 5.5(b) Reduction + carboxamidomethylation of G-CSF protein

Aliquots from the G-CSF protein samples were reduced and carboxamidomethylated as described elsewhere (Guillermina Forno, Mariela Bollati Fogolin, Marcos Oggero, Ricardo Kratje, Marina Etcheverrigaray, Harald S. Conradt, Manfred Nimtz (2004) N- and O-linked carbohydrates and glycosylation site occupancy in recombinant human granulocyte-macrophage colony-stimulating factor secreted by a Chinese hamster ovary cell line; European J. Biochem, 273(5), 907-919). Carboxamidomethylation leads to modified cystein residues. Endoproteinase Glu-C digestion of the carboxamidomethylated protein was performed in 25 mM NH₄HCO₃ containing 1 M urea at pH 7,8 and using an enzyme /substrate ratio of 0.2:10 for 18 - 24 hours.

### 5.5(c) Separation of Endo-Glu-C peptides by RP-HPLC

The peptides generated by the Endo-Glu-C digestion were separated on a Dionex HPLC system consisting of a pump P 680 A HPG, degassing unit Degasys DG 1210, an autosampler and injector ASI-100, a sample loop 250 µl, a thermostatted column department TCC 100 along with a UV/Vis-Detektor UVD170U equipped with a Software Chromeleon Chromatography Management System was used. A precolumn CC 8/4 Nucleosil 120-5 C4, Macherey-Nagel, Cat. No. 721889, and a separation column 40 C-4 Nucleosil MPN, 5 µm, 125 x 4 mm RP-column (Macherey-Nagel, ordering No.7200 45.40) were used. Solvent A was H₂O plus 0,06 % (v/v) trifluoroacetic acid and solvent B was 90 % acetonitrile inH₂O, containing 0,06 % (v/v)trifluoroacetic acid; flow rate was : 1 ml/min. The following gradient was applied:
0-5 min: 10 % B

- 17 min: 45 % B
- 65 min: 100 % B
- 67 min: 100 % B
- 69 min: 10 % B
- 75 min: 10 % B

UV detection was at 214 ,221, 260 and at 280 nm wavelength. Peptides generated by the Endo-Glu-C digestion were separated (data not shown).

### 5.5(d) Analysis of proteolytic peptides by Matrix-Assisted Laser Desorption/ Ionization Time-of-Flight Mass Spectrometry (MALDI/TOF/TOF-MS)

Mass spectrometry was used to detect the intact N-terminus of G-CSF's in the different samples prepared. Samples (3 - 5 µg) resulting from Endoproteinase Glu-C digestions of reduced and carboxamidomethylated protein samples were used directly for MS-analysis (without the RP-HPLC of step 6.3) and purified using ZipTip pipette tips containing C18 reversed-phase material according to the manufacturer's instructions. After washing with 0.1% (v/v) formic acid, elution of peptides was performed with 10 µl 0.1% (v/v) formic acid in 60% (v/v) acetonitrile.

Proteolytic (Endo-Glu-C) peptide fragments were analyzed with a Bruker ULTRAFLEX time-of-flight (TOF/TOF) instrument in the linear positive ion mode using a matrix of 22.4 mg 3,5-dimethoxy-4-hydroxy-cinnamic acid in 400 µl acetonitrile and 600 µl 0.1% (v/v) trifluoroacetic acid in H₂O; (glyco)-peptides were measured using a matrix of 19 mg α-cyano-4-hydroxycinnamic acid in the same solvent mixture using the reflectron for enhanced resolution. Sample solutions of 1 µl and an approximate concentration of 1-10 pmol·µl⁻¹ were mixed with equal amounts of the respective matrix. This mixture was spotted onto a stainless steel target and dried at room temperature before analysis. Spectra were recorded in the mass range 900 - 5000 dalton. The following Table correlates the expected masses with the respective G-CSF peptides.

**Table: Theoretical (monoisotopic] masses of Endo-Glu-C peptides resulting from XM02**

| **Mass** (**Dalton)** | **Observed in this study** | **aa position** | **artif.modification(s)** | **peptide sequence** |
|---|---|---|---|---|
| **2132.11** | + | 1-20 | Cys_CAM: pos18 m/z 2189.13 | MTPLGPASSLPQSFLLKCLE |
| **1512.81** | + | 21-34 | ------- | QVRKIQGDGAALQE |
| **1534.74** | + | 35-47 | Cys_CAM: pos 37,43 m/z 1648.78 | KLCATYKLCHPEE |
| **4942.63** | - | 48-94 | Cys_CAM: Pos 65, 75 m/z 5056.68 | LVLLGHSLGIPWAPLSSCPS QALQLAGCLSQLHSGLFLYQ GLLQALE |
| **502.25** | - | 95-99 | ------- | GISPE |
| **2835.37** | - | 100-124 | ------ | LGPTLDTLQLDVADFATTIW QQMEE |
| **4026.08** | + | 125-163 | ------ | LGMAPALQPTQGAMPAFASA FQRRAGGVLVASHLQSFLE |
| **1438.83** | + | 164-175 | ------ | VSYRVLRHLAQP |

Cystein residues were carboxamidomethylated; peptides marked as fat were detected in MALDI/TOF spectrum of the non modified G-CSF.

The N-terminal Endo-Glu-C peptide (MTPLGPASSLPQSFLLKCLE; m/z 2189.1) comprising position 1-20 of the protein was detected in MALDI/TOF-MS spectra of samples after proteolytic treatment of G-CSF with endoproteinase Glu-C as described above.

### 5.6. Results

### 5.6(a) Purification of G-CSF and HES modified variants

A32, A60 and non modified G-CSF were subjected to purification using a DEAE-Sepharose CL-6B column as described under A4.

In the case of the unmodified sample 0401-15/A33, no significant absorption at 280 nm was detected in the flow-through and the protein eluted at a concentration of 40-50% buffer B (0.16-0.20 M NaCl) in a volume of 6 ml, with a specific peak area of 660 mAU x ml x mg⁻¹ at 280 nm.

The sample 0401-14/A32 (derived from 0401-15/A33; HESylation with AldehydoHES 10/0.4) eluted over a large range of the gradient at a concentration of buffer B from 20-80% (0.08-0.32 M NaCl) in a volume of 12 ml. About 90% of the total peak area detected at 280 nm was found in the flow-through, containing about 50% of the total protein with an apparently slightly higher molecular mass when compared to the eluted protein, as detected by SDS-PAGE analysis as shown in Figure 13 above.

The sample 0402-03/A60 (HESylated with HES 10/0.4, following the overall procedure of Example 4.2) eluted in a volume of 10.5 ml at a similar concentration of 20-80% buffer B. In this case, about 35% of the total peak area detected at 280 nm was found in the flow-through, however, by SDS-PAGE analysis, no unbound protein was detected in this fraction. When compared to the specific peak area of sample 0401-15/A33, the protein content in the eluate of the sample 0402-03/A60 was 45% higher than the stated protein amount that was applied to the column.

Recovery of proteins was calculated based on the peak area (A280 nm) of the eluting fractions compared to the non modified G-CSF protein.

**Table 1: Comparison of the peak areas at 280 nm detection**

| DEAE-Sepharose Run no. | Description | Calculated protein amount for injection | Eluate Area [280 nm] (mAU x ml) | Yield in eluate compared to eluate from run DS01 A33 ** |
|---|---|---|---|---|
| DS01 **A33** | G-CSF | 0.5 mg | 330 | (0.50 mg) |
| DS03 **A32** | Hesylated | 4.0 mg | 1560 | 2.36 mg |
| | HES 10/0.4 | | | |
| DS04 **A60** | Hesylated | 0.9 mg | 870 | 1.32 mg |
| | HES 10/0.4 | | | |
| | | | | |

| | | | | |
|---|---|---|---|---|
| ** RP-HPLC quantitation of the protein confirmed these results | | | | |

### 5.6(b) Analysis of proteins by peptide mapping and MALDI/TOF MS after treatment with endoproteinase Glu-C

The N-terminal peptide resulting from endoproteinase Glu-C digestion of both the carboxamidomethylated unmodified G-CSF (Figure 14) and the market product Neupogen (data not shown), was clearly detected by MALDI/TOF-MS (*MTPLGPASSLPQSFLLKC*LE,* m/z 2189.1; cystein carboxamidomethylated). This signal was absent in samples subjected to HES-modification by reductive amination (Figure 15) and in Neulasta (data not shown), indicating modification of this peptide. In the case of HES-modified G-CSF, wherein modification was carried out by activated ester chemistry, the N-terminal peptide was detected at a relative signal intensity comparable to that of the non modified starting material A32 (Figure 16) indicating that HES modification of this derivatives has been achieved at different amino acid side chains.

N-terminal sequencing of HES modified G-CSF (sample A33 and the market product Neulasta) revealed a blocked N-terminus suggesting that in fact the N-terminal methionine residue of this protein derivative is modified by HES derivative. Since the signal corresponding to the peptide comprising amino acid residues pos.35-47 (KLCATYKLCHPEE; both cysteine residues carboxamidomethylated m/z 1648.78) was not detected in sample A60, it is concluded that one or both lysine residues (at pos 35 and pos 41) might be modified by HES.

### References:

Guillermina Forno, Mariela Bollati Fogolin, Marcos Oggero, Ricardo Kratje, Marina Etcheverrigaray, Harald S. Conradt, Manfred Nimtz (2004) N- and O-linked carbohydrates and glycosylation site occupancy in recombinant human granulocyte-macrophage colony-stimulating factor secreted by a Chinese hamster ovary cell line Eur. J. Biochem, 271 (5), 907-919
Nimtz, M., Grabenhorst, E., Conradt, H.S., Sanz, L. & Calvete, J.J. (1999) Structural characterization of the oligosaccharide chains of native and crystallized boar seminal plasma spermadhesin PSP-I and PSP-II glycoforms. Eur. J. Biochem. 265, 703-718.
Nimtz, M., Martin, W., Wray, V., Klöppel, K.-D., Agustin, J. & Conradt, H.S. (1993) Structures of sialylated oligosaccharides of human erythropoietin expressed in recombinant BHK-21 cells. Eur J. Biochem. 213, 39-56
Nimtz, M., Noll G., Pâques, E. & Conradt, H.S. (1990) Carbohydrate structures of human tissue plasminogen activator variant expressed in recombinant Chinese hamster ovary cells. FEBS Lett. 271, 14-18.
Schröter, S., Derr, P., Conradt, H.S., Nimtz, M., Hale, G. & Kirchhoff, C. (1999) Male-specific modification of human CD52. J. Biol. Chem. 274, 29862-29873
E.Grabenhorst and H.S.Conradt (1999)The Cytoplasmic, Transmembrane and the Stem Regions of Glycosyltransferases specify their in vivo functional sublocalization and stability in the Golgi J. Biol. Chem., 274, 36107-36116
E. Grabenhorst, A.Hoffmann, M.Nimtz, G. Zettlmeiß1 and H. S.Conradt (1995) Construction of stable BHK-21 cells coexpressing human secretory glycoproteins and human Galβ1-4GlcNAc-R □2,6-sialyltransferase: □2,6-linked NeuAc is preferably attached to the Galβ1-4GlcNAcβ1-2Manβ1-3-branch of biantennary oligosaccharides from secreted recombinant -trace protein. Eur.J.Biochem., 232, 718-725

### Example 6: In vitro results of the G-CSF-conjugate obtained in Examples 2.5 and 4.2 and purified according to Example 5: Mitogenicity of G-CSF variants for mouse NFS-60 cells

G-CSF is known for its specific effects on the proliferation, differentiation, an activation of hematopoietic cells of the neutrophilic granulocyte lineage. The mitogenic capacity of G-CSF variants was tested using mouse NFS-60 cells (N. Shirafuji et al., Exp. Hematol. 1989, 17, 116-119). Cells grown in RPMI medium with 10% fetal calf serum (Gibco INVITROGEN GmbH, Karlsruhe, D) containing 5-10 % WEHI-3B (DSMZ, Braunschweig, D; cultivated as described by the DSMZ) conditioned medium as source of exogenous IL-3 were harvested by centrifugation, washed and aliquoted at 100,000 cells per well in a 24-well plate. Cells were allowed to adapt for 1 hour at 37 °C in RPMI medium without WEHI-3B conditioned media before G-CSF growth factors sample diluted in the same media were added. NFS-60 cells were exposed to purified G-CSF variants for 3 days at 37°C and than the cells were electronically counted (Casy TT Cell Counter, Schärfe System, Reutlingen, D). The results are summarised in Figure 12. As seen in Figure 12, the different G-CSF variants (0.5 - 50 pg/ml) were able to stimulate an increase in the number of cells after 3 days compared to medium that did not contain added growth factors.

Unmodified control proteins G-CSF/A33 and G-CSF/A58 stimulated cells at a very similar extend (ED50=5 - 10 pg/ml) while G-CSF conjugates G-CSF/A60 G-CSF/A32 and G-CSF/A57 showed only a minor decrease in activity if compared to the unmodified version (ED50=10 - 25 pg/ml).
(see Figure 8)

### Example 7 Synthesis of G-CSF-Conjugates

### Example 7.1. Synthesis of the Aldehydo-HES Derivatives

### Example 7.1(a) Synthesis of AminoHES10/0.4

5.12 g of oxo HES10/0.4 (MW = 10000 D, DS = 0.4, Supramol Parenteral Colloids GmbH, Rosbach-Rodheim, D according to DE 196 28 705 A1) were heated over night at 80°C in vacuo and dissolved under nitrogen in 25 mL dry dimethyl sulphoxide (Fluka, Sigma-Aldrich Chemie GmbH, Taufkirchen, D) and 5.13 mL of 1,4-diaminobutane were added. After stirring at 40°C for 17 h the reaction mixture was added to 150 mL of an ice-cold 1:1 mixture of acetone and ethanol (v/v). The precipitated product was collected by centrifugation at 4°C, washed with 40 mL of an ice-cold 1:1 mixture of acetone and ethanol (v/v) and collected by centrifugation. The crude product was dissolved in 80 mL water, dialysed for 4 d against water (SnakeSkin dialysis tubing, 3.5 kD cut off, Perbio Sciences Deutschland GmbH, Bonn, D) and lyophilized. The yield of isolated product was 67%.

### Example 7.1(b) Synthesis of AldehydoHES10/0.4

105 mg 4-formylbenzoic acid and 135 mg 1-hydroxy-1H-benzotriazole (both Aldrich, Sigma-Aldrich Chemie GmbH, Taufkirchen, D) were dissolved in 7 mL N,N-dimethylformamide (Peptide synthesis grade, Biosolve, Valkenswaard, NL) and 135 µL N,N'-diisopropylcarbodiimide (Fluka, Sigma-Aldrich Chemie GmbH, Taufkirchen, D) were added. After incubation at 21 °C for 30 min, 0.7 g of aminoHES 10/0.4 (synthesised as described in 1.1) were added. After shaking for 18 h at 22°C, the reaction mixture was added to 42 mL of an ice-cold 1:1 mixture of acetone and ethanol (v/v). The precipitated product was collected by centrifugation at 4°C, re-dissolved in 5 mL DMF and precipitated with 42 mL ethanol/ acetone as described above. After centrifugation, the collected precipitate was dissolved with water, dialysed for 1 d against water (SnakeSkin dialysis tubing, 3.5 kD cut off, Perbio Sciences Deutschland GmbH, Bonn, D) and lyophilized. The yield of isolated product was 95%.

### Example 7.1(c) - Synthesis of AminoHES10/0.7

6.02 g of oxo-HES 10/0.7 (MW = 10000 D, DS = 0.7, Supramol Parenteral Colloids GmbH, Rosbach-Rodheim, D, according to DE 196 28 705) were dissolved under nitrogen in 32 mL dry dimethyl sulphoxide (Fluka, Sigma-Aldrich Chemie GmbH, Taufkirchen, D) and 6.03 mL of 1,4-diaminobutane were added. After stirring at 40°C for 17 h the reaction mixture was added to 150 mL of an ice-cold 1:1 mixture of acetone and ethanol (v/v). The precipitated product was collected by centrifugation at 4°C, washed with 40 mL of an ice-cold 1:1 mixture of acetone and ethanol (v/v) and collected by centrifugation. The crude product was dissolved in 80 mL water, dialysed for 4 d against water (SnakeSkin dialysis tubing, 3.5 kD cut off, Perbio Sciences Deutschland GmbH, Bonn, D) and lyophilized. The yield of isolated product was 52%.

### Example 7.1(d) Synthesis of AldehydoHES10/0.7

150 mg 4-formylbenzoic acid and 230 mg 1-hydroxy-1H-benzotriazole (both Aldrich, Sigma-Aldrich Chemie GmbH, Taufkirchen, D) were dissolved in 10 mL N,N-dimethylformamide (Peptide synthesis grade, Biosolve, Valkenswaard, NL) and 204 µL N,N'-diisopropylcarbodiimide (Fluka, Sigma-Aldrich Chemie GmbH, Taufkirchen, D) were added. After incubation at 21°C for 30 min, 1 g of aminoHES10/0.7 (synthesised as described in 1.3) were added. After shaking for 19 h at 22°C, the reaction mixture was added to 84 mL of ice-cold 2-propanol. The precipitated product was collected by centrifugation at 4°C, re-dissolved in 50 mL water, dialysed for 2 d against water (SnakeSkin dialysis tubing, 3.5 kD cut off, Perbio Sciences Deutschland GmbH, Bonn, D) and lyophilized. The yield of isolated product was 83%.

### Example 7.1(e) Synthesis of AminoHES30/0.4

5 g of oxo-HES 30/0.4 (MW = 30000 D, DS = 0.4, Supramol Parenteral Colloids GmbH, Rosbach-Rodheim, D, using molar ratios of the ingredients according to DE 196 28 705 A1) were heated over night at 80°C in vacuo and were then dissolved under nitrogen in 28 mL dry dimethyl sulphoxide (Fluka, Sigma-Aldrich Chemie GmbH, Taufkirchen, D) and 1.67 mL of 1,4-diaminobutane were added. After stirring at 40°C for 17 h the reaction mixture was added to 175 mL of an ice-cold 1:1 mixture of acetone and ethanol (v/v). The precipitated product was collected by centrifugation at 4°C. The crude product was dissolved in 40 mL water, dialysed for 2 d against water (SnakeSkin dialysis tubing, 3.5 kD cut off, Perbio Sciences Deutschland GmbH, Bonn, D) and lyophilized. The yield of isolated product was not determined.

### Example 7.1(f) Synthesis of AldehydoHES30/0.4

130 mg 4-formylbenzoic acid and 153 mg 1-hydroxy-1H-benzotriazole (both Aldrich, Sigma-Aldrich Chemie GmbH, Taufkirchen, D) were dissolved in 36 mL N,N-dimethylformamide (Peptide synthesis grade, Biosolve, Valkenswaard, NL) and 110 µL N,N'-diisopropylcarbodiimide (Fluka, Sigma-Aldrich Chemie GmbH, Taufkirchen, D) were added. After incubation at 21°C for 30 min, 2.61 g of aminoHES30/0.4 (synthesised as described in 1.5) were added. After shaking for 22.5 h at 22°C, the reaction mixture was added to 160 mL of an ice-cold 1:1 mixture of acetone and ethanol (v/v). The precipitated product was collected by centrifugation at 4°C and washed with an ice-cold 1:1 mixture of acetone and ethanol (v/v). After centrifugation, the precipitate was dissolved in 30 mL water, dialysed for 1 d against water (SnakeSkin dialysis tubing, 3.5 kD cut off, Perbio Sciences Deutschland GmbH, Bonn, D) and lyophilized. The yield of isolated product was 81%.

### Example 7.1(g) Synthesis of AminoHES30/0.7

5 g of oxo-HES 30/0.7 (MW = 30000 D, DS = 0.7, Supramol Parenteral Colloids GmbH, Rosbach-Rodheim, D, using molar ratios of the ingredient according to DE 196 28 705 A1) were heated over night at 80°C in vacuo and were then dissolved under nitrogen in 28 mL dry dimethyl sulphoxide (Fluka, Sigma-Aldrich Chemie GmbH, Taufkirchen, D) and 1.67 mL of 1,4-diaminobutane were added. After stirring at 40°C for 17 h the reaction mixture was added to 175 mL of an ice-cold 1:1 mixture of acetone and ethanol (v/v). The precipitated product was collected by centrifugation at 4°C. The crude product was dissolved in 40 mL water, dialysed for 2 d against water (SnakeSkin dialysis tubing, 3.5 kD cut off, Perbio Sciences Deutschland GmbH, Bonn, D) and lyophilized. The yield of isolated product was not determined.

### Example 7.1(h) Synthesis of AldehydoHES30/0.7

122 mg 4-formylbenzoic acid and 144 mg 1-hydroxy-1H-benzotriazole (both Aldrich, Sigma-Aldrich Chemie GmbH, Taufkirchen, D) were dissolved in 34 mL N,N-dimethylformamide (Peptide synthesis grade, Biosolve, Valkenswaard, NL) and 103 µL N,N'-diisopropylcarbodiimide (Fluka, Sigma-Aldrich Chemie GmbH, Taufkirchen, D) were added. After incubation at 21°C for 30 min, 2.46 g of aminoHES30/0.7 (synthesised as described in 1.7) were added. After shaking for 22.5 h at 22°C, the reaction mixture was added to 160 mL of an ice-cold 1:1 mixture of acetone and ethanol (v/v). The precipitated product was collected by centrifugation at 4°C and washed with an ice-cold 1:1 mixture of acetone and ethanol (v/v). After centrifugation, the precipitate was dissolved in 30 mL water, dialysed for 1 d against water (SnakeSkin dialysis tubing, 3.5 kD cut off, Perbio Sciences Deutschland GmbH, Bonn, D) and lyophilized. The yield of isolated product was 87%.

### Example 7.1(i) Synthesis of AminoHES50/0.7

6.09 g of oxo-HES 50/0.7 (MW = 50000 D, DS = 0.7, Supramol Parenteral Colloids GmbH, Rosbach-Rodheim, D, using molar rations of the ingredients according to DE 196 28 705 A1) were heated over night at 80°C in vacuo and were then dissolved under nitrogen in 32 mL dry dimethyl sulphoxide (Fluka, Sigma-Aldrich Chemie GmbH, Taufkirchen, D) and 1.22 mL of 1,4-diaminobutane were added. After stirring at 40°C for 17 h the reaction mixture was added to 150 mL of an ice-cold 1:1 mixture of acetone and ethanol (v/v). The precipitated product was collected by centrifugation at 4°C, washed with 40 mL of an ice-cold 1:1 mixture of acetone and ethanol (v/v) and collected by centrifugation. The crude product was dissolved in 80 mL water, dialysed for 4 d against water (SnakeSkin dialysis tubing, 3.5 kD cut off, Perbio Sciences Deutschland GmbH, Bonn, D) and lyophilized. The yield of isolated product was 82%.

### Example 7.1(j) Synthesis of AldehydoHES50/0.7

125 mg 4-formylbenzoic acid and 174 mg 1-hydroxy-1H-benzotriazole (both Aldrich, Sigma-Aldrich Chemie GmbH, Taufkirchen, D) were dissolved in 38 mL N,N-dimethylformamide (Peptide synthesis grade, Biosolve, Valkenswaard, NL) and 155 µL N,N'-diisopropylcarbodiimide (Fluka, Sigma-Aldrich Chemie GmbH, Taufkirchen, D) were added. After incubation at 21°C for 30 min, 3.8 g of aminoHES50/0.7 (synthesised as described in 1.9) were added. After shaking for 19 h at 22°C, the reaction mixture was added to 160 mL of an ice-cold 1:1 mixture of acetone and ethanol (v/v). The precipitated product was collected by centrifugation at 4°C, re-dissolved in 20 mL N,N-dimethylformamide and precipitated with 80 mL of an ice-cold 1:1 mixture of acetone and ethanol (v/v) as described above. After centrifugation, the precipitate was dissolved in 50 mL water, dialysed for 2 d against water (SnakeSkin dialysis tubing, 3.5 kD cut off, Perbio Sciences Deutschland GmbH, Bonn, D) and lyophilized. The yield of isolated product was 77%.

### Example 7.2 Synthesis of the HES-G-CSF Conjugates by reductive amination

### Example 7.2(a) Buffer exchange A:

33 mL of a 0.454 mg/mL solution of hG-CSF (XM02, BioGeneriX AG, Mannheim, D) in 10 mM sodium acetate, 50 mg/mL sorbitol and 0.004% Tween 80 at pH 4.0 were concentrated by diafiltration at 0°C to 4 mL with a Vivaspin 15R concentrator (VS15RH11, 5KD MWCO, Vivascience AG, Hannover, D) and re-diluted to 15 mL with a 0.1 M sodium acetate buffer at pH 5.0. This diafiltration was repeated twice. The final concentration in the last diafiltration step was 3 mg/mL.

### Example 7.2(b) Reaction of hG-CSF with aldehydoHES derivatives of examples 7.1(b), 7.1(d) and 7.1(j)

To 1.67 mL of a solution of hG-CSF after buffer exchange into 0.1 M sodium acetate buffer, pH 5.0 (as described in 7.2(a) above) 1.67 mL of a solution of the HES-derivative and 1.67 mL of a 60 mM solution of sodium cyanoborohydride, both in the same buffer, were added and the solution was incubated for 15.5 h at 4°C. All solutions were cooled to 0°C before mixing.

The following final HES concentrations were employed:
39.4 mg/mL for the HES derivatives prepared according to example 7.1(b) and 7.1(d).
197 mg/mL for the HES derivative prepared according to example 7.1(j).
197 mg/mL HES50/0.7 (MW = 50000 D, DS = 0.7, Supramol Parenteral Colloids GmbH, Rosbach-Rodheim, D) as reaction control.

The reaction mixtures were analysed by gel electrophoresis (see figure 17)

### Example 7.2(c) Buffer exchange B:

20 mL of a 0.454 mg/mL solution of hG-CSF (XM02, BioGeneriX AG, Mannheim, D) in 10 mM sodium acetate, 50 mg/mL sorbitol and 0.004% Tween 80 at pH 4.0 was concentrated by diafiltration at 15°C to 4 mL with a Vivaspin 15R concentrator (VS15RH11, 5KD MWCO, Vivascience AG, Hannover, D) and re-diluted to 15 mL with a 0.1 M sodium acetate buffer at pH 5.0. This diafiltration was repeated twice. The final concentration in the last diafiltration step was 1.5 mg/mL.

### Example 7.2(d) Reaction of hG-CSF with aldehydoHES derivatives of examples 7.1(f) and 7.1(h)

To 3.3 mL of a solution of hG-CSF after buffer exchange into 0.1 M sodium acetate buffer, pH 5.0 (as described in example 7.2(c) above) 3.3 mL of a solution of 789 mg of the HES-derivative and 3.3 mL of a 60 mM solution of sodium cyanoborohydride, both in the same buffer, were added and the solution was incubated for 30 h at 4°C. All solutions were cooled to 0°C before mixing.

After 17 h samples were removed for reaction control. The reaction mixtures were analysed by gel electrophoresis (see figure 18).

### Example 7.3 (not according to the invention) Synthesis of HES-GCFS conjugates by NN'-succinimidyl carbonate coupling

### Example 7.3(a) Synthesis of G-CSF conjugates by reaction of hydroxyethyl starch having a reactive ester group with G-CSF

400 mg of oxo-HES10/0.7 (prepared by Supramol Parenteral Colloids GmbH, Rosbach-Rodheim, D; according to DE 196 28 705 A1, degree of oxidation of oxo-HES was 95 %) were dissolved in 1 ml anhydrous DMF. To this solution, 21 mg of N,N'-disuccinimidyl carbonate were added, and the mixture was stirred for 2 h at room temperature. The resulting solution had a reactive HES concentration of 40 percent by weight.

A solution of G-CSF (Strathmann Biotec AG, Hamburg, D), having a concentration of about 0.5 mg G-CSF/ml, was concentrated to a concentration of 10 mg/ml by ultracentrifugation at a cut-off of 100 kD using a cooling centrifuge.

To 0.5 ml of this concentrated G-CSF solution, 180 µl of a sodium bicarbonate solution were added. Subsequently, 3 portions (100 µl each) of the reactive HES solution were added dropwise to the protein solution, until, after about 30 min., the reaction had come to an end. Thus, the overall molar ratio of reactive HES : G-CSF was about 50:1. Then, the pH of the mixture was adjusted to 4.0 using 0.1 N HCl.

A HPGPC analysis (High-Performance Gel Permeation Chromatography) gave a yield of more than 95 %. No non-reacted G-CSF could be detected. This result is shown in Fig. 19.

### Example 7.4 In vitro assay

### Mitogenicity of G-CSF variants for mouse NFS-60 cells

G-CSF is known for its specific effects on the proliferation, differentiation and activation of hematopoietic cells of the neutrophilic granulocyte lineage. The mitogenic capacity of G-CSF variants was tested using mouse NFS-60 cells (N. Shirafuji et al., Exp. Hematol. 1989, 17, 116-119). Cells grown in RPMI medium with 10% fetal calf serum (Gibco INVITROGEN GmbH, Karlsruhe, D) containing 5-10% WEHI-3B (DSMZ, Braunschweig, D; cultivated as described by the DSMZ) conditioned medium as source of exogenous IL-3 were harvested by centrifugation, washed and aliquoted at 100,000 cells per well in a 24-well plate. Cells were allowed to adapt for 1 hour at 37°C in RPMI medium without WEHI-3B conditioned media before G-CSF growth factor samples diluted in the same media were added. NFS-60 cells were exposed to purified G-CSF variants (purification according to examples 5.3, 5.4, protein quantification according to example 5.5(a)):
Neupogen®, Neulasta® both from Amgen,
"HES-GCFS 10/0.4 conjugate" prepared in example 7.2(b),
"HES-GCFS10/0.7 conjugate" prepared in example 7.2(b),
"HES-GCFS30/0.4 conjugate" prepared in example 7.2(d),
"HES-GCFS30/0.7 conjugate" prepared in example 7.2(d),
"HES-GCFS50/0.7 conjugate" prepared in example 7.2(b),
"HES-GCFS 10/0.7 conjugate (Supramol)" prepared according to example 7.3(a),
"Mock incubation"(= reaction control, 197mg/ml HES50/0.7, MW 50000D, DS 7, Supramol Parenteral Colloids GmbH, Rosbach Rodheim, Germany),
for 3 days at 37°C and than the cells were electronically counted (Casy TT Cell Counter, Schärfe System, Reutlingen, D). The results are summarised in Table 2 and Figure 20. In all cases, the amounts of protein given in Table 2 and Figure 20 represent the G-CSF content of the conjugates only and are based on the concentrations determined by GlycoThera. As can be seen in Figure 20, all of the different G-CSF variants (2.5 - 250 pg/ml) were able to stimulate an increase in the number of cells after 3 days compared to a medium that did not contain added growth factors. All variants reached the same maximum stimulation level at a concentration of 250 pg/ml.

**Table 2: Proliferation of mouse NFS-60 cells, induced by G-CSF variants**

| **Concentration [pg/ml]** | *0* | *2.5* | *2.8* | *5* | *5.7* | *10* | *11.3* | *25* | *28.4* | *50* | *56.7* | *250* | *283.5* |
|---|---|---|---|---|---|---|---|---|---|---|---|---|---|
| **Neupogen** | 0.44 | 0.86 | | 1.20 | | 1.69 | | 2.33 | | 2.49 | | 2.41 | |
| HES-GCSF 10/0.7 | 0.44 | 0.72 | | 0.93 | | 1.44 | | 2.14 | | 2.41 | | 2.41 | |
| HES-GCSF 10/0.4 | 0.44 | 0.72 | | 0.97 | | 1.40 | | 2.17 | | 2.67 | | 2.75 | |
| HES-GCSF 50/0.7 | 0.44 | 0.62 | | 0.70 | | 0.97 | | 1.68 | | 2.15 | | 2.32 | |
| Mock-incubation | 0.44 | 0.85 | | 1.31 | | 1.91 | | 2.38 | | 2.47 | | 2.41 | |
| HES-GCSF 30/0.4 | 0.44 | 0.82 | | 1.21 | | 1.62 | | 2.28 | | 2.50 | | 2.60 | |
| HES-GCSF 30/0.7 | 0.44 | 0.80 | | 1.09 | | 1.66 | | 2.20 | | 2.35 | | 2.44 | |
| Neulasta | 0.44 | 0.63 | | 0.80 | | 1.12 | | 1.83 | | 2.25 | | 2.33 | |
| HES-GCSF 10/0.7 | 0.44 | | 0.73 | | 1.13 | | 1.58 | | 2.24 | | 2.48 | | 2.46 |
| (Supramol) | | | | | | | | | | | | | |

### Example 7.5 in vivo biological effects of hG-CSF conjugates in rats

Upon arrival, the rats [male CRL:CD^{®} rats (7 weeks old), Charles River Deutschland GmbH, Sanghofer Weg 7, D-97633 Sulzfeld)] were randomly sorted into groups of 5. After 7 days acclimatization, rats in poor condition were excluded and replaced by spare animals. The weight of the rats upon arrival was 181-203 g.

Each group of five randomly selected rats was then intravenously administered 100 µg protein per kg body weight (injection speed 15 sec/dosis, vehicle: 5ml PBS/kg bodyweight) of the following non-conjugated or conjugated G-CSF samples (purified according to examples 5.3, 5.4, protein quantification according to example 5.5(a)):
Neupogen® and Neulasta®, both from Amgen,
"HES-GCSF10/0.4 conjugate" (10/0.4) prepared in example 7.2(b),
"HES-GCSF10/0.7 conjugate" (10/0.7) prepared in example 7.2(b),
"HES-GCSF30/0.4 conjugate" (30/0.4) prepared in example 7.2(d),
"HES-GCSF30/0.7 conjugate" (30/0.7) prepared in example 7.2(d),
"HES-GCSF50/0.7 conjugate" (50/0.7) prepared in example 7.2(b),
"HES-GCSF 10/0.7 Supramol" (S 10/0.7) prepared according to example 7.3(a)
"Mock incubation"(= reaction control, 197mg/ml HES50/0.7, MW 50000D, DS 7, Supramol Parenteral Colloids GmbH, Rosbach Rodheim, Germany) and
a vehicle control.

Blood samples from all animals of approx. 200 µl EDTA whole blood were taken from the retrobulbar venous plexus under light ether anaesthesia. On test day -5 blood was taken once in the morning from all animals after overnight fasting. On test days 1 to 8 blood was taken twice daily at an interval of 12 hours. The first blood sample on day 1 was taken prior to G-CSF/GCSF-conjugate administration.

White blood cell (WBC) counts were carried out using a Bayer ADVIA^{™} 120 (Fernwald, Germany). The results are shown in figure 21.

## Claims

1. A method for preparing a conjugate comprising a protein and a polymer derivative, wherein the polymer is a hydroxyalkyl starch (HAS) and the protein is a granulocyte colony stimulating factor (G-CSF), the method comprising reacting at least one functional group A of the polymer derivative with at least one functional group Z of the protein and thereby forming a covalent linkage, wherein Z is an amino group, and wherein A is selected from the group consisting of an aldehyde group, a keto group or a hemiacetal group, wherein the method further comprises introducing A in the polymer to give a polymer derivative by reacting the polymer with an at least bifunctional compound, one functional group of which reacts with the polymer and at least one other functional group of which is an aldehyde group, a keto group or a hemiacetal group, or is a functional group which is further chemically modified to give an aldehyde group, a keto group or a hemiacetal group, and wherein the reaction of the polymer derivative with the protein is a reductive amination.

2. The method as claimed in claim 1 wherein the hydroxyalkyl starch is hydroxyethyl starch.

3. The method as claimed in claim 2 wherein the hydroxyethyl starch has a molecular weight of from 2 to 200 kD, preferably of from 4 to 130 kD, more preferably of from 4 to 70 kD.

4. The method as claimed in any of claims 1 to 3, the method further comprising reacting the polymer with a functional group M of an at least bifunctional compound to give a polymer derivative, the at least bifunctional compound further comprising at least one other functional group Q which is the aldehyde group, keto group or hemiacetal group A.

5. The method as claimed in claim 4, wherein M comprises an amino group.

6. The method as claimed in any of claims 1 to 3, the method further comprising reacting the polymer with a functional group M of an at least bifunctional compound to give a polymer derivative, the at least bifunctional compound further comprising at least one other functional group Q which is not an aldehyde group, keto group or hemiacetal group, the method further comprising reacting the functional group Q with at least one suitable compound to give the polymer derivative comprising the aldehyde group, keto group or hemiacetal group A.

7. The method as claimed in claim 5, wherein M and Q comprise an amino group.

8. The method as claimed in claim 6 or 7, wherein the at least one suitable compound comprises a carboxy group and an aldehyde group, keto group or hemiacetal group.

9. The method as claimed in claim 8, wherein the at least one suitable compound is formylbenzoic acid or 4-(4-formyl-3,5-dimethoxyphenoxy)butyric acid.

10. The method as claimed in claim 6 or 7, wherein M comprises an amino group and Q comprises a beta hydroxy amino group.

11. The method as claimed in claim 10, wherein the polymer is reacted at its oxidized reducing end with a functional group M of an at least bifunctional compound.

12. The method as claimed in claim 10, further comprising oxidizing the beta hydroxyamino group to give the aldehyde group.

13. The method as claimed in claim 12, wherein the oxidation reaction is carried out using a periodate.

14. The method as claimed in any of claims 1 to 13, wherein the reductive amination is carried out in the presence of NaCNBH₃.

15. The method as claimed in any of claims 1 to 14, wherein the reductive amination is carried out at a pH of 7 or less.

16. The method as claimed in claim 15, wherein the pH is 6 or less.

17. The method as claimed in any of claims 1 to 16, wherein the reductive amination is carried out at a temperature of from 0 to 25 °C.

18. The method as claimed in any of claims 1 to 17, wherein the reductive amination is carried out in an aqueous medium.

19. A conjugate as obtainable by a method as claimed in any of claims 1 to 18.

20. A conjugate, comprising a protein and a polymer or a derivative thereof, wherein the polymer is a hydroxyalkyl starch (HAS) and the protein is a granulocyte colony stimulating factor (G-CSF), having a structure according to the formula 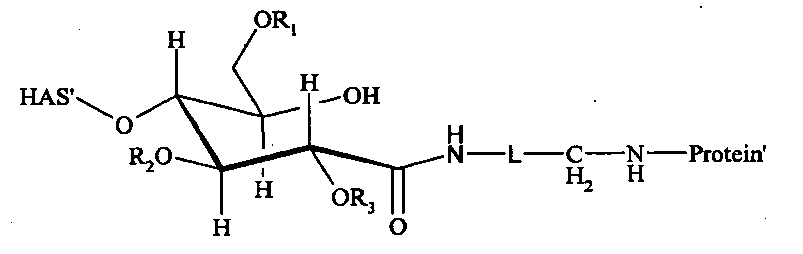 and/or 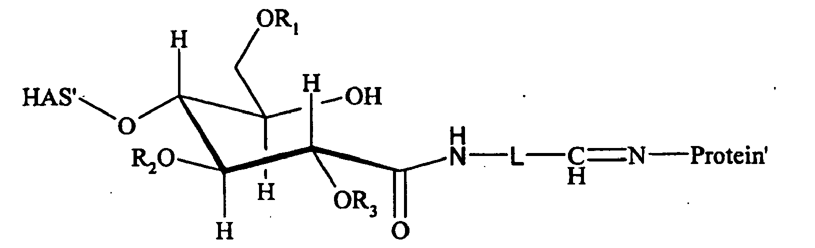 wherein R₁, R₂ and R₃ are independently hydrogen or a hydroxyalkyl group, a hydroxyaryl group, a hydroxyaralkyl group or a hydroxyalkaryl group having of from 2 to 10 carbon atoms, preferably hydrogen or a hydroxyalkyl group, more
preferably hydrogen or a hydroxyethyl group, and
wherein L is an optionally substituted, linear, branched and/or cyclic hydrocarbon residue, optionally comprising at least one heteroatom, having from 1 to 60 preferably from 1 to 40, more preferably from 1 to 20, more preferably from 1 to 10, more preferably from 1 to 6 more preferably from 1 to 2 carbon atoms and especially preferably 1 carbon atom, L being in particular CH₂.

21. A conjugate, comprising a protein and a polymer or a derivative thereof, wherein the polymer is a hydroxyalkyl starch (HAS) and the protein is a granulocyte colony stimulating factor (G-CSF), having a structure according to the formula 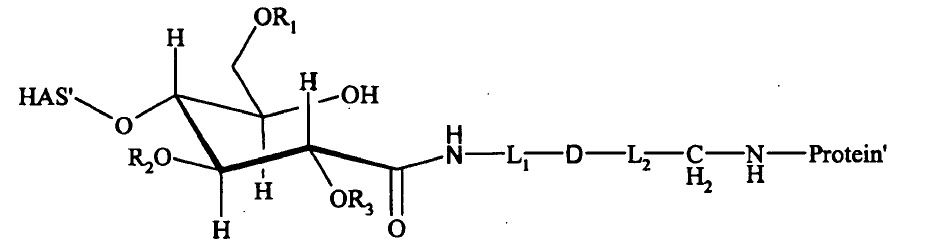 wherein R₁, R₂ and R₃ are independently hydrogen or a hydroxyalkyl group, a hydroxyaryl group, a hydroxyaralkyl group or a hydroxyalkaryl group having of from 2 to 10 carbon atoms, preferably hydrogen or a hydroxyalkyl group, more preferably hydrogen or a hydroxyethyl group, and
wherein L₁ and L₂ are independently an optionally substituted, linear, branched and/or cyclic hydrocarbon residue, optionally comprising at least one heteroatom, comprising an alkyl, aryl, aralkyl heteroalkyl, and/or heteroaralkyl moiety, said residue having from 1 to 60 preferably from 1 to 40, more preferably from 1 to 20, more preferably from 1 to 10 carbon atoms, and
wherein D is a linkage, preferably a covalent linkage which was formed by a suitable functional group F₂ linked to L₁ and a suitable functional group F₃ linked to L₂.

22. The conjugate as claimed in claim 21, wherein L₁ is -(CH₂)n- with n = 2, 3, 4, 5, 6, 7, 8, 9, 10, preferably 2, 3, 4, 5, 6, more preferably 2, 3, 4, and especially preferably 4.

23. The conjugate as claimed in claim 21 or 22, wherein L₂ comprises an optionally suitably substituted aryl moiety, preferably an aryl moiety containing 6 carbon atoms, L₂ being especially preferably C₆H₄.

24. The conjugate as claimed in any of claims 21 to 23, wherein is F₂ selected from the group consisting of
- C-C-double bonds or C-C-triple bonds or aromatic C-C-bonds;
- the thiol group or the hydroxy groups;
- alkyl sulfonic acid hydrazide, aryl sulfonic acid hydrazide;
- 1,2-dioles;
- 1,2 amino-thioalcohols;
- azides;
- 1,2-aminoalcohols;
- the amino group -NH₂ or derivatives of the amino groups comprising the structure unit -NH- such as aminoalkyl groups, aminoaryl group, aminoaralkyl groups, or alkarylaminogroups;
- the hydroxylamino group -O-NH₂, or derivatives of the hydroxylamino group comprising the structure unit -O-NH-, such as hydroxylalkylamino groups, hydroxylarylamino groups, hydroxylaralkylamino groups, or hydroxylalkarylamino groups;
- alkoxyamino groups, aryloxyamino groups, aralkyloxyamino groups, or alkaryloxyamino groups, each comprising the structure unit -NH-O-;
- residues having a carbonyl group, -Q-C(=G)-M, wherein G is O or S, and M is, for example,
-- -OH or -SH;
-- an alkoxy group, an aryloxy group, an aralkyloxy group, or an alkaryloxy group;
-- an alkylthio group, an arylthio group, an aralkylthio group, or an alkarylthio group;
-- an alkylcarbonyloxy group, an arylcarbonyloxy group, an aralkylcarbonyloxy group, an alkarylcarbonyloxy group;
-- activated esters such as esters of hydroxylamines having imid structure such as N-hydroxysuccinimide or having a structure unit O-N where N is part of a heteroaryl compound or, with G = O and Q absent, such as aryloxy compounds with a substituted aryl residue such as pentafluorophenyl, paranitrophenyl or trichlorophenyl;
wherein Q is absent or NH or a heteroatom such as S or O;
- -NH-NH₂, or -NH-NH-;
- -NO₂;
- the nitril group;
- carbonyl groups such as the aldehyde group or the keto group;
- the carboxy group;
- the -N=C=O group or the -N=C=S group;
- vinyl halide groups such as the vinyl iodide or the vinyl bromide group or triflate;
- -C=C-H;
- -(C=NH₂Cl)-OAlkyl
- groups -(C=O)-CH₂-Hal wherein Hal is Cl, Br, or I;
- -CH=CH-SO₂-;
- a disulfide group comprising the structure -S-S-;
- the group 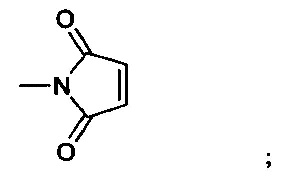
- the group 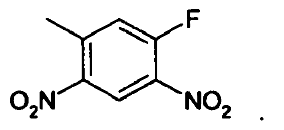 and wherein F₃ is a functional group capable of forming a chemical linkage with F₂ and is preferably selected from the above-mentioned group, F₂ preferably comprising the moiety -NH-, more preferably comprising an amino group, F₃ preferably comprising the moiety -(C=G)-, more preferably -(C=O)-, more preferably the moiety -(C=G)-G-, still more preferably -(C=O)-G-, and especially preferably - (C=O)-O, D being particularly preferably an amide linkage.

25. The conjugate as claimed in any of claims 20 to 24, wherein the hydroxyalkyl starch is hydroxyethyl starch.

26. The conjugate as claimed in claim 25 wherein the hydroxyethyl starch has a molecular weight of from 2 to 200 kD, preferably of from 4 to 130 kD, more preferably of from 4 to 70 kD.

27. A conjugate as claimed in any of claims 19 to 26, for use in a method for the treatment of the human or animal body.

28. A pharmaceutical composition comprising in a therapeutically effective amount a conjugate as claimed in any of claims 19 to 26.

29. A pharmaceutical composition as claimed in claim 28, further comprising at least one pharmaceutically acceptable diluent, adjuvant, or carrier.

30. A conjugate as claimed in any of claims 19 to 26 for use in a method of treating a disorder **characterized by** a reduced hematopoietic or immune function.

31. The conjugate as claimed in claim 30, wherein the disorder is a result of chemotherapy, radiation therapy, infectious disease, severe chronic neutropenia, or leukemia.

## Patentansprüche

1. Verfahren zur Herstellung eines Konjugats umfassend ein Protein und ein Polymerderivat, wobei das Polymer Hydroxyalkylstärke (HAS) und das Protein ein Granulozyten-Kolonie stimulierender Faktor (G-CSF) ist, wobei das Verfahren das Reagieren mindestens einer funktionellen Gruppe A des Polymerderivates mit mindestens einer funktionellen Gruppe Z des Proteins umfasst, wobei dabei eine kovalente Bindung ausgebildet wird, und wobei Z eine Aminogruppe ist und wobei A ausgewählt ist aus der Gruppe bestehend aus einer Aldehydgruppe, einer Ketogruppe oder einer Halbacetalgruppe, wobei das Verfahren weiterhin das Einführen von A in das Polymer unter Erhalt eines Polymerderivates durch Reagieren des Polymers mit einer mindestens bifunktionalen Verbindung umfasst, wobei eine funktionelle Gruppe davon mit dem Polymer reagiert und mindestens eine andere funktionelle Gruppe davon eine Aldehydgruppe, eine Ketogruppe oder eine Halbacetalgruppe ist oder eine funktionelle Gruppe, die weiter chemisch modifiziert wird unter Erhalt einer Aldehydgruppe, einer Ketogruppe oder einer Halbacetalgruppe, und wobei die Reaktion des Polymerderivates mit dem Protein eine reduktive Aminierung ist.

2. Das Verfahren nach Anspruch 1, wobei die Hydroxyalkylstärke Hydroxyethylstärke ist.

3. Das Verfahren nach Anspruch 2, wobei die Hydroxyethylstärke ein Molekulargewicht von 2 bis 200 kDa, bevorzugt von 4 bis 130 kDa, weiter bevorzugt von 4 bis 70 kDa aufweist.

4. Das Verfahren nach einem der Ansprüche 1 bis 3, wobei das Verfahren weiterhin das Reagieren des Polymers mit einer funktionellen Gruppe M einer mindestens bifunktionalen Verbindung unter Erhalt eines Polymerderivates umfasst, wobei die mindestens bifunktionale Verbindung weiterhin mindestens eine andere funktionelle Gruppe Q umfasst, welche die Aldehydgruppe, Ketogruppe oder Halbacetalgruppe A ist.

5. Das Verfahren nach Anspruch 4, wobei M eine Aminogruppe umfasst.

6. Das Verfahren nach einem der Ansprüche 1 bis 3, wobei das Verfahren weiterhin das Reagieren des Polymers mit einer funktionellen Gruppe M einer mindestens bifunktionalen Verbindung unter Erhalt eines Polymerderivates umfasst, wobei die mindestens bifunktionale Verbindung weiterhin mindestens eine andere funktionelle Gruppe Q umfasst, die keine Aldehydgruppe, Ketogruppe oder Halbacetalgruppe ist, wobei das Verfahren weiter das Reagieren der funktionellen Gruppe Q mit mindestens einer geeigneten Verbindung unter Erhalt eines Polymerderivates, umfassend die Aldehydgruppe, Ketogruppe oder Halbacetalgruppe A, umfasst.

7. Das Verfahren nach Anspruch 5, wobei M und Q eine Aminogruppe umfassen.

8. Das Verfahren nach Anspruch 6 oder 7, wobei die mindestens eine geeignete Verbindung eine Carboxylgruppe und eine Aldehydgruppe, Ketogruppe oder Halbacetalgruppe umfasst.

9. Das Verfahren nach Anspruch 8, wobei die mindestens eine geeignete Verbindung Formylbenzoesäure oder 4-(4-Formyl-3,5-dimethoxyphenoxy)buttersäure ist.

10. Das Verfahren nach Anspruch 6 oder 7, wobei M eine Aminogruppe und Q eine Beta-Hydroxyaminogruppe umfasst.

11. Das Verfahren nach Anspruch 10, wobei das Polymer über sein oxidiertes reduzierendes Ende mit der funktionellen Gruppe M einer mindestens bifunktionalen Verbindung umgesetzt wird.

12. Das Verfahren nach Anspruch 10, weiterhin umfassend das Oxidieren der Beta-Hydroxyaminogruppe unter Erhalt der Aldehydgruppe.

13. Das Verfahren nach Anspruch 12, wobei die Oxidationsreaktion ausgeführt wird unter Verwendung eines Perjodats.

14. Das Verfahren nach einem der Ansprüche 1 bis 13, wobei die reduktive Aminierung in Anwesenheit von NaCNBH₃ durchgeführt wird.

15. Das Verfahren nach einem der Ansprüche 1 bis 14, wobei die reduktive Aminierung bei einem pH-Wert von 7 oder weniger durchgeführt wird.

16. Das Verfahren nach Anspruch 15, wobei der pH-Wert 6 oder weniger ist.

17. Das Verfahren nach einem der Ansprüche 1 bis 16, wobei die reduktive Aminierung bei einer Temperatur im Bereich von 0 bis 25 °C durchgeführt wird.

18. Das Verfahren nach einem der Ansprüche 1 bis 17, wobei die reduktive Aminierung in wässrigem Medium durchgeführt wird.

19. Konjugat, erhältlich durch ein Verfahren nach einem der Ansprüche 1 bis 18.

20. Konjugat, umfassend ein Protein und ein Polymer oder ein Derivat davon, wobei das Polymer Hydroxyalkylstärke (HAS) und das Protein ein Granulozyten-Kolonie stimulierender Faktor (G-CSF) ist, wobei das Konjugat die folgende Struktur hat: 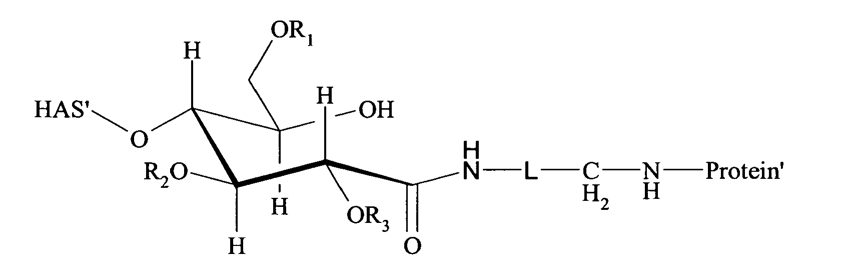 und/oder 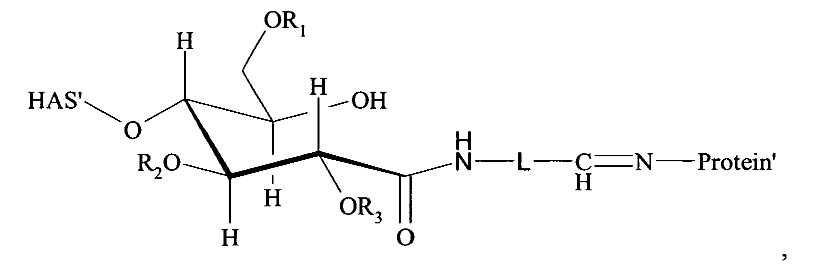 wobei R₁, R₂ und R₃ unabhängig voneinander Wasserstoff oder eine Hydroxyalkylgruppe, eine Hydroxyarylgruppe, eine Hydroxyaralkylgruppe oder eine Hydroxyalkarylgruppe sind, die 2 bis 10 Kohlenstoffatome aufweisen, bevorzugt Wasserstoff oder eine Hydroxyalkylgruppe, weiter bevorzugt Wasserstoff oder eine Hydroxyethylgruppe und
wobei L ein gegebenenfalls substituierter, linearer, verzweigter und/oder zyklischer Kohlenwasserstoffrest ist, gegebenenfalls umfassend mindestens ein Heteroatom, wobei L von 1 bis 60, bevorzugt von 1 bis 40, weiter bevorzugt von 1 bis 20, weiter bevorzugt von 1 bis 10, weiter bevorzugt von 1 bis 6, weiter bevorzugt von 1 bis 2 Kohlenstoffatome und besonders bevorzugt 1 Kohlenstoffatom aufweist, wobei L besonders bevorzugt CH₂ ist.

21. Konjugat umfassend ein Protein und ein Polymer oder Derivat davon, wobei das Polymer Hydroxyalkylstärke (HAS) und das Protein ein Granulozyten-Kolonie stimulierender Faktor (G-CSF) ist, wobei das Konjugat die folgende Struktur aufweist 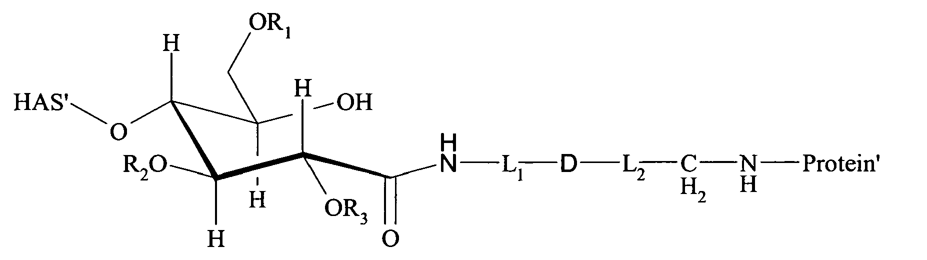 wobei R₁. R₂ und R₃ unabhängig voneinander Wasserstoff oder eine Hydroxyalkylgruppe, eine Hydroxyarylgruppe, eine Hydroxyaralkylgruppe oder eine Hydroxyalkarylgruppe, die 2 bis 10 Kohlenstoffatome aufweis, bevorzugt Wasserstoff oder eine Hydroxyalkylgruppe, weiter bevorzugt Wasserstoff oder eine Hydroxyethylgruppe sind, und
wobei L₁ und L₂ unabhängig voneinander ein gegebenenfalls substituierter, linearer, verzweigter und/oder zyklischer Kohlenwasserstoffrest sind, gegebenenfalls umfassend mindestens ein Heteroatom, umfassend eine Alkyl-, Aryl-, Aralkyl-, Heteroalkyl-und/oder Heteroaralkyleinheit, wobei dieser Rest von 1 bis 60, bevorzugt von 1 bis 40, weiter bevorzugt von 1 bis 20, weiter bevorzugt von 1 bis 10 Kohlenstoffatome aufweist, und
wobei D eine Bindung, bevorzugt eine kovalente Bindung, ist, die gebildet wurde durch Bindung einer geeigneten funktionellen Gruppe F₂ an L₁ und einer geeigneten funktionellen Gruppe F₃ an L₂.

22. Das Konjugat nach Anspruch 21, wobei L₁ -(CH₂)n- ist, mit n = 2, 3, 4, 5, 6, 7, 8, 9, 10, bevorzugt 2, 3, 4, 5, 6, weiter bevorzugt 2, 3, 4 und besonders bevorzugt 4.

23. Das Konjugat nach Anspruch 21 oder 22, wobei L₂ eine gegebenenfalls geeignet substituierte Aryleinheit umfasst, bevorzugt eine Aryleinheit enthaltend 6 Kohlenstoffatome, wobei L₂ besonders bevorzugt C₆H₄ ist.

24. Das Konjugat nach einem der Ansprüche 21 bis 23, wobei F₂ ausgewählt ist aus der Gruppe bestehend aus
- C-C-Doppelbindungen oder C-C-Dreifachbindungen oder aromatischen C-C-Bindungen;
- der Thiolgruppe oder den Hydroxygruppen;
- Alkylsulfonsäurehydrazid, Arylsulfonsäurehydrazid;
- 1,2-Diolen;
- 1,2-Aminothioalkoholen;
- Aziden;
- 1,2-Aminoalkoholen;
- der Aminogruppe -NH₂ oder Derivaten der Aminogruppen umfassend die Struktureinheit -NH- wie Aminoalkylgruppen, Aminoarylgruppe, Aminoaralkylgruppen oder Alkylarylaminogruppen;
- der Hydroxylaminogruppe -O-NH₂ oder Derivaten der Hydroxylaminogruppe umfassend die Struktureinheit -O-NH-, wie Hydroxylalkylaminogruppen, Hydroxylarylaminogruppen, Hydroxylaralkylaminogruppen oder Hydroxylalkarylaminogruppen;
- Alkoxyaminogruppen, Aryloxyaminogruppen, Aralkyloxyaminogrupen oder Alkaryloxyaminogruppen, jede umfassend die Struktureinheit -NH-O-;
- Resten mit einer Carbonylgruppe, -Q-C(=G)-M, wobei G O oder S ist und M z. B.
-- -OH oder -SH;
-- eine Alkoxygruppe, eine Aryloxygruppe, eine Aralkyloxygruppe oder eine Alkaryloxygruppe;
-- eine Alkylthiogruppe, eine Arylthiogruppe, eine Aralkylthiogruppe oder eine Alkarylthiogruppe;
-- eine Alkylcarbonyloxygruppe, eine Arylcarbonyloxygruppe, eine Aralkylcarbonyloxygruppe, eine Alkarylcarbonyloxygruppe;
-- aktivierte Ester wie Ester von Hydroxylaminen, die eine Imidstruktur haben wie N-Hydroxysuccinimid oder eine Struktureinheit O-N haben, wobei N Teil einer Heteroarylverbindung ist oder G = O ist und wobei Q fehlt wie Aryloxyverbindungen mit einem substituierten Arylrest wie Pentafluorphenyl, Paranitrophenyl oder Trichlorphenyl ist;
wobei Q fehlt oder NH oder ein Heteroatom wie S oder O ist;
- -NH-NH₂, oder -NH-NH-;
- -NO₂;
- die Nitrilgruppe;
- Carbonylgruppen wie die Aldehydgruppe oder die Ketogruppe;
- die Carboxygruppe;
- die -N=C=O-Gruppe oder die -N=C=S-Gruppe;
- Vinylhalogengruppen wie die Vinyljodid- oder die Vinylbromidgruppe oder Triflat;
- -C=C-H;
- -(C=NH₂Cl)-O-Alkyl
- Gruppen -(C=O)-CH₂-Hal, wobei Hal Cl, Br oder I ist;
- -CH=CH-SO₂-;
- eine Disulfidgruppe umfassend die Struktur -S-S-;
- die Gruppe 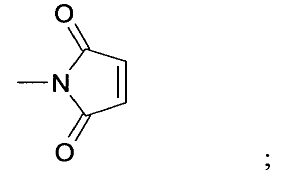
- die Gruppe 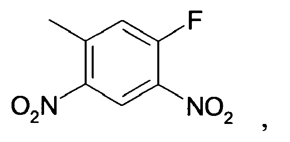 und wobei F₃ eine funktionelle Gruppe ist, die geeignet ist, eine chemische Bindung mit F₂ auszubilden, und bevorzugt ausgewählt ist aus den oben genannten Gruppen, F₂ bevorzugt die Einheit -NH- umfasst, weiter bevorzugt eine Aminogruppe umfasst, F₃ bevorzugt die Einheit -(C=G)- umfasst, weiter bevorzugt -(C=O)-, weiter bevorzugt die Einheit -(C=G)-G-, noch mehr bevorzugt -(C=O)-G- und besonders bevorzugt -(C=O)-O, wobei D besonders bevorzugt eine Amidbindung ist.

25. Das Konjugat nach einem der Ansprüche 20 bis 24, wobei die Hydroxyalkylstärke Hydroxyethylstärke ist.

26. Das Konjugat nach Anspruch 25, wobei die Hydroxyethylstärke ein Molekulargewicht von 2 bis 200 kDa, bevorzugt von 4 bis 130 kDa, weiter bevorzugt von 4 bis 70 kDa hat.

27. Konjugat nach einem der Ansprüche 19 bis 26 zur Verwendung in einem Verfahren zur Behandlung des menschlichen oder tierischen Körpers.

28. Pharmazeutische Zusammensetzung umfassend ein Konjugat nach einem der Ansprüche 19 bis 26 in einer therapeutisch wirksamen Menge.

29. Pharmazeutische Zusammensetzung nach Anspruch 28, weiterhin umfassend mindestens ein/einen pharmazeutisch verträgliches/verträglichen Verdünnungsmittel, Hilfsstoff oder Träger.

30. Konjugat nach einem der Ansprüche 19 bis 26 zur Verwendung in einem Verfahren zur Behandlung einer Störung, die charakterisiert ist durch eine reduzierte Blut-bildende Funktion oder reduzierte Immunfunktion.

31. Das Konjugat nach Anspruch 30, wobei die Störung das Resultat einer Chemotherapie, einer Strahlentherapie, einer infektiösen Krankheit, schwerer chronischer Neutropenie oder Leukämie ist.

## Revendications

1. Procédé de préparation d'un conjugué qui comprend une protéine et un dérivé de polymère, le polymère étant un amidon d'hydroxyalkyle (HAS) et la protéine étant un facteur de stimulation des colonies de granulocytes (G-CSF), le procédé comprenant la mise en réaction d'au moins un groupe fonctionnel A du dérivé de polymère avec au moins un groupe fonctionnel Z de la protéine, et ainsi la formation d'une liaison covalente, Z étant un groupe amino et A étant choisi dans le groupe constitué par un groupe aldéhyde, un groupe céto ou un groupe hémiacétal, le procédé comprenant également l'introduction de A dans le polymère pour obtenir un dérivé de polymère par mise en réaction du polymère avec un composé au moins bifonctionnel, dont un groupe fonctionnel réagit avec le polymère et dont au moins un autre groupe fonctionnel est un groupe aldéhyde, un groupe céto ou un groupe hémiacétal, ou est un groupe fonctionnel qui est modifié chimiquement de manière supplémentaire pour obtenir un groupe aldéhyde, un groupe céto ou un groupe hémiacétal, et la réaction du dérivé de polymère avec la protéine étant une amination réductrice.

2. Procédé selon la revendication 1, dans lequel l'amidon d'hydroxyalkyle est un amidon d'hydroxyéthyle.

3. Procédé selon la revendication 2, dans lequel l'amidon d'hydroxyéthyle a un poids moléculaire de 2 à 200 kD, de préférence de 4 à 130 kD, de manière davantage préférée de 4 à 70 kD.

4. Procédé selon l'une quelconque des revendications 1 à 3, le procédé comprenant également la mise en réaction du polymère avec un groupe fonctionnel M d'un composé au moins bifonctionnel pour obtenir un dérivé de polymère, le composé au moins bifonctionnel comprenant également au moins un autre groupe fonctionnel Q qui est le groupe aldéhyde, le groupe céto ou le groupe hémiacétal A.

5. Procédé selon la revendication 4, dans lequel M comprend un groupe amino.

6. Procédé selon l'une quelconque des revendications 1 à 3, le procédé comprenant également la mise en réaction du polymère avec un groupe fonctionnel M d'un composé au moins bifonctionnel pour obtenir un dérivé de polymère, le composé au moins bifonctionnel comprenant également au moins un autre groupe fonctionnel Q qui n'est pas un groupe aldéhyde, un groupe céto ou un groupe hémiacétal, le procédé comprenant également la mise en réaction du groupe fonctionnel Q avec au moins un composé approprié pour obtenir le dérivé de polymère qui comprend le groupe aldéhyde, le groupe céto ou le groupe hémiacétal A.

7. Procédé selon la revendication 5, dans lequel M et Q comprennent un groupe amino.

8. Procédé selon la revendication 6 ou 7, dans lequel le ou les composés appropriés comprennent un groupe carboxy et un groupe aldéhyde, un groupe céto ou un groupe hémiacétal.

9. Procédé selon la revendication 8, dans lequel le ou les composés appropriés sont l'acide formylbenzoïque ou l'acide 4-(4-formyl-3,5-diméthoxyphénoxy)butyrique.

10. Procédé selon la revendication 6 ou 7, dans lequel M comprend un groupe amino et Q comprend un groupe bêta hydroxyamino.

11. Procédé selon la revendication 10, dans lequel le polymère est mis en réaction à son extrémité réductrice oxydée avec un groupe fonctionnel M d'un composé au moins bifonctionnel.

12. Procédé selon la revendication 10, comprenant également l'oxydation du groupe bêta hydroxyamino pour obtenir le groupe aldéhyde.

13. Procédé selon la revendication 12, dans lequel la réaction d'oxydation est réalisée en utilisant un périodate.

14. Procédé selon l'une quelconque des revendications 1 à 13, dans lequel l'amination réductrice est réalisée en présence de NaCNBH₃.

15. Procédé selon l'une quelconque des revendications 1 à 14, dans lequel l'amination réductrice est réalisée à un pH inférieur ou égal à 7.

16. Procédé selon la revendication 15, dans lequel le pH est inférieur ou égal à 6.

17. Procédé selon l'une quelconque des revendications 1 à 16, dans lequel l'amination réductrice est réalisée à une température de 0 à 25°C.

18. Procédé selon l'une quelconque des revendications 1 à 17, dans lequel l'amination réductrice est réalisée dans un milieu aqueux.

19. Conjugué tel que pouvant être obtenu par un procédé selon l'une quelconque des revendications 1 à 18.

20. Conjugué, comprenant une protéine et un polymère ou un de ses dérivés, le polymère étant un amidon d'hydroxyalkyle (HAS) et la protéine étant un facteur de stimulation des colonies de granulocytes (G-CSF), ayant une structure de formule 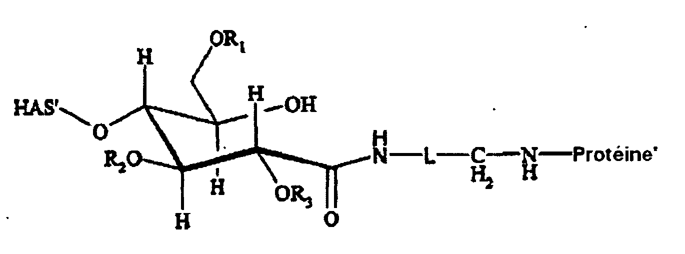 et/ou 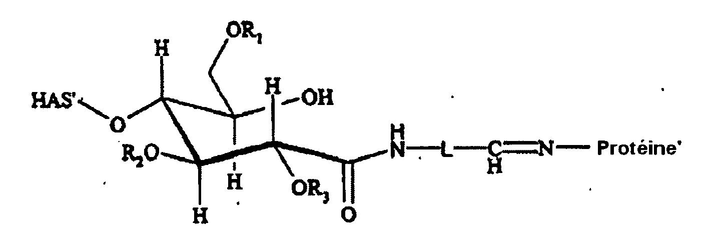 dans lesquelles R₁, R₂ et R₃ sont indépendamment l'hydrogène ou un groupe hydroxyalkyle, un groupe hydroxyaryle, un groupe hydroxyaralkyle ou un groupe hydroxyalkaryle comportant 2 à 10 atomes de carbone, de préférence l'hydrogène ou un groupe hydroxyalkyle, de manière davantage préférée l'hydrogène ou un groupe hydroxyéthyle, et
dans lesquelles L est un résidu hydrocarboné linéaire, ramifié et/ou cyclique, éventuellement substitué, comprenant éventuellement au moins un hétéroatome, comportant 1 à 60, de préférence 1 à 40, de manière davantage préférée 1 à 20, de manière davantage préférée 1 à 10, de manière davantage préférée 1 à 6, de manière davantage préférée 1 à 2 atomes de carbone et de manière notamment préférée 1 atome de carbone, L étant notamment CH₂.

21. Conjugué, comprenant une protéine et un polymère ou un de ses dérivés, le polymère étant un amidon d'hydroxyalkyle (HAS) et la protéine étant un facteur de stimulation des colonies de granulocytes (G-CSF), ayant une structure de formule 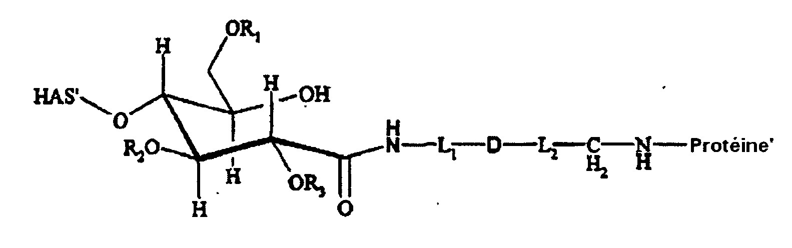 dans laquelle R₁, R₂ et R₃ sont indépendamment l'hydrogène ou un groupe hydroxyalkyle, un groupe hydroxyaryle, un groupe hydroxyaralkyle ou un groupe hydroxyalkaryle comportant 2 à 10 atomes de carbone, de préférence l'hydrogène ou un groupe hydroxyalkyle, de manière davantage préférée l'hydrogène ou un groupe hydroxyéthyle, et
dans laquelle L₁ et L₂ sont indépendamment un résidu hydrocarboné linéaire, ramifié et/ou cyclique, éventuellement substitué, comprenant éventuellement au moins un hétéroatome, comprenant une fraction alkyle, aryle, aralkyle, hétéroalkyle et/ou hétéroaralkyle, ledit résidu comportant 1 à 60, de préférence 1 à 40, de manière davantage préférée 1 à 20, de manière davantage préférée 1 à 10 atomes de carbone, et
dans laquelle D est une liaison, de préférence une liaison covalente qui a été formée par un groupe fonctionnel F₂ approprié relié à L₁ et un groupe fonctionnel F₃ approprié relié à L₂.

22. Conjugué selon la revendication 21, dans lequel L₁ est -(CH₂)ₙ- avec n = 2, 3, 4, 5, 6, 7, 8, 9, 10, de préférence 2, 3, 4, 5, 6, de manière davantage préférée 2, 3, 4 et de manière notamment préférée 4.

23. Conjugué selon la revendication 21 ou 22, dans lequel L₂ comprend une fraction aryle éventuellement substituée de manière appropriée, de préférence une fraction aryle contenant 6 atomes de carbone, L₂ étant de manière notamment préférée C₆H₄.

24. Conjugué selon l'une quelconque des revendications 21 à 23, dans lequel F₂ est choisi dans le groupe constitué par
- les doubles liaisons C-C ou les triples liaisons C-C ou les liaisons C-C aromatiques ;
- le groupe thiol ou les groupes hydroxy ;
- l'hydrazide d'acide alkylsulfonique, l'hydrazide d'acide arylsulfonique ;
- les 1,2-diols ;
- les 1,2-amino-thioalcools ;
- les azides ;
- les 1,2-aminoalcools ;
- le groupe amino -NH₂ ou les dérivés des groupes amino comprenant l'unité structurale -NH-, tels que les groupes aminoalkyle, le groupe aminoaryle, les groupes aminoaralkyle ou les groupes alkarylamino ;
- le groupe hydroxylamino -O-NH₂ ou les dérivés du groupe hydroxylamino comprenant l'unité structurale -O-NH-, tels que les groupes hydroxylalkylamino, les groupes hydroxylarylamino, les groupes hydroxylaralkylamino ou les groupes hydroxylalkarylamino ;
- les groupes alcoxyamino, les groupes aryloxyamino, les groupes aralkyloxyamino ou les groupes alkaryloxyamino, comprenant chacun l'unité structurale
- NH-O- ;
- les résidus comportant un groupe carbonyle, -Q-C-(=G)-M, G étant O ou S, et M étant, par exemple,
- -OH ou -SH ;
- un groupe alcoxy, un groupe aryloxy, un groupe aralkyloxy ou un groupe alkaryloxy ;
- un groupe alkylthio, un groupe arylthio, un groupe aralkylthio ou un groupe alkarylthio ;
- un groupe alkylcarbonyloxy, un groupe arylcarbonyloxy, un groupe aralkylcarbonyloxy, un groupe alkarylcarbonyloxy ;
- des esters activés tels que des esters d'hydroxylamines ayant une structure imide, telles que le N-hydroxysuccinimide, ou comportant une unité structurale O-N, N faisant partie d'un composé hétéroaryle, ou avec G = O et Q étant absent, tels que les composés aryloxy comportant un résidu aryle substitué tel qu'un pentafluorophényle, un paranitrophényle ou un trichlorophényle ;
Q étant absent ou NH ou un hétéroatome tel que S ou O ;
- -NH-NH₂ ou -NH-NH- ;
- -NO₂ ;
- le groupe nitrile ;
- les groupes carbonyle tels que le groupe aldéhyde ou le groupe céto ;
- le groupe carboxy ;
- le groupe -N=C=O ou le groupe -N=C=S ;
- des groupes halogénure de vinyle tels que le groupe iodure de vinyle ou bromure de vinyle ou triflate ;
- -C≡C-H ;
- -(C=NH₂Cl)-O-alkyle ;
- les groupes -(C=O)-CH₂-Hal, Hal étant Cl, Br ou I ;
- -CH=CH-SO₂- ;
- un groupe disulfure comprenant la structure -S-S- ;
- le groupe 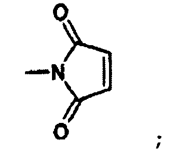
- le groupe 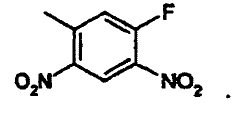 et dans lequel F₃ est un groupe fonctionnel capable de former une liaison chimique avec F₂ et est de préférence choisi parmi les groupes susmentionnés, F₂ comprenant de préférence la fraction -NH-, comprenant de manière davantage préférée un groupe amino, F₃ comprenant de préférence la fraction -(C=G)-, de manière davantage préférée -(C=O)-, de manière davantage préférée la fraction -(C=G)-G-, de manière encore davantage préférée -(C=O)-G-, et de manière notamment préférée - (C=O)-O, D étant de manière particulièrement préférée une liaison amide.

25. Conjugué selon l'une quelconque des revendications 20 à 24, dans lequel l'amidon d'hydroxyalkyle est l'amidon d'hydroxyéthyle.

26. Conjugué selon la revendication 25, dans lequel l'amidon d'hydroxyéthyle a un poids moléculaire de 2 à 200 kD, de préférence de 4 à 130 kD, de manière davantage préférée de 4 à 70 kD.

27. Conjugué selon l'une quelconque des revendications 19 à 26, destiné à une utilisation dans un procédé de traitement du corps humain ou animal.

28. Composition pharmaceutique comprenant un conjugué selon l'une quelconque des revendications 19 à 26 en une quantité thérapeutiquement efficace.

29. Composition pharmaceutique selon la revendication 28, comprenant également au moins un diluant, adjuvant ou véhicule pharmaceutiquement acceptable.

30. Conjugué selon l'une quelconque des revendications 19 à 26, destiné à une utilisation dans un procédé de traitement d'un trouble **caractérisé par** une fonction hématopoïétique ou immunitaire réduite.

31. Conjugué selon la revendication 30, dans lequel le trouble est le résultat d'une chimiothérapie, d'une radiothérapie, d'une maladie infectieuse, d'une neutropénie chronique grave ou d'une leucémie.
